(19) Europäisches Patentamt / European Patent Office / Office européen des brevets

(11) EP 4 737 445 A1

(12) EUROPEAN PATENT APPLICATION
published in accordance with Art. 153(4) EPC

(43) Date of publication:
**06.05.2026 Bulletin 2026/19**

(21) Application number: **23943860.9**

(22) Date of filing: **01.12.2023**

(51) International Patent Classification (IPC):
***C07D 277/82*** (2006.01) ***A61K 31/5377*** (2006.01)
***A61K 31/5386*** (2006.01) ***A61P 25/28*** (2006.01)
***C07D 263/58*** (2006.01) ***C07D 235/30*** (2006.01)
***C07D 498/08*** (2006.01) ***C07D 413/04*** (2006.01)
***C07D 471/04*** (2006.01) ***C07D 498/04*** (2006.01)

(52) Cooperative Patent Classification (CPC):
**A61K 31/5377; A61K 31/5386; A61P 25/28; C07D 235/30; C07D 263/58; C07D 277/82; C07D 413/04; C07D 471/04; C07D 498/04; C07D 498/08**

(86) International application number:
**PCT/KR2023/019633**

(87) International publication number:
**WO 2025/005367 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.06.2023 KR 20230084197**

(71) Applicant: **Korea Institute of Science and Technology**
**Seoul 02792 (KR)**

(72) Inventors:
- **PAE, Ae Nim**
  **Seoul 02792 (KR)**
- **KIM, Yun Kyung**
  **Seoul 02792 (KR)**
- **LIM, Sung Su**
  **Seoul 02792 (KR)**
- **LIM, Sang Min**
  **Seoul 02792 (KR)**
- **LEE, Ha Eun**
  **Seoul 08806 (KR)**
- **JEONG, Da Yeon**
  **Seoul 02792 (KR)**
- **LEE, Chang Yong**
  **Seoul 02792 (KR)**
- **GOTINA, Lizaveta**
  **Seoul 02792 (KR)**
- **KANG, Dong Min**
  **Seoul 02792 (KR)**
- **LUKIANENKO, Nataliia**
  **Seoul 02792 (KR)**
- **KIM, Kyu Hyeon**
  **Seoul 02792 (KR)**
- **ABDILDINOVA, Aizhan**
  **Seoul 02792 (KR)**

(74) Representative: **advotec.**
**Patent- und Rechtsanwaltspartnerschaft mbB**
**Widenmayerstraße 4**
**80538 München (DE)**

# (54) NOVEL SATURATED HETEROCYCLE-SUBSTITUTED HETEROARYL-CARBONOHYDRAZONOYL DICYANIDE COMPOUND AND USE THEREOF

(57) The present invention relates to a novel saturated heterocycle-substituted heteroaryl-carbonohydrazonoyl dicyanide compound or a pharmaceutically acceptable salt thereof, a preparation method therefor, and a pharmaceutical composition comprising same as an active ingredient for preventing or treating neurological diseases. The saturated heterocycle-substituted heteroaryl-carbonohydrazonoyl dicyanide compound of the present invention inhibits the aggregation or hyperphosphorylation of tau protein and/or TDP-43, and thus can be used for preventing and treating neurological diseases including tauopathy and TDP-43 proteinopathy.



Processed by Luminess, 75001 PARIS (FR)

(Cont. next page)

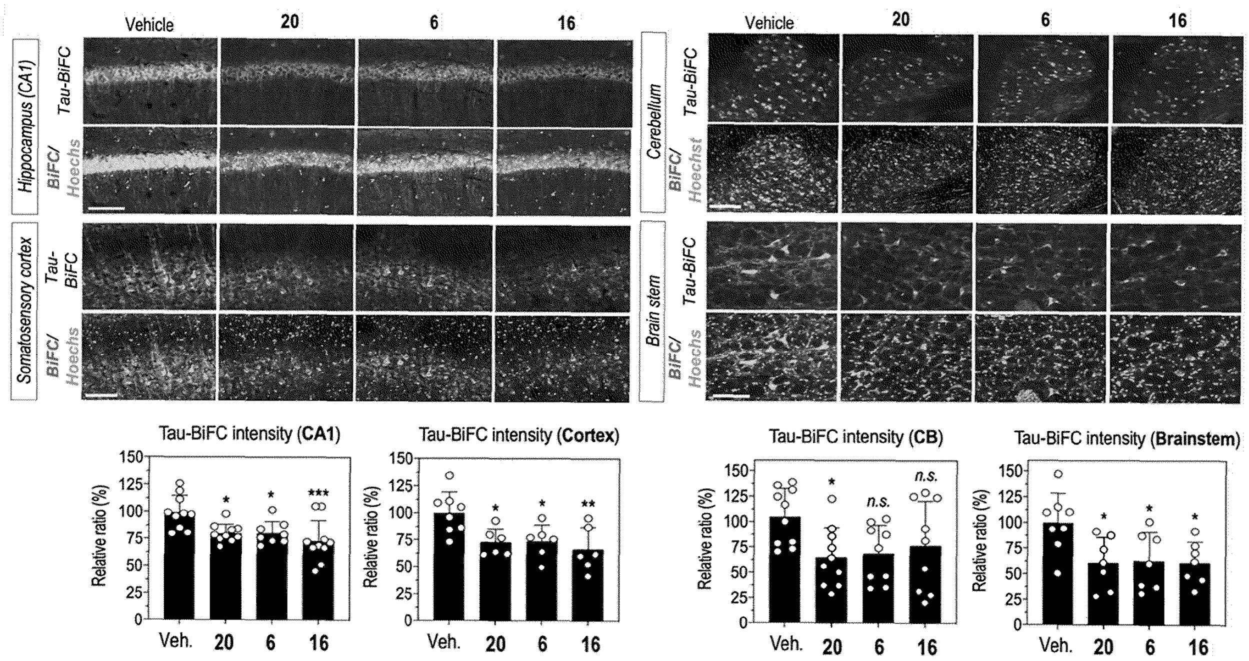
Vehicle
20
6
16
Hippocampus (CA1)
Tau-BiFC
BiFC/ Hoechs
Somatosensory cortex
Tau-BiFC
BiFC/ Hoechs
Cerebellum
Tau-BiFC
BiFC/ Hoechst
Brain stem
Tau-BiFC
BiFC/ Hoechs
Tau-BiFC intensity (CA1)
Tau-BiFC intensity (Cortex)
Tau-BiFC intensity (CB)
Tau-BiFC intensity (Brainstem)
Relative ratio (%)
150
125
100
75
50
25
0
*
***
**
n.s.
Veh.
20
6
16

FIG. 2

## Description

### TECHNICAL FIELD

The disclosure relates to novel heteroaryl-carbonohydrazonoyl dicyanide compounds substituted with saturated heterocycles and uses thereof.

### BACKGROUND ART

Tau protein is a microtubule-associated protein (MAP) that is mainly expressed in axons of neurons, plays a role in stabilizing microtubules, and exhibits molecular diversity by phosphorylation. In humans, tau generates six isoforms by insertion of 29 or 58 amino acid residues in the N-terminal region and alternative splicing of 3 or 4 repeat structures (referred to as microtubule binding sites) at the C-terminus.

In healthy neurons, tau stabilizes microtubules by promoting growth from axons and neuronal polarization. When pathologically hyperphosphorylated, tau separates from microtubules and accumulation and aggregation occur in the cytoplasm. The hyperphosphorylation and/or aggregation of the tau protein causes abnormal accumulation of these tau proteins in neurons, which is pointed to as the cause of various degenerative neurological diseases. Abnormal tau protein aggregation also appears in tauopathies, and although the exact role of tau protein aggregation in the disease exacerbation stage of tauopathy is not known, it appears similar to the aggregation phenomenon that commonly appears in general degenerative brain diseases.

TDP-43 (TDP-43) protein is a protein that mainly exists in the nucleus and is known to be involved in gene expression by binding to DNA or RNA to inhibit gene transcription and regulate RNA splicing and translation processes. In a pathological environment, when TDP-43 protein present in the cell nucleus undergoes modification, it migrates to the cytoplasm and forms abnormal aggregates.

As such, tau protein and/or TDP-43 protein are known to be hyperphosphorylated and/or aggregated alone or in combination in neurons to cause various degenerative brain diseases including tauopathies such as amyotrophic lateral sclerosis, frontotemporal dementia, and Alzheimer's dementia. In particular, when tau protein and TDP-43 protein are aggregated in combination and tauopathy appears, it has been reported that the progression of tau pathology is accelerated and intensified. However, the specific mechanism by which these tau protein and/or TDP-43 protein-mediated diseases transmit signals and exhibit toxicity has not yet been elucidated, and no clear treatment or therapeutic agent that can treat these diseases has yet been known. Accordingly, the present inventors intend to complete and provide an invention that can be applied to tau protein and/or TDP-43 protein-related proteinopathies. Prior art related to the disclosure includes Mol. Neurodegeneration, 2009, 4: 13, Biophys. J., 2006, 90: 3739-3748, J. Biol. Chem., 1995, 270: 7679-7688, J. Neurol. Neurosurg. Psychiatry, 2021, 92: 86-95., Front. Neuroscience, 2022, 16, 815765, Geroscience 2021, 43, 1627-1634, and Brain 2022, 145, 2769-2784.

### DISCLOSURE OF THE INVENTION

### TECHNICAL GOALS

A technical goal to be solved by the disclosure is to provide novel heteroaryl-carbonohydrazonoyl dicyanide compounds substituted with saturated heterocycles or pharmaceutically acceptable salts thereof.

A technical goal to be solved by the disclosure is to provide a method of preparing the heteroaryl-carbonohydrazonoyl dicyanide compounds or pharmaceutically acceptable salts thereof.

In addition, another object of the disclosure is to provide a pharmaceutical composition for preventing or treating diseases caused by aggregation or hyperphosphorylation of tau protein or TDP-43 protein, which includes the heteroaryl-carbonohydrazonoyl dicyanide compound or pharmaceutically acceptable salt thereof as an active ingredient.

However, the technical goals to be solved by the disclosure are not limited to the goals mentioned above, and other problems not mentioned will be clearly understood by those skilled in the art from the description below.

### TECHNICAL SOLUTIONS

To achieve the above goals, the disclosure provides a carbonohydrazonoyl dicyanide compound represented by Formula 1 or a pharmaceutically acceptable salt thereof:

[Formula 1]

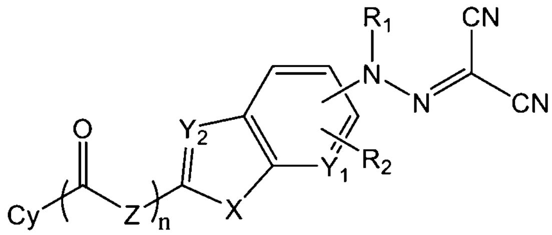

,

wherein in Formula 1,

n may be 0 or 1;
$R_1$ may be any one selected from hydrogen, $C_{1-4}$ alkyl, or $C_{1-4}$ alkylcarbonyl;
$R_2$ may be absent or halogen;
X may be any one selected from O, S, or $NR_3$;
$R_3$ may be hydrogen or $C_{1-4}$ alkyl;
$Y_1$ and $Y_2$ may be C or N;
Z may be absent or NH; and
Cy may be a 5- to 10-membered saturated heterocyclyl that is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.
According to an aspect,
$R_1$ may be any one selected from hydrogen, methyl, or acetyl;
$R_2$ may be absent or chlorine;
X may be any one selected from O, S, or $NR_3$;
$R_3$ may be any one selected from hydrogen, methyl, or ethyl; and
Cy may be a 5- to 10-membered saturated heterocyclyl substituted with one or more selected from the group consisting of fluoro, methyl, isopropyl, methoxy, methylcarbonyl, and tert-butoxycarbonyl.

According to an aspect, the carbonohydrazonoyl dicyanide compound represented by [Formula 1] may be represented by [Formula 1-1] or [Formula 1-2]:

[Formula 1-1]

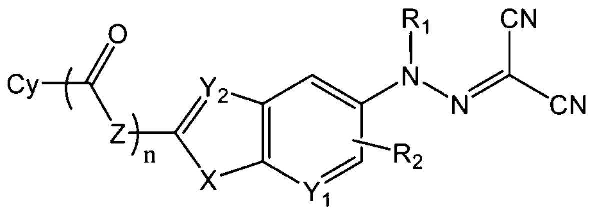

[Formula 1-2]

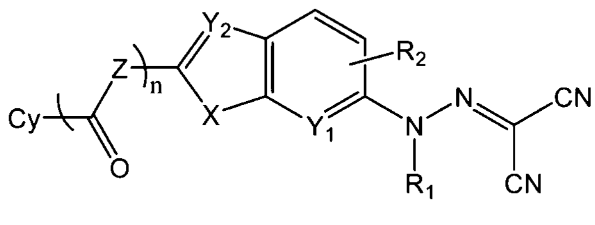

.

According to an aspect,
the compound may be:

1. (2-morpholinobenzo[d]thiazol-5-yl)carbonohydrazonoyl dicyanide,

2. (2-morpholinobenzo[d]thiazol-6-yl)carbonohydrazonoyl dicyanide,
3. (2-morpholinobenzo[d]oxazol-5-yl)carbonohydrazonoyl dicyanide,
4. (2-morpholinobenzo[d]oxazol-6-yl)carbonohydrazonoyl dicyanide,
5. (1-methyl-2-morpholino-1H-benzo[d]imidazol-6-yl)carbonohydrazonoyl dicyanide,
6. (1-methyl-2-morpholino-1H-benzo[d]imidazol-5-yl)carbonohydrazonoyl dicyanide,
7. (2-morpholino-1H-benzo[d]imidazol-6-yl)carbonohydrazonoyl dicyanide,
8. (2-(4-methylpiperazin-1-yl)benzo[d]thiazol-6-yl)carbonohydrazonoyl dicyanide,
9. (2-(4-methoxypiperidin-1-yl)benzo[d]thiazol-6-yl)carbonohydrazonoyl dicyanide,
10. (2-(2,6-dimethylmorpholino)benzo[d]thiazol-6-yl)carbonohydrazonoyl dicyanide,
11. (2-(3-oxa-8-azabicyclo[3.2.1]octan-8-yl)benzo[d]thiazol-6-yl)carbonohydrazonoyl dicyanide,
12. (2-(2-methylmorpholino)benzo[d]thiazol-6-yl)carbonohydrazonoyl dicyanide,
13. (2-(3-methylmorpholino)benzo[d]thiazol-6-yl)carbonohydrazonoyl dicyanide,
14. (2-(4-methylpiperazin-1-yl)benzo[d]oxazol-6-yl)carbonohydrazonoyl dicyanide,
15. (2-(2-methylmorpholino)benzo[d]oxazol-6-yl)carbonohydrazonoyl dicyanide,
16. (2-(3-methylmorpholino)benzo[d]oxazol-6-yl)carbonohydrazonoyl dicyanide,
17. (2-(4-methoxypiperidin-1-yl)benzo[d]oxazol-6-yl)carbonohydrazonoyl dicyanide,
18. (2-(2,6-dimethylmorpholino)benzo[d]oxazol-6-yl)carbonohydrazonoyl dicyanide,
19. (5-chloro-2-morpholinobenzo[d]oxazol-6-yl)carbonohydrazonoyl dicyanide,
20. (2-(3-oxa-8-azabicyclo[3.2. 1]octan-8-yl)benzo[d]oxazol-6-yl)carbonohydrazonoyl dicyanide,
21. tert-butyl 4-(6-(2-(dicyanomethylene)hydrazinyl)benzo[d]oxazol-2-yl)piperazine-1-carboxylate,
22. tert-butyl 4-(6-(2-(dicyanomethylene)hydrazinyl)benzo[d]thiazol-2-yl)piperazine-1-carboxylate,
23. (2-(8-oxa-3-azabicyclo[3.2.1]octan-3-yl)benzo[d]thiazol-6-yl)carbonohydrazonoyl dicyanide,
24. (2-(piperazin-1-yl)benzo[d]thiazol-6-yl)carbonohydrazonoyl dicyanide,
25. (2-(piperazin-1-yl)benzo[d]oxazol-6-yl)carbonohydrazonoyl dicyanide,
26. (2-(8-oxa-3-azabicyclo[3.2.1]octan-3-yl)benzo[d]oxazol-6-yl)carbonohydrazonoyl dicyanide,
27. (2-(4-isopropylpiperazin-1-yl)benzo[d]thiazol-6-yl)carbonohydrazonoyl dicyanide,
28. (2-(4-isopropylpiperazin-1-yl)benzo[d]oxazol-6-yl)carbonohydrazonoyl dicyanide,
29. (2R,6S)-tert-butyl 4-(6-(2-(dicyanomethylene)hydrazinyl)benzo[d]thiazol-2-yl)-2,6-dimethylpiperazine-1-carboxylate,
30. (2-(4-acetylpiperazin-1-yl)benzo[d]thiazol-6-yl)carbonohydrazonoyl dicyanide,
31. (2-(4-acetylpiperazin-1-yl)benzo[d]oxazol-6-yl)carbonohydrazonoyl dicyanide,
32. (1-ethyl-2-morpholino-1H-benzo[d]imidazol-6-yl)carbonohydrazonoyl dicyanide,
33. (1-ethyl-2-morpholino-1H-benzo[d]imidazol-5-yl)carbonohydrazonoyl dicyanide,
34. (2-(3-oxa-8-azabicyclo[3.2.1]octan-8-yl)-1-methyl-1H-benzo[d]imidazol-6-yl)carbonohydrazonoyl dicyanide,
35. (2-(3-oxa-8-azabicyclo[3.2.1]octan-8-yl)-1-methyl-1H-benzo[d]imidazol-5-yl)carbonohydrazonoyl dicyanide,
36. (2-(3-oxa-8-azabicyclo[3.2.1]octan-8-yl)benzo[d]thiazol-6-yl)(methyl)carbonohydrazonoyl dicyanide,
37. acetyl(2-morpholinobenzo[d]thiazol-6-yl)carbonohydrazonoyl dicyanide,
38. (2-(morpholine-4-carboxamido)benzo[d]thiazol-6-yl)carbonohydrazonoyl dicyanide,
39. (2-(2,6-dimethylmorpholine-4-carboxamido)benzo[d]thiazol-6-yl)carbonohydrazonoyl dicyanide,
40. (2-(8-oxa-3-azabicyclo[3.2.1]octane-3-carboxamido)benzo[d]thiazol-6-yl)carbonohydrazonoyl dicyanide,
41. (R)-(2-(3-fluoropyrrolidin-1-yl)benzo[d]oxazol-6-yl)carbonohydrazonoyl dicyanide,
42. (S)-(2-(3-fluoropiperidin-1-yl)benzo[d]oxazol-6-yl)carbonohydrazonoyl dicyanide,
43. (S)-(2-(3-fluoropyrrolidin-1-yl)benzo[d]oxazol-6-yl)carbonohydrazonoyl dicyanide,
44. (2-(3-fluoropiperidin-1-yl)benzo[d]oxazol-6-yl)carbonohydrazonoyl dicyanide,
45. (2-(pyrrolidin-1-yl)benzo[d]oxazol-6-yl)carbonohydrazonoyl dicyanide,
46. (2-(piperidin-1-yl)benzo[d]oxazol-6-yl)carbonohydrazonoyl dicyanide,
47. (R)-N-(2-(3-fluoropiperidin-1-yl)benzo[d]oxazol-6-yl)carbonohydrazonoyl dicyanide,
48. N-(2-(morpholine-4-carbonyl)-1H-pyrrolo[2,3-b]pyridin-5-yl)carbonohydrazonoyl dicyanide, or
49. N-(2-morpholinooxazolo[4,5-b]pyridin-6-yl)carbonohydrazonoyl dicyanide.

According to another embodiment of the disclosure, a method of preparing a compound represented by Formula 1 is provided, which includes a first step of forming an imine bond by reacting a compound represented by Formula 2, which includes a reactive amine group at one terminal, with sodium nitrite and malononitrile in the presence of an acid; and optionally, when $R_1$ is a substituent other than hydrogen, further includes a second step of introducing an $R_1$ substituent to the product obtained from the first step:

[Formula 1]

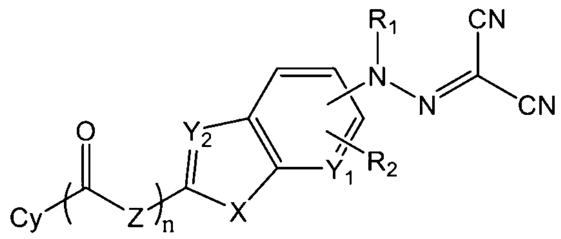

[Formula 2]

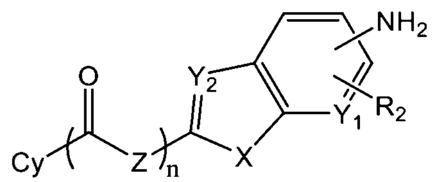

wherein in Formulas 1 and 2,

n is 0 or 1;
$R_1$ is any one selected from hydrogen, $C_{1-4}$ alkyl, or $C_{1-4}$ alkylcarbonyl;
$R_2$ is absent or halogen;
X is any one selected from O, S, or $NR_3$;
$R_3$ is hydrogen or $C_{1-4}$ alkyl;
$Y_1$ and $Y_2$ are C or N;
Z is absent or NH; and
Cy is a 5- to 10-membered saturated heterocyclyl that is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.

According to an aspect, the second step may be performed by:

i) when $R_1$ is $C_{1-6}$ alkyl, reacting with a halogenated alkane in the presence of potassium tert-butoxide or sodium hydride in an organic solvent, and
ii) when $R_1$ is $C_{1-4}$ alkylcarbonyl, reacting with a halogenated alkylcarbonyl in the presence of a base and triethylamine.

According to an aspect,
the compound represented by Formula 2 may be obtained by:

i) when n is 0 and X is S or $NR_3$, reacting Cy-H with a compound represented by Formula 3-a under microwave irradiation,
ii) when n is 0 and X is O, reacting Cy-H with a compound represented by Formula 3-b in the presence of triethylamine,
iii) when n is 1, reacting Cy-H with a compound represented by Formula 3-c, wherein, when $R_N$ is nitro, the reaction product is additionally reduced, and
iv) when $Y_2$ is N, n is 1, and Z is NH, reacting with a compound represented by Formula 3-d.

[Formula 3-a]

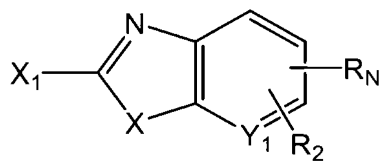

[Formula 3-b]

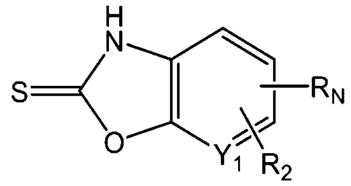

[Formula 3-c]

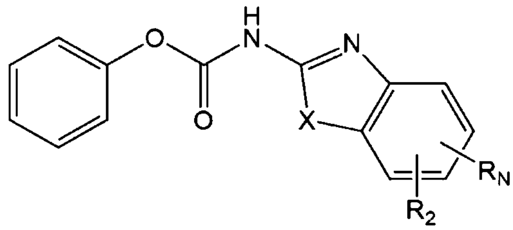

[Formula 3-d]

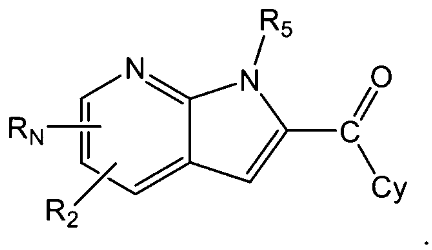

In Formulas 3-a to 3-d,

$X_1$ is halogen, $R_N$ is nitro or amino;
$R_5$ is any one selected from tert-butoxycarbonyl, hydrogen, or benzyloxycarbonyl; and
Cy is a 5- to 10-membered saturated heterocyclyl that is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.

According to an aspect, in the compound represented by Formula 3-a, when X is $NR_3$ and $R_3$ is $C_{1-4}$ alkyl, the compound may be prepared by preparing a compound wherein $R_3$ is hydrogen by the step i) and then reacting with halogenated $R_3$ in the presence of DBU (1,8-Diazabicyclo[5.4.0]undec-7-ene).

According to an aspect, the compound represented by Formula 3-b may be prepared by reacting a compound represented by Formula 4 with potassium ethyl xanthogenate:

[Formula 4]

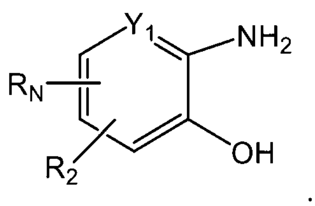

According to an aspect, the compound represented by Formula 3-c may be prepared by reacting a compound represented by Formula 5 with phenyl chloroformate in the presence of triethylamine:

[Formula 5]

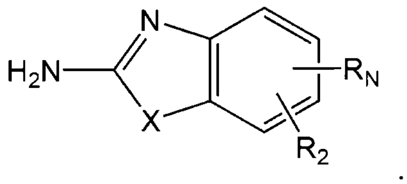

According to another aspect of the disclosure, a pharmaceutical composition for preventing or treating diseases caused by aggregation or hyperphosphorylation of tau protein or TDP-43 protein, which includes the above-described compound as an active ingredient, is provided.

According to an aspect, the disease caused by aggregation or hyperphosphorylation of tau protein may be selected from the group consisting of Alzheimer's disease, Parkinson's disease, vascular dementia, acute stroke, trauma, cerebrovascular disease, brain cord trauma, spinal cord trauma, peripheral neuropathy, retinopathy, glaucoma, and tauopathies.

According to an aspect, the tauopathy may be selected from the group consisting of chronic traumatic encephalopathy (CTE), primary age-related tauopathy, progressive supranuclear palsy, corticobasal degeneration, Pick's disease, argyrophilic grain disease (AGD), frontotemporal dementia (FTD), Parkinsonism linked to chromosome 17, Lytico-Bodig disease (Parkinson-dementia complex of Guam), ganglioglioma, gangliocytoma, meningioangiomatosis, postencephalitic parkinsonism, subacute sclerosing panencephalitis, lead encephalopathy, tuberous sclerosis, Pantothenate kinase-associated neurodegeneration, lipofuscinosis, posttraumatic stress disorder, and traumatic brain injury.

According to an aspect, the disease caused by aggregation or hyperphosphorylation of TDP-43 protein may be selected from the group consisting of amyotrophic lateral sclerosis (ALS), Familial ALS-SOD1, sporadic amyotrophic lateral sclerosis, superoxide dismutase 1 (SOD1), frontotemporal lobar degeneration (FTLD), ALS-FTLD, Multiple system proteinopathy (MSP), limbic-predominant age-related TDP-43 encephalopathy (LATE)/CART (cerebral age-related TDP-43 with sclerosis), Perry disease, facial onset sensory and motor neuronopathy (FOSMN), and sporadic inclusion body myositis (sIBM).

According to an aspect, the composition may inhibit aggregation of tau protein or TDP-43 protein.

According to an aspect, the composition may inhibit hyperphosphorylation of tau protein or TDP-43 protein.

## EFFECTS OF THE INVENTION

The novel heteroaryl-carbonohydrazonoyl dicyanide compounds substituted with saturated heterocycles of the disclosure can efficiently inhibit aggregation and/or hyperphosphorylation of tau protein and TDP-43, and therefore can be usefully used for preventing or treating diseases caused thereby, such as various degenerative brain diseases including frontotemporal dementia, Alzheimer's dementia, and tauopathies.

The effects of the disclosure are not limited to the effects described above, and should be understood to include all effects that can be inferred from the configuration of the disclosure described in the detailed description or claims of the disclosure.

## BRIEF DESCRIPTION OF DRAWINGS

FIG. 1 shows experimental results of cognitive function improvement effect in a tau transgenic animal model.

FIG. 2 shows experimental results of tau aggregation inhibitory effect by BiFC fluorescence imaging of brain tissue.

FIG. 3 shows experimental results of tau pathology improvement effect through inhibition of immunofluorescence staining using a tau phosphorylation antibody.

FIG. 4 shows experimental results of tau pathology inhibitory efficacy effect through brain lysate immunoblot.

## BEST MODE FOR CARRYING OUT THE INVENTION

The present inventors synthesized a novel heteroaryl-carbonohydrazonoyl dicyanide compound substituted with a saturated heterocycle and confirmed that it has utility for diseases related to aggregation and/or hyperphosphorylation of tau protein and TDP-43, thereby completing the invention.

To solve the above problem, the disclosure provides a carbonohydrazonoyl dicyanide compound represented by

Formula 1 or a pharmaceutically acceptable salt thereof:

[Formula 1]

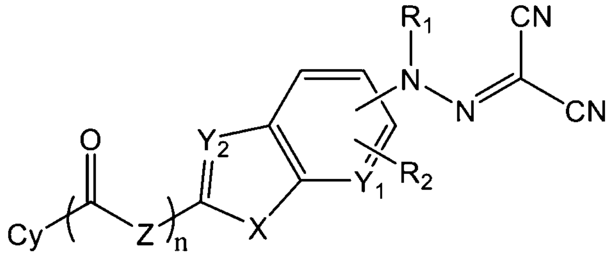

.

In Formula 1,

n may be 0 or 1;
$R_1$ may be any one selected from hydrogen, $C_{1-4}$ alkyl, or $C_{1-4}$ alkylcarbonyl;
$R_2$ may be absent or halogen;
X may be any one selected from O, S or $NR_3$;
$R_3$ may be hydrogen, or $C_{1-4}$ alkyl;
$Y_1$ and $Y_2$ may be C or N;
Z may be absent or NH; and
Cy may be, but is not limited to, a 5- to 10-membered saturated heterocyclyl which is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.

Preferably,

$R_1$ may be any one selected from hydrogen, methyl or acetyl;
$R_2$ may be absent or chlorine;
X may be any one selected from O, S or $NR_3$;
$R_3$ may be any one selected from hydrogen, methyl, or ethyl; and
Cy may be, but is not limited to, a 5- to 10-membered saturated heterocyclyl substituted with one or more selected from the group consisting of fluoro, methyl, isopropyl, methoxy, methylcarbonyl, and tert-butoxycarbonyl.

According to an aspect, the carbonohydrazonoyl dicyanide compound represented by [Formula 1] may be represented by [Formula 1-1] or [Formula 1-2]:

[Formula 1-1]

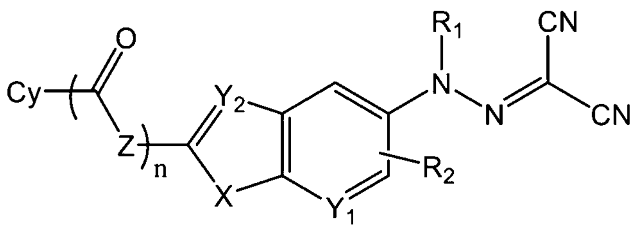

[Formula 1-2]

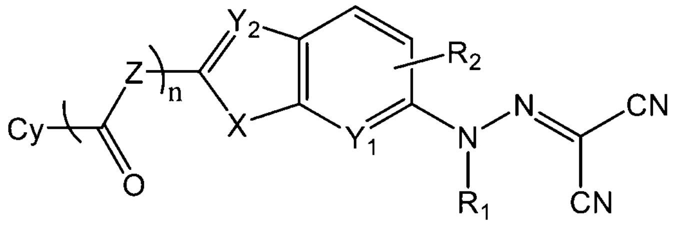

.

According to an aspect,
The compound may be

1. (2-morpholinobenzo[d]thiazol-5-yl)carbonohydrazonoyl dicyanide,
2. (2-morpholinobenzo[d]thiazol-6-yl)carbonohydrazonoyl dicyanide,
3. (2-morpholinobenzo[d]oxazol-5-yl)carbonohydrazonoyl dicyanide,
4. (2-morpholinobenzo[d]oxazol-6-yl)carbonohydrazonoyl dicyanide,
5. (1-methyl-2-morpholino-1H-benzo[d]imidazol-6-yl)carbonohydrazonoyl dicyanide,
6. (1-methyl-2-morpholino-1H-benzo[d]imidazol-5-yl)carbonohydrazonoyl dicyanide,
7. (2-morpholino-1H-benzo[d]imidazol-6-yl)carbonohydrazonoyl dicyanide,
8. (2-(4-methylpiperazin-1-yl)benzo[d]thiazol-6-yl)carbonohydrazonoyl dicyanide,
9. (2-(4-methoxypiperidin-1-yl)benzo[d]thiazol-6-yl)carbonohydrazonoyl dicyanide,
10. (2-(2,6-dimethylmorpholino)benzo[d]thiazol-6-yl)carbonohydrazonoyl dicyanide,
11. (2-(3-oxa-8-azabicyclo[3.2.1]octan-8-yl)benzo[d]thiazol-6-yl)carbonohydrazonoyl dicyanide,
12. (2-(2-methylmorpholino)benzo[d]thiazol-6-yl)carbonohydrazonoyl dicyanide,
13. (2-(3-methylmorpholino)benzo[d]thiazol-6-yl)carbonohydrazonoyl dicyanide,
14. (2-(4-methylpiperazin-1-yl)benzo[d]oxazol-6-yl)carbonohydrazonoyl dicyanide,
15. (2-(2-methylmorpholino)benzo[d]oxazol-6-yl)carbonohydrazonoyl dicyanide,
16. (2-(3-methylmorpholino)benzo[d]oxazol-6-yl)carbonohydrazonoyl dicyanide,
17. (2-(4-methoxypiperidin-1-yl)benzo[d]oxazol-6-yl)carbonohydrazonoyl dicyanide,
18. (2-(2,6-dimethylmorpholino)benzo[d]oxazol-6-yl)carbonohydrazonoyl dicyanide,
19. (5-chloro-2-morpholinobenzo[d]oxazol-6-yl)carbonohydrazonoyl dicyanide,
20. (2-(3-oxa-8-azabicyclo[3.2.1]octan-8-yl)benzo[d]oxazol-6-yl)carbonohydrazonoyl dicyanide,
21. tert-butyl 4-(6-(2-(dicyanomethylene)hydrazinyl)benzo[d]oxazol-2-yl)piperazine-1-carboxylate,
22. tert-butyl 4-(6-(2-(dicyanomethylene)hydrazinyl)benzo[d]thiazol-2-yl)piperazine-1-carboxylate,
23. (2-(8-oxa-3-azabicyclo[3.2.1]octan-3-yl)benzo[d]thiazol-6-yl)carbonohydrazonoyl dicyanide,
24. (2-(piperazin-1-yl)benzo[d]thiazol-6-yl)carbonohydrazonoyl dicyanide,
25. (2-(piperazin-1-yl)benzo[d]oxazol-6-yl)carbonohydrazonoyl dicyanide,
26. (2-(8-oxa-3-azabicyclo[3.2.1]octan-3-yl)benzo[d]oxazol-6-yl)carbonohydrazonoyl dicyanide,
27. (2-(4-isopropylpiperazin-1-yl)benzo[d]thiazol-6-yl)carbonohydrazonoyl dicyanide,
28. (2-(4-isopropylpiperazin-1-yl)benzo[d]oxazol-6-yl)carbonohydrazonoyl dicyanide,
29. (2R,6S)-tert-butyl 4-(6-(2-(dicyanomethylene)hydrazinyl)benzo[d]thiazol-2-yl)-2,6-dimethylpiperazine-1-carboxylate,
30. (2-(4-acetylpiperazin-1-yl)benzo[d]thiazol-6-yl)carbonohydrazonoyl dicyanide,
31. (2-(4-acetylpiperazin-1-yl)benzo[d]oxazol-6-yl)carbonohydrazonoyl dicyanide,
32. (1-ethyl-2-morpholino-1H-benzo[d]imidazol-6-yl)carbonohydrazonoyl dicyanide,
33. (1-ethyl-2-morpholino-1H-benzo[d]imidazol-5-yl)carbonohydrazonoyl dicyanide,
34. (2-(3-oxa-8-azabicyclo[3.2.1]octan-8-yl)-1-methyl-1H-benzo[d]imidazol-6-yl)carbonohydrazonoyl dicyanide,
35. (2-(3-oxa-8-azabicyclo[3.2.1]octan-8-yl)-1-methyl-1H-benzo[d]imidazol-5-yl)carbonohydrazonoyl dicyanide,
36. (2-(3-oxa-8-azabicyclo[3.2.1]octan-8-yl)benzo[d]thiazol-6-yl)(methyl)carbonohydrazonoyl dicyanide,
37. acetyl(2-morpholinobenzo[d]thiazol-6-yl)carbonohydrazonoyl dicyanide,
38. (2-(morpholine-4-carboxamido)benzo[d]thiazol-6-yl)carbonohydrazonoyl dicyanide,
39. (2-(2,6-dimethylmorpholine-4-carboxamido)benzo[d]thiazol-6-yl)carbonohydrazonoyl dicyanide,
40. (2-(8-oxa-3-azabicyclo[3.2.1]octane-3-carboxamido)benzo[d]thiazol-6-yl)carbonohydrazonoyl dicyanide,
41. (R)-(2-(3-fluoropyrrolidin-1-yl)benzo[d]oxazol-6-yl)carbonohydrazonoyl dicyanide
42. (S)-(2-(3-fluoropiperidin-1-yl)benzo[d]oxazol-6-yl)carbonohydrazonoyl dicyanide
43. (S)-(2-(3-fluoropyrrolidin-1-yl)benzo[d]oxazol-6-yl)carbonohydrazonoyl dicyanide,

44. (2-(3-fluoropiperidin-1-yl)benzo[d]oxazol-6-yl)carbonohydrazonoyl dicyanide,
45. (2-(pyrrolidin-1-yl)benzo[d]oxazol-6-yl)carbonohydrazonoyl dicyanide
46. (2-(piperidin-1-yl)benzo[d]oxazol-6-yl)carbonohydrazonoyl dicyanide
47. (R)-N-(2-(3-fluoropiperidin-1-yl)benzo[d]oxazol-6-yl)carbonohydrazonoyl dicyanide
48. N-(2-(morpholine-4-carbonyl)-1H-pyrrolo[2,3-b]pyridin-5-yl)carbonohydrazonoyl dicyanide, or
49. N-(2-morpholinooxazolo[4,5-b]pyridin-6-yl)carbonohydrazonoyl dicyanide, and these may be represented as shown in Table 1 below.

[Table 1]

| | | | |
|---|---|---|---|
| 1 | 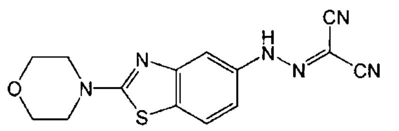 | 2 | 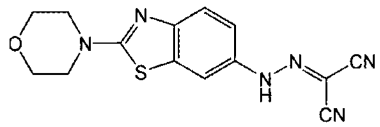 |
| 3 | 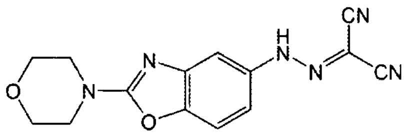 | 4 | 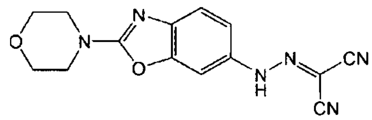 |
| 5 | 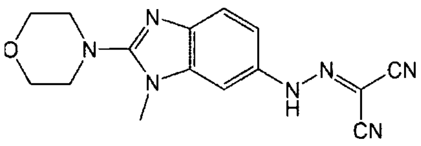 | 6 | 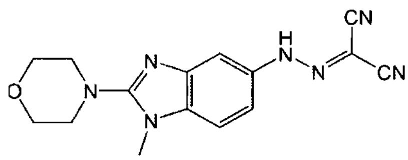 |
| 7 | 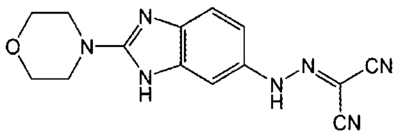 | 8 | 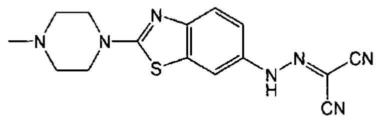 |
| 9 | 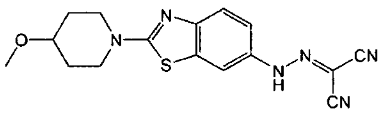 | 10 | 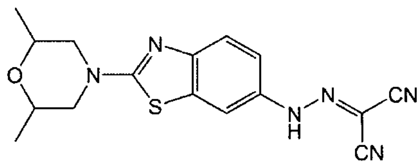 |
| 11 | 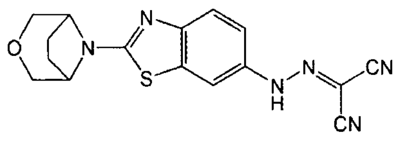 | 12 | 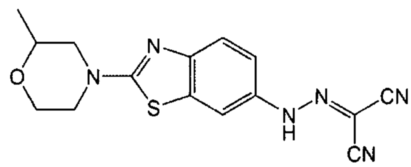 |
| 13 | 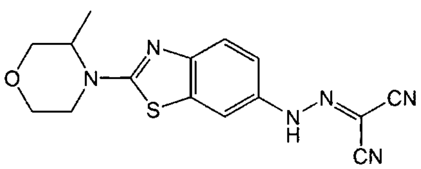 | 14 | 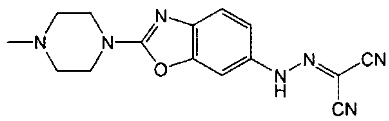 |
| 15 | 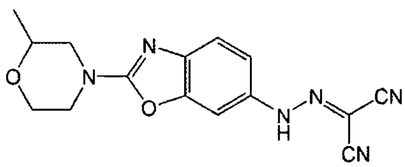 | 16 | 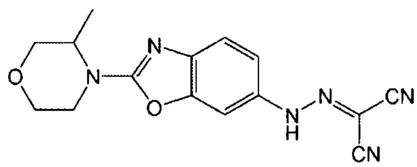 |

(continued)

| | | | |
|---|---|---|---|
| 17 | 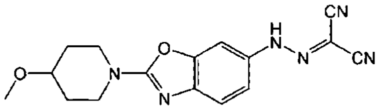 | 18 | 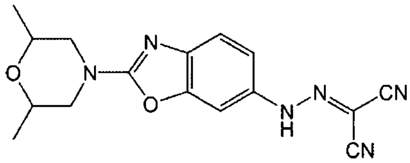 |
| 19 | 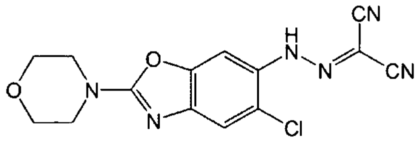 | 20 | 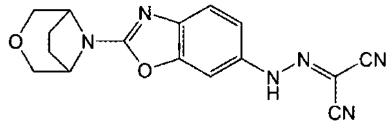 |
| 21 | 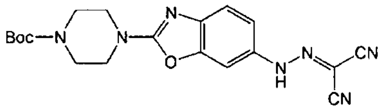 | 22 | 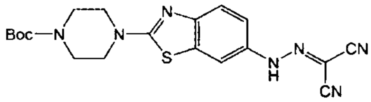 |
| 23 | 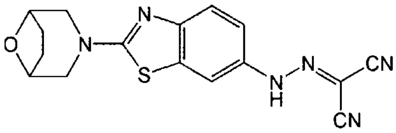 | 24 | 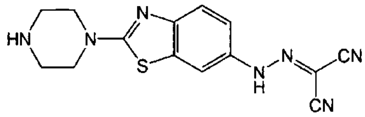 |
| 25 | 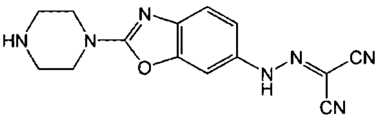 | 26 | 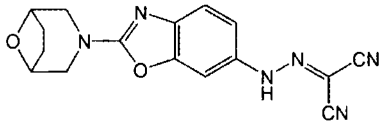 |
| 27 | 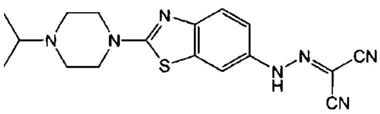 | 28 | 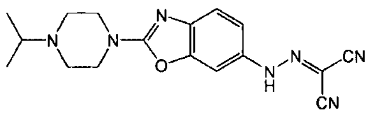 |
| 29 | 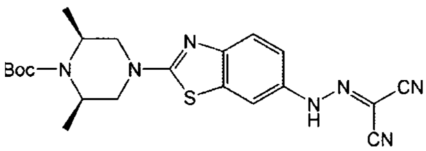 | 30 | 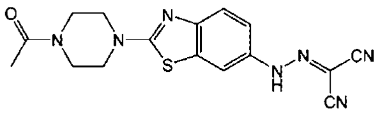 |
| 31 | 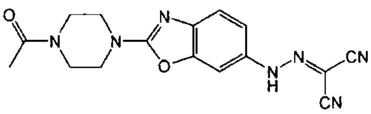 | 32 | 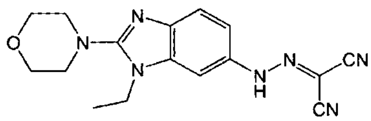 |
| 33 | 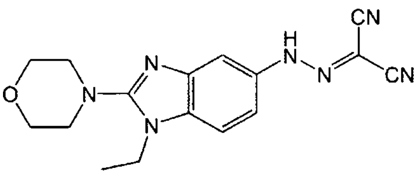 | 34 | 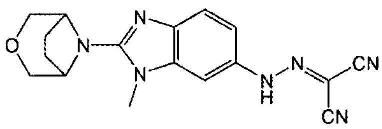 |
| 35 | 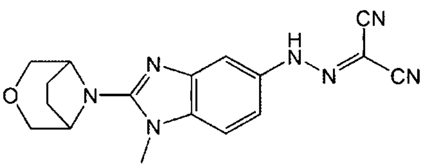 | 36 | 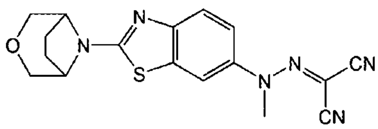 |
| 37 | 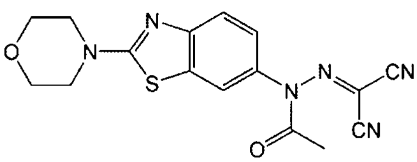 | 38 | 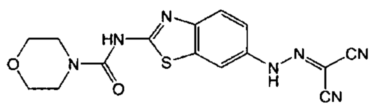 |

(continued)

| | | | |
|---|---|---|---|
| 39 | 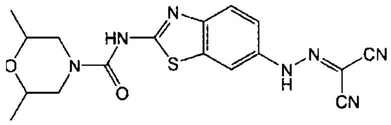 | 40 | 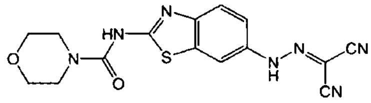 |
| 41 | 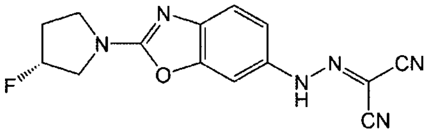 | 42 | 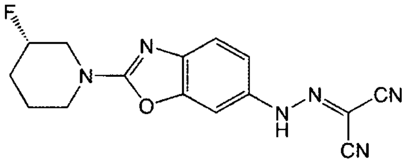 |
| 43 | 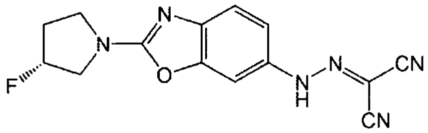 | 44 | 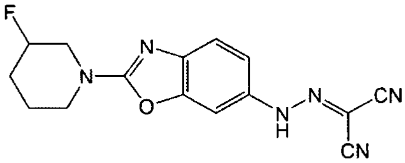 |
| 45 | 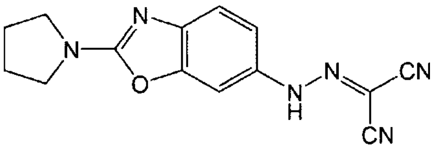 | 46 | 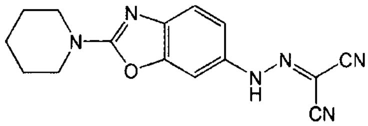 |
| 47 | 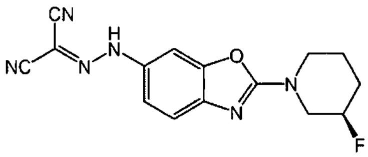 | 48 | 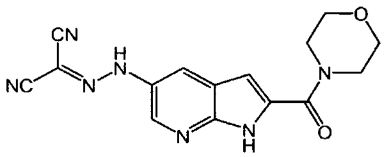 |
| 49 | 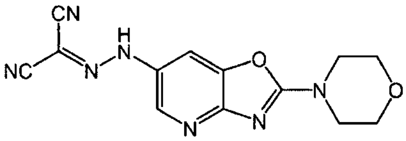 | | |

In the disclosure, the term "substitution" refers to a reaction in which an atom or atomic group included in a molecule of a compound is replaced with another atom or atomic group.

In the disclosure, the term "cyclo or ring" refers to a structure in which both ends of the skeleton of an organic compound are connected to form a ring. The present inventors have confirmed that when the heteroaryl side terminal in the heteroaryl-carbonohydrazonoyl-dicyanide structure is substituted with a saturated heterocyclyl, there is an excellent effect of inhibiting aggregation and hyperphosphorylation of tau protein and/or TDP-43.

In the disclosure, the term "chain or cyclic alkyl group" means a monovalent linear or branched or cyclic saturated hydrocarbon residue consisting solely of carbon and hydrogen atoms, having 1 to 20 carbon atoms. Examples of these alkyl groups include, but are not limited to, methyl, ethyl, propyl, isopropyl, butyl, isobutyl, 2-butyl, 3-butyl, pentyl, n-hexyl, cyclobutyl group, cyclopentyl group, cyclohexyl group, and the like.

In the disclosure, the term "heterocycloalkyl group" typically refers to a saturated or unsaturated (but not aromatic) cyclohydrocarbon, which may be optionally unsubstituted, monosubstituted or polysubstituted, and in the structure of which at least one is selected from a heteroatom of N, O or S.

In the disclosure, the term "aryl group" means an unsaturated aromatic ring compound having 3 to 12 carbon atoms having a single ring (e.g., phenyl) or multiple condensed rings (e.g., naphthyl). Examples of such aryl groups include, but are not limited to, phenyl, naphthyl, and the like.

In the disclosure, the term "heteroaryl group" refers to a single ring or multiple condensed rings in which at least one of the atoms constituting the ring has a heteroatom of N, O or S. Examples of such heteroaryl groups include, but are not limited to, a pyridyl group, a pyrimidinyl group, a pyrazinyl group, an oxazolyl group, a furyl group, and a thiophenyl group.

In the disclosure, "halogen group" may be fluorine (F), chloride (Cl), bromine (Br), or iodine (I), and in the disclosure, "carbonyl group" means a -C(=O)- group.

In addition, the compound of the disclosure may exist in the form of a pharmaceutically acceptable salt. Among the salts, an acid addition salt formed by a pharmaceutically acceptable free acid is useful. The term "pharmaceutically acceptable salt" of the disclosure means any and all organic or inorganic addition salts of the compound represented by Formulas 1 to 1-2 that are relatively non-toxic and harmless to patients at concentrations having an effective action, and in which side effects attributable to the salt do not impair the beneficial efficacy of the compound represented by Formulas 1 to 1-2.

The acid addition salt is prepared by a conventional method, for example, by dissolving the compound in an excess aqueous acid solution and precipitating the salt using a water-miscible organic solvent, for example, methanol, ethanol, acetone, or acetonitrile. Equal molar amounts of the compound and an acid or alcohol (e.g., glycol monomethyl ether) in water are heated, and then the mixture is evaporated to dryness, or the precipitated salt may be suction filtered.

At this time, organic acids and inorganic acids can be used as free acids, and inorganic acids such as hydrochloric acid, phosphoric acid, sulfuric acid, nitric acid, and tartaric acid can be used, and organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, and hydroiodic acid can be used, but are not limited thereto.

In addition, a pharmaceutically acceptable metal salt may be made using a base. An alkali metal salt or alkaline earth metal salt is obtained, for example, by dissolving the compound in an excess alkali metal hydroxide or alkaline earth metal hydroxide solution, filtering the undissolved compound salt, and then evaporating and drying the filtrate. At this time, as the metal salt, it is pharmaceutically suitable to prepare a sodium, potassium, or calcium salt, in particular, but is not limited thereto. In addition, a corresponding silver salt thereof may be obtained by reacting an alkali metal or alkaline earth metal salt with a suitable silver salt (e.g., silver nitrate).

The pharmaceutically acceptable salt of the compound of the disclosure includes, unless otherwise indicated, salts of acidic or basic groups that may be present in the compounds of Formulas 1 to 1-2. For example, pharmaceutically acceptable salts may include sodium, calcium, and potassium salts of a hydroxy group, and other pharmaceutically acceptable salts of an amino group include hydrobromide, sulfate, hydrogen sulfate, phosphate, hydrogen phosphate, dihydrogen phosphate, acetate, succinate, citrate, tartrate, lactate, mandelate, methanesulfonate (mesylate), and p-toluenesulfonate (tosylate) salts, and may be prepared through a method of preparing a salt known in the art.

As the salt of the compound of Formulas 1 to 1-2 of the disclosure, any salt of the compound of Formulas 1 to 1-2 may be used without limitation as long as it is a pharmaceutically acceptable salt and exhibits pharmacological activity equivalent to that of the compound of Formulas 1 to 1-2, for example, inhibiting aggregation and/or hyperphosphorylation of tau protein or TDP-43 protein.

In addition, the compound represented by Formulas 1 to 1-2 according to the disclosure includes, without limitation, not only pharmaceutically acceptable salts thereof, but also solvates such as possible hydrates that may be prepared therefrom, and all possible stereoisomers. Solvates and stereoisomers of the compound represented by Formulas 1 to 1-2 may be prepared from the compound represented by Formulas 1 to 1-2 using methods known in the art.

Furthermore, the compound represented by Formulas 1 to 1-2 according to the disclosure may be prepared in a crystalline form or an amorphous form, and when prepared in a crystalline form, may be optionally hydrated or solvated. The disclosure may include not only stoichiometric hydrates of the compound represented by Formulas 1 to 1-2, but also compounds containing various amounts of water. Solvates of the compound represented by Formulas 1 to 1-2 according to the disclosure include both stoichiometric solvates and non-stoichiometric solvates.

According to another embodiment of the disclosure, there is provided a method of preparing a compound represented by Formula 1, including a first step of forming an imine bond by reacting a compound represented by Formula 2, which includes a reactive amine group at one terminal, with sodium nitrite and malononitrile in the presence of an acid; and

optionally, when $R_1$ is a substituent other than hydrogen, further including a second step of introducing an $R_1$ substituent into a product obtained from the first step:

[Formula 1]

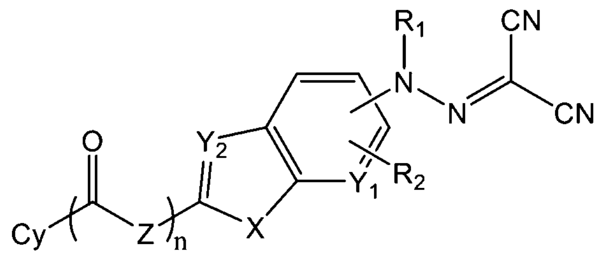

[Formula 2]

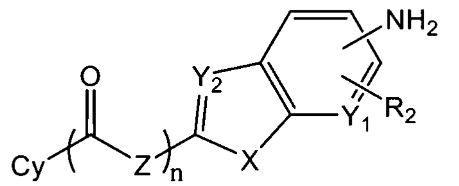

In Formulas 1 and 2,

n is be 0 or 1;
$R_1$ is any one selected from hydrogen, $C_{1-4}$ alkyl, or $C_{1-4}$ alkylcarbonyl;
$R_2$ is absent or halogen;
X is any one selected from O, S or $NR_3$;
$R_3$ is hydrogen, or $C_{1-4}$ alkyl;
$Y_1$ and $Y_2$ are C or N;
Z is absent or NH; and
Cy is a 5- to 10-membered saturated heterocyclyl which is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.

Specifically, the first step of the process may be performed by, but is not limited to, a series of processes including:

a step 1-1 of dissolving the compound of Formula 2 and sodium nitrite in a water-containing solvent, and adding an aqueous acid solution at -5 to 5°C to form a diazonium salt,
a step 1-2 of adding malononitrile to a reaction solution containing the diazonium salt obtained from step 1-1 and reacting at 15 to 40°C, and
a step 1-3 of adding an aqueous base solution to the reaction solution of step 1-2 to neutralize it.

In addition, the first step may be performed by, but is not limited to, performing the reaction of step 1-1 while stirring for 2 minutes to 1 hour at a low temperature of about 0°C using a 1 M hydrochloric acid solution in an aqueous solution, and then stirring at room temperature for 2 minutes to 1 hour to perform the reaction of step 1-2.

In addition, the second step may be performed by, but is not limited to, i) when $R_1$ is $C_{1-6}$ alkyl, reacting with a halogenated alkane in the presence of potassium tert-butoxide or sodium hydride in an organic solvent, and ii) when $R_1$ is $C_{1-4}$ alkylcarbonyl, reacting with a halogenated alkylcarbonyl in the presence of a base and triethylamine.

Specifically, when $R_1$ is $C_{1-6}$ alkyl, the carbonohydrazonoyl dicyanide compound in which $R_1$ is not substituted, obtained from the previous step, is dissolved in an organic solvent such as DMF, and a haloalkane corresponding to $R_1$, for example, an alkane iodide, is added to the reaction solution and reacted at 50 to 70°C, and the reaction solution may further include potassium tert-butoxide, but is not limited thereto.

In addition, when $R_1$ is $C_{1-6}$ alkylcarbonyl, the carbonohydrazonoyl dicyanide compound in which $R_1$ is not substituted, obtained from the previous step, is dissolved in a lower alcohol such as methanol and reacted with a base such as potassium hydroxide, and then the product obtained by solidifying is reacted with a halogenated alkylcarbonyl corresponding to $C_{1-6}$ alkylcarbonyl, for example, acetyl chloride, in the presence of triethylamine in an organic solvent such as acetonitrile, but is not limited thereto.

According to an aspect,
the compound represented by Formula 2 may be obtained by:

i) when n is 0 and X is S or $NR_3$, reacting Cy-H with a compound represented by Formula 3-a under microwave irradiation,
ii) when n is 0 and X is O, reacting Cy-H with a compound represented by Formula 3-b in the presence of triethylamine,
iii) when n is 1, reacting Cy-H with a compound represented by Formula 3-c, and when $R_N$ is nitro, further reducing the reaction product, and
iv) when $Y_2$ is N, n is 1, and Z is NH, reacting with a compound represented by 3-d:

[Formula 3-a]

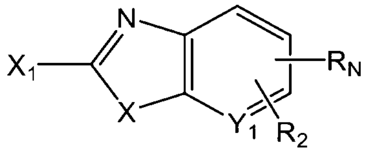

[Formula 3-b]

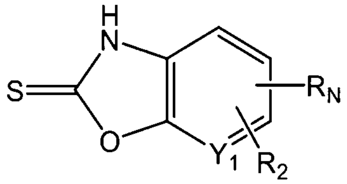

[Formula 3-c]

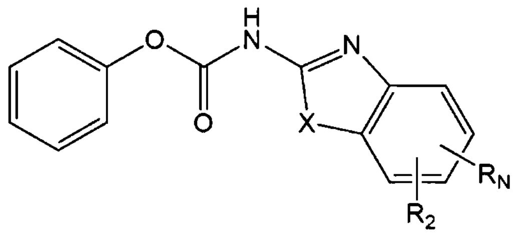

[Formula 3-d]

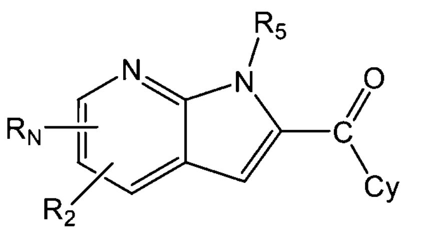

In Formulas 3-a to 3-d,

$X_1$ is halogen, $R_N$ is nitro or amino,
$R_5$ is any one selected from t-butoxycarbonyl, hydrogen, or benzyloxycarbonyl; and
Cy is a 5- to 10-membered saturated heterocyclyl which is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.

Preferably, the reduction may be performed by, but is not limited to, reacting in an organic solvent such as 1,4-dioxane or methanol under a Pd/C catalyst, reacting with AcOH in the presence of Fe, or reacting with ammonium chloride in the presence of Fe.

According to an aspect, in the compound represented by Formula 3-a, when X is $NR_3$ and $R_3$ is $C_{1-4}$ alkyl, the compound may be prepared by preparing a compound in which $R_3$ is hydrogen according to step i) and reacting the compound with halogenated $R_3$ in the presence of DBU (1,8-Diazabicyclo[5.4.0]undec-7-ene), and may be preferably performed under microwave irradiation, but is not limited thereto.

According to an aspect, the compound represented by Formula 3-b may be prepared by reacting a compound represented by Formula 4 with potassium ethyl xanthogenate, and may be preferably performed at 100 to 125°°C in a state of being dissolved in an organic solvent such as pyridine, but is not limited thereto:

[Formula 4]

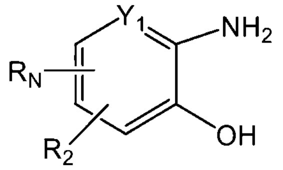

.

According to an aspect, the compound represented by Formula 3-c may be prepared by reacting a compound represented by Formula 5 with phenyl chloroformate in the presence of triethylamine, and may be preferably performed in an organic solvent such as dichloromethane, dimethylformamide, or the like, alone or in a mixed solvent, but is not limited thereto:

[Formula 5]

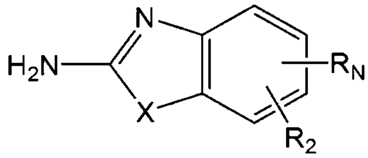

.

In the preparing method of the disclosure, the reactants and/or precursors used in each step may be purchased as commercially available compounds, or the reactants or intermediates of each step may be purchased and synthesized through a single reaction known in the art or by combining several reactions, but are not limited thereto.

Additionally, after each reaction, a process for isolating and/or purifying the product may be additionally included, as needed, and this can be performed using various methods known in the art.

According to another aspect of the disclosure, a pharmaceutical composition for preventing or treating a disease caused by aggregation or hyperphosphorylation of tau protein or TDP-43 protein, including the above-described compound as an active ingredient, is provided.

In the disclosure, the term "prevention" means any act of inhibiting or delaying the occurrence, spread or recurrence of a neurological disease by administering the composition of the disclosure, and "treatment" means any act of improving or beneficially changing the symptoms of the disease by administering the composition of the disclosure.

In the disclosure, the term "pharmaceutical composition" means a composition manufactured for the purpose of preventing or treating the above-described disease, and can be formulated and used in various forms according to conventional methods. For example, the pharmaceutical composition can be formulated into oral dosage forms such as powder, granules, tablets, capsules, suspensions, emulsions, and syrups, and can be formulated and used in the form of external preparations, suppositories, and sterile injectable solutions.

In the disclosure, "including as an active ingredient" means that the corresponding component is included in an amount necessary or sufficient to realize a desired biological effect. In actual application, the determination of the amount included as an active ingredient is an amount for treating a target disease, and may be determined by considering factors that do not cause other toxicity, and may vary according to various factors such as, for example, the disease or condition being treated, the form of the composition being administered, the size of the subject, or the severity of the disease or condition. A person skilled in the art to which the disclosure pertains can empirically determine the effective amount of an individual composition without undue experimentation.

In the term "tau protein" of the disclosure, tau is an abbreviation for 'tubulin associated unit', and the tau protein is one of the well-known microtubule-binding proteins, and is a group of six highly soluble protein isoforms generated by alternative splicing from the MAPT (microtubule-associated protein tau) gene. The tau protein mainly plays a role in maintaining the stability of microtubules in axons, and is abundantly present in neurons of the central nervous system (CNS), where the cerebral cortex is the most abundant. The tau protein is less common in other parts, but is also expressed at very low levels in CNS astrocytes and oligodendrocytes.

The term "TDP-43 protein" of the disclosure, also referred to as 'transactive response DNA binding protein 43 kDa' or 'TAR DNA-binding protein 43', is a transcriptional repressor that binds to chromosomally integrated TAR DNA and inhibits HIV-1 transcription. In addition, the protein regulates alternate splicing of the CFTR gene. The TDP-43 protein binds to both DNA and RNA, and has various functions in transcriptional repression, pre-mRNA splicing, and translational regulation.

As described above, since the compound of the disclosure not only inhibits aggregation or hyperphosphorylation of tau protein and/or TDP-43 protein but also does not exhibit toxicity to cells, a pharmaceutical composition including the

same as an active ingredient may be used for preventing or treating a disease caused by aggregation or hyperphosphorylation of tau protein or TDP-43 protein.

For example, the disease caused by aggregation or hyperphosphorylation of tau protein to which the pharmaceutical composition of the disclosure may be applied may be Alzheimer's disease, Parkinson's disease, vascular dementia, acute stroke, trauma, cerebrovascular disease, brain cord trauma, spinal cord trauma, peripheral neuropathy, retinopathy, glaucoma, or tauopathies. Non-limiting examples of the tauopathy include chronic traumatic encephalopathy (CTE), primary age-related tauopathy, progressive supranuclear palsy, corticobasal degeneration, Pick's disease, argyrophilic grain disease (AGD), frontotemporal dementia (FTD), Parkinsonism linked to chromosome 17, Lytico-Bodig disease (Parkinson-dementia complex of Guam), ganglioglioma, gangliocytoma, meningioangiomatosis, postencephalitic parkinsonism, subacute sclerosing panencephalitis, lead encephalopathy, tuberous sclerosis, Pantothenate kinase-associated neurodegeneration, lipofuscinosis, posttraumatic stress disorder, and traumatic brain injury.

In addition, the disease caused by aggregation or hyperphosphorylation of TDP-43 protein to which the pharmaceutical composition of the disclosure may be applied includes amyotrophic lateral sclerosis (ALS), Familial ALS-SOD1, sporadic amyotrophic lateral sclerosis, superoxide dismutase 1 (SOD1), frontotemporal lobar degeneration (FTLD), ALS-FTLD, Multiple system proteinopathy (MSP), limbic-predominant age-related TDP-43 encephalopathy (LATE)/CART (cerebral age-related TDP-43 with sclerosis), Perry disease, facial onset sensory and motor neuronopathy (FOSMN), sporadic inclusion body myositis (sIBM), and the like.

In addition, the pharmaceutical composition of the disclosure may, depending on each formulation, additionally contain one or more pharmaceutically acceptable carriers in addition to the active ingredients described above.

The pharmaceutically acceptable carrier may be saline solution, sterile water, Ringer's solution, buffered saline, dextrose solution, maltodextrin solution, glycerol, ethanol, and mixtures of one or more of these components, and may further include other conventional additives such as antioxidants, buffers, and bacteriostatic agents, as needed. In addition, diluents, dispersants, surfactants, binders and lubricants may be additionally added to formulate the composition into injectable formulations such as aqueous solutions, suspensions and emulsions, pills, capsules, granules or tablets. Furthermore, the composition may be preferably formulated for each disease or ingredient using an appropriate method in the art or a method disclosed in Remington's Pharmaceutical Science (Mack Publishing Company, Easton PA).

The composition of the disclosure may be administered orally or parenterally in a pharmaceutically effective amount according to a desired method, and the term "pharmaceutically effective amount" of the disclosure means an amount that is sufficient to treat a disease with a reasonable benefit/risk ratio applicable to medical treatment and does not cause side effects, and the effective dose level may be determined according to factors including the patient's health condition, severity, activity of the drug, sensitivity to the drug, administration method, administration time, administration route and excretion rate, treatment period, drugs used in combination or concurrently, and other factors well known in the medical field.

Therefore, the pharmaceutical composition of the disclosure may be administered to a subject to prevent, treat, and/or diagnose diseases associated with aggregation or hyperphosphorylation of tau protein or TDP-43, examples of which are as described above.

In the disclosure, the term "subject" is not limited to a mammal such as livestock or a human that requires prevention, treatment, and/or diagnosis of the above-described exemplary disease, but may preferably be a human.

The pharmaceutical composition of the disclosure may be formulated in various forms for administration to a subject, and a representative form for parenteral administration is an injectable form, preferably an isotonic aqueous solution or suspension.

The formulation for parenteral administration may be prepared by conventional mixing, granulating or coating methods and may contain the active ingredient in an amount of about 0.1 to 75 wt%, preferably about 1 to 50 wt%. A unit dosage form for a mammal weighing about 50 to 70 kg may contain about 10 to 200 mg of active ingredient. Injectable formulations can be prepared according to techniques known in the art using suitable dispersing or wetting agents and suspending agents. For example, each component can be dissolved in saline or a buffer solution and formulated for injection. In addition, formulations for oral administration include, for example, ingestible tablets, buccal tablets, troches, capsules, elixirs, suspensions, syrups, and wafers, and these formulations may contain, in addition to active ingredients, diluents (e.g., lactose, dextrose, sucrose, mannitol, sorbitol, cellulose and/or glycine) and lubricants (e.g., silica, talc, stearic acid and its magnesium or calcium salts and/or polyethylene glycol). The tablets may contain binding agents such as magnesium aluminum silicate, starch paste, gelatin, tragacanth, methylcellulose, sodium carboxymethylcellulose and/or polyvinylpyrrolidine, and may optionally further contain disintegrants such as starch, agar, alginic acid or its sodium salt, absorbents, colorants, flavoring agents and/or sweetening agents. The formulations may be prepared by conventional mixing, granulation or coating methods.

In addition, the pharmaceutical composition of the disclosure may further include auxiliary agents such as preservatives, wetting agents, emulsifying agents, salts for osmotic pressure control or buffers, and other therapeutically useful substances, and may be formulated according to conventional methods.

The pharmaceutical composition according to the disclosure may be administered through various routes

including oral, transdermal, subcutaneous, intravenous or intramuscular routes, and the dosage of the active ingredient may be appropriately selected according to various factors such as the route of administration, the patient's age, sex, weight, and the severity of the patient's condition. In addition, the composition of the disclosure may be administered in combination with known compounds that can enhance the desired effect.

As routes of administration for the pharmaceutical composition according to the disclosure, the pharmaceutical composition may be administered to humans and animals orally or parenterally such as intravenously, subcutaneously, intranasally, or intraperitoneally. Oral administration also includes sublingual application. Parenteral administration includes injection methods such as subcutaneous injection, intramuscular injection, and intravenous injection, and instillation methods.

In the pharmaceutical composition of the disclosure, the total effective amount of the heteroaryl-carbonohydrazonoyl-dicyanide compound or pharmaceutically acceptable salt thereof according to the disclosure may be administered to a patient as a single dose, or may be administered by a fractionated treatment protocol in which a multiple dose is administered over a long period of time. The pharmaceutical composition of the disclosure may vary the content of the active ingredient depending on the severity of the disease, but may typically be administered several times a day at an effective dose of 100 $\mu$g to 3,000 mg per administration for adults. However, the concentration of the heteroaryl-carbonohydrazonoyl-dicyanide compound or pharmaceutically acceptable salt thereof may be determined in consideration of various factors such as the route of administration and the number of treatments, as well as the age, weight, health condition, sex, severity of disease, diet, and excretion rate of the patient.

In addition, the pharmaceutical composition according to the disclosure is not particularly limited in its formulation, route of administration, and method of administration as long as the effect of the disclosure is exhibited, and the pharmaceutical composition of the disclosure may further include a known drug in addition to the heteroaryl-carbonohydrazonoyl dicyanide compound or pharmaceutically acceptable salt thereof as an active ingredient, and may be used in combination with other known treatments for treating these diseases.

According to an aspect, the composition may inhibit aggregation of tau protein or TDP-43 protein.

According to an aspect, the composition may inhibit hyperphosphorylation of tau protein or TDP-43 protein.

As another aspect of the disclosure, there is provided a method of preventing or treating a disease caused by aggregation or hyperphosphorylation of tau protein or TDP-43 protein, including administering the pharmaceutical composition of the disclosure to a subject in need thereof.

By administering the pharmaceutical composition of the disclosure to a subject, the disease may be effectively prevented or treated. In addition, since the pharmaceutical composition of the disclosure exhibits a therapeutic effect by inhibiting aggregation or hyperphosphorylation of tau protein or TDP-43 protein, a synergistic effect may be exhibited by administering it in combination with existing therapeutic agents.

The term "administration" of the disclosure means providing a predetermined substance to a patient by any appropriate method, and the administration route of the composition of the disclosure may be through any general route as long as it reaches the target tissue. Administration may be intraperitoneal administration, intravenous administration, intramuscular administration, subcutaneous administration, intradermal administration, oral administration, topical administration, intranasal administration, intrapulmonary administration, or intrarectal administration, but is not limited thereto. In addition, the pharmaceutical composition of the disclosure may be administered by any device capable of transporting the active substance to target cells. Preferred modes of administration and formulations are intravenous injections, subcutaneous injections, intradermal injections, intramuscular injections, drip injections, and the like. Injections may be prepared using aqueous solvents such as physiological saline and Ringer's solution, non-aqueous solvents such as vegetable oils, higher fatty acid esters (e.g., ethyl oleate, etc.), alcohols (e.g., ethanol, benzyl alcohol, propylene glycol, glycerin, etc.), and the like, and may include pharmaceutical carriers such as stabilizers for preventing deterioration (e.g., ascorbic acid, sodium hydrogen sulfite, sodium pyrosulfite, BHA, tocopherol, EDTA, etc.), emulsifiers, buffers for pH adjustment, and preservatives for inhibiting microbial growth (e.g., phenylmercuric nitrate, thimerosal, benzalkonium chloride, phenol, cresol, benzyl alcohol, etc.).

The terminology used herein is for the purpose of describing particular embodiments only and is not to be limiting of the embodiments. The singular form is intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

The present disclosure can be modified in various ways and has various embodiments. Specific embodiments

are illustrated in the drawings and described in detail in the detailed description below. However, this is not intended to limit the disclosure to specific embodiments, but should be understood to include all changes, equivalents, or substitutes included in the idea and technical scope of the disclosure. In the description of the disclosure, detailed description of well-known related technology will be omitted when it is deemed that such description will cause ambiguous interpretation of the disclosure.

Hereinafter, the disclosure will be described in more detail with reference to examples. These examples are merely intended for the purpose of describing the disclosure in more detail, and thus, the scope of the disclosure is not limited to the examples.

BEST MODE FOR CARRYING OUT THE INVENTION

**Example 1: Preparation of (2-morpholinobenzo[d]thiazol-5-yl)carbonohydrazonoyl dicyanide (Compound 1)**

**Step 1-1: Preparation of 2-morpholinobenzo[d]thiazol-5-amine**

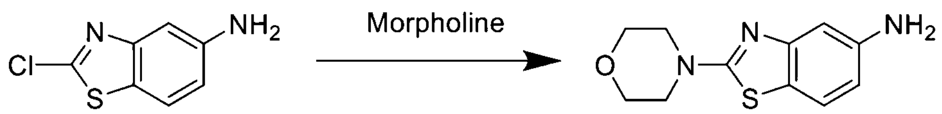


**1-1**

2-Chlorobenzo[d]thiazol-5-amine (300 mg, 1.62 mmol) was dissolved in morpholine (3 mL) and reacted at 180°C for 10 minutes under microwave irradiation. Thereafter, distilled water was added and the mixture was stirred at room temperature for 1 hour, and then filtered to obtain 311 mg of the title compound (81% yield).

$^1$H NMR (400 MHz, DMSO-$d_6$) δ 7.34 (d, *J* = 8.4 Hz, 1H), 6.69 (d, *J* = 2.2 Hz, 1H), 6.43 - 6.34 (m, 1H), 5.00 (s, 2H), 3.71 (t, *J* = 4.9 Hz, 4H), 3.47 (t, *J* = 4.9 Hz, 4H).

**Step 1-2: Preparation of (2-morpholinobenzo[d]thiazol-5-yl)carbonohydrazonoyl dicyanide**

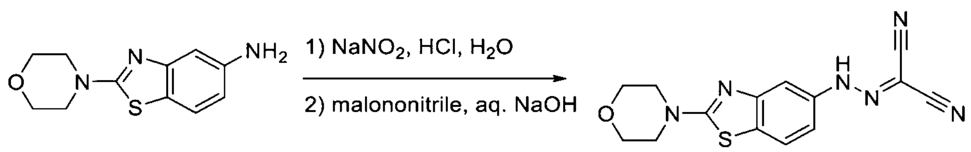


**1-1**

**1**

2-Morpholinobenzo[d]thiazol-5-amine (300 mg, 1.27 mmol) and sodium nitrite (132 mg, 1.91 mmol) prepared according to step 1-1 were dissolved in distilled water, and then 1.0 M hydrochloric acid aqueous solution (3.8 mL, 3.82 mmol) was added at 0°C. The reaction mixture was stirred at 0°C for 10 minutes to form a diazonium salt. Malononitrile (168 mg, 2.55 mmol) was added to the reaction mixture containing the diazonium salt, and the mixture was stirred at room temperature for 10 minutes. Thereafter, the pH of the reaction solution was adjusted to 5.0 with an aqueous sodium hydroxide solution, and then the mixture was stirred at room temperature for 1 hour. When the reaction was completed, the produced solid was filtered to obtain 362 mg of the title compound (91% yield).

$^1$H NMR (400 MHz, DMSO-$d_6$) δ 13.03 (s, 1H), 7.80 (d, *J* = 8.6 Hz, 1H), 7.51 (d, *J* = 2.1 Hz, 1H), 7.23 (dd, *J* = 8.6, 2.2 Hz, 1H), 3.73 (t, *J* = 4.9 Hz, 4H), 3.56 (t, *J* = 4.8 Hz, 4H).

**Example 2: Preparation of (2-morpholinobenzo[d]thiazol-6-yl)carbonohydrazonoyl dicyanide (Compound 2)**

**Step 2-1: Preparation of 2-morpholinobenzo[d]thiazol-5-amine**

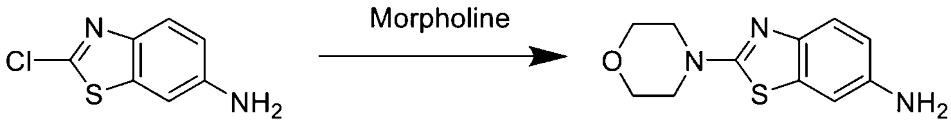


2-1

The reaction was performed in a similar manner to step 1-1 of Example 1, except that 2-chlorobenzo[d]thiazol-6-amine (300 mg, 1.62 mmol) was used in place of 2-chlorobenzo[d]thiazol-5-amine, to obtain 338 mg of the title compound (88% yield).

$^1H$ NMR (400 MHz, DMSO-$d_6$) δ 7.19 (d, $J$ = 8.5 Hz, 1H), 6.91 (d, $J$= 2.3 Hz, 1H), 6.57 (dd, $J$ = 8.5, 2.3 Hz, 1H), 4.92 (s, 2H), 3.74 - 3.67 (m, 4H), 3.45 - 3.38 (m, 4H).

**Step 2-2: Preparation of (2-morpholinobenzo[d]thiazol-6-yl)carbonohydrazonoyl dicyanide**

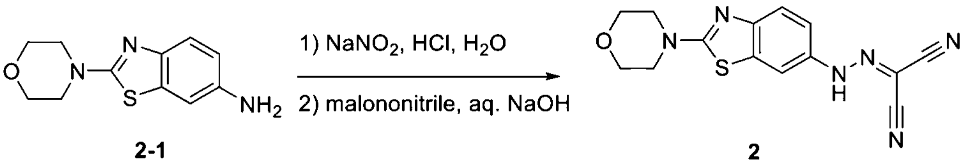


2-1 2

The reaction was performed in a similar manner to step 1-2 of Example 1, except that 2-morpholinobenzo[d]thiazol-6-amine (300 mg, 1.27 mmol) obtained from step 2-1 was used in place of 2-morpholinobenzo[d]thiazol-5-amine, to obtain 219 mg of the title compound (55% yield).

$^1H$ NMR (400 MHz, DMSO-$d_6$) δ 13.10 (s, 1H), 7.92 (d, $J$= 2.1 Hz, 1H), 7.51 - 7.40 (m, 2H), 3.72 (t, $J$ = 4.9 Hz, 4H), 3.55 (t, $J$ = 4.9 Hz, 4H).

**Example 3: Preparation of (2-morpholinobenzo[d]oxazol-5-yl)carbonohydrazonoyl dicyanide (Compound 3)**

**Step 3-1: Preparation of 5-nitrobenzo[d]oxazole-2(3H)-thione**

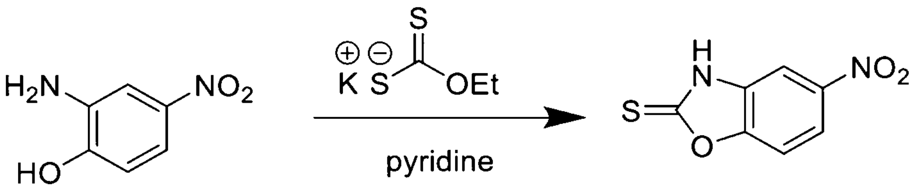


3-1

2-Amino-4-nitrophenol (1 g, 6.49 mmol) was dissolved in pyridine, and potassium ethyl xanthogenate (1.14 g, 7.14 mmol) was added, and the mixture was stirred at 120°C for 8 hours. 2 N HCl was added to the reaction mixture at room temperature until a solid reactant was produced, and then the mixture was stirred for 30 minutes, and thereafter filtered with distilled water to obtain 528 mg of the title compound (41% yield).

$^1H$ NMR (400 MHz, DMSO-$d_6$) δ 8.19 (dq, $J$ = 8.9, 2.3 Hz, 1H), 7.94 (d, $J$ = 2.0 Hz, 1H), 7.73 (dd, $J$ = 9.1, 3.3 Hz, 1H).

**Step 3-2: Preparation of 2-morpholino-5-nitrobenzo[d]oxazole**

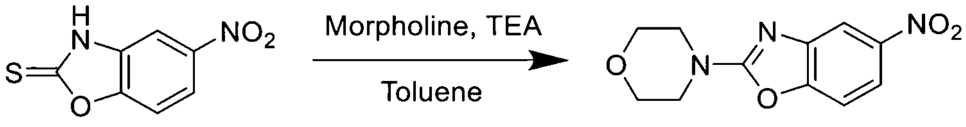


3-1 3-2

5-Nitrobenzo[d]oxazole-2(3H)-thione (100 mg, 0.51 mmol) prepared according to step 3-1 was dissolved in toluene, and morpholine (53 μL, 0.61 mmol) and triethylamine (213 μL, 1.53 mmol) were added, and the mixture was stirred at 150°C for 12 hours. When the reaction was completed, the mixture was extracted with distilled water and ethyl acetate, and the organic layer was dried over anhydrous magnesium sulfate and then filtered. The filtrate was concentrated under reduced pressure, solidified with hexane and ether, and then filtered to obtain 45 mg of the title compound (35% yield).

$^1$H NMR (400 MHz, DMSO-$d_6$) δ 8.09 (d, *J* = 2.3 Hz, 1H), 7.99 (dd, *J* = 8.8, 2.4 Hz, 1H), 7.66 (d, *J* = 8.8 Hz, 1H), 3.78 - 3.70 (m, 4H), 3.71 - 3.61 (m, 4H).

**Step 3-3: Preparation of 2-morpholinobenzo[d]oxazol-5-amine**

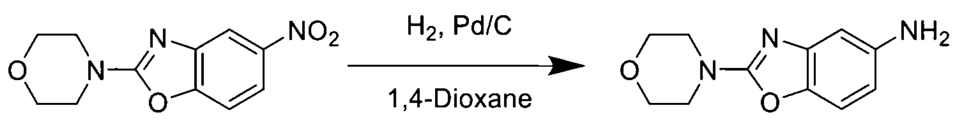


3-2 3-3

2-Morpholino-5-nitrobenzo[d]oxazole (150 mg, 0.60 mmol) and 10% Pd/C (128 mg, 0.12 mmol) prepared according to step 3-2 were dissolved in 1,4-dioxane and stirred at room temperature for 4 hours under hydrogen. When the reaction was completed, the reaction mixture was filtered, and then the filtrate was concentrated under reduced pressure to obtain 83 mg of the title compound (63% yield).

$^1$H NMR (400 MHz, DMSO-$d_6$) δ 7.03 (d, *J* = 8.4 Hz, 1H), 6.50 (d, *J* = 2.2 Hz, 1H), 6.25 (dd, *J* = 8.5, 2.2 Hz, 1H), 4.79 (s, 2H), 3.72 - 3.66 (m, 4H), 3.54 - 3.48 (m, 4H).

**Step 3-4: Preparation of (2-morpholinobenzo[d]oxazol-5-yl)carbonohydrazonoyl dicyanide**

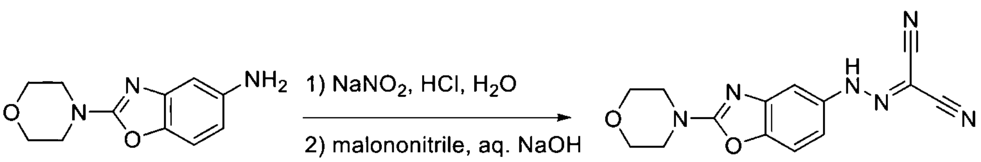


3-3 3

The reaction was performed in a similar manner to step 1-2 of Example 1, except that 2-morpholinobenzo[d]oxazol-5-amine (60 mg, 0.27 mmol) obtained from step 3-3 was used in place of 2-morpholinobenzo[d]thiazol-5-amine, to obtain 62 mg of the title compound (85% yield).

$^1$H NMR (400 MHz, DMSO-$d_6$) δ 13.01 (s, 1H), 7.44 (d, *J* = 8.6 Hz, 1H), 7.35 (d, *J* = 2.2 Hz, 1H), 7.17 (dd, *J* = 8.6, 2.2 Hz, 1H), 3.75 - 3.68 (m, 5H), 3.63 - 3.56 (m, 3H).

**Example 4: Preparation of (2-morpholinobenzo[d]oxazol-6-yl)carbonohydrazonoyl dicyanide (Compound 4)**

**Step 4-1: Preparation of 6-nitrobenzo[d]oxazole-2(3H)-thione**

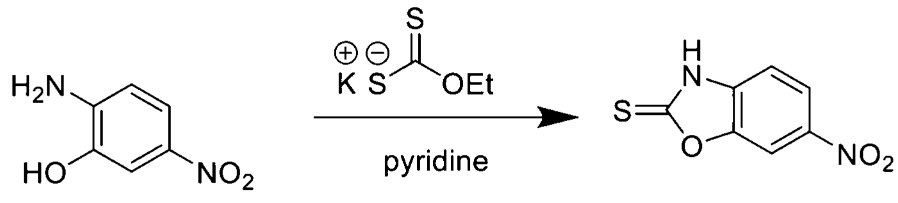


4-1

The reaction was performed in a similar manner to step 3-1 of Example 3, except that 2-amino-5-nitrophenol (1 g, 6.49 mmol) was used in place of 2-amino-4-nitrophenol, to obtain 373 mg of the title compound (29% yield).

$^1H$ NMR (400 MHz, DMSO-$d_6$) δ 14.37 (s, 1H), 8.43 (d, $J$ = 2.2 Hz, 1H), 8.23 (dd, $J$ = 8.7, 2.2 Hz, 1H), 7.41 (d, $J$ = 8.7 Hz, 1H).

**Step 4-2: Preparation of 2-morpholino-6-nitrobenzo[d]oxazole**

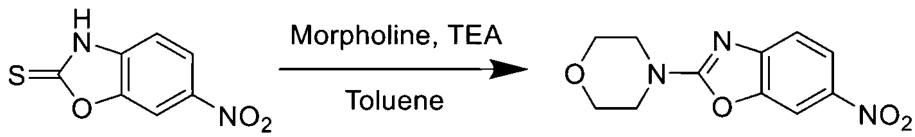


4-1 4-2

The reaction was performed in a similar manner to step 3-2 of Example 3, except that 6-nitrobenzo[d]oxazole-2(3H)-thione (350 mg, 1.78 mmol) obtained from step 4-1 was used in place of 5-nitrobenzo[d]oxazole-2(3H)-thione, to obtain 220 mg of the title compound (49% yield).

$^1H$ NMR (400 MHz, DMSO-$d_6$) δ 8.31 (d, $J$ = 2.3 Hz, 1H), 8.16 (dd, $J$ = 8.7, 2.3 Hz, 1H), 7.42 (d, $J$ = 8.7 Hz, 1H), 3.78 - 3.71 (m, 4H), 3.71 - 3.66 (m, 4H).

**Step 4-3: Preparation of 2-morpholinobenzo[d]oxazol-6-amine**

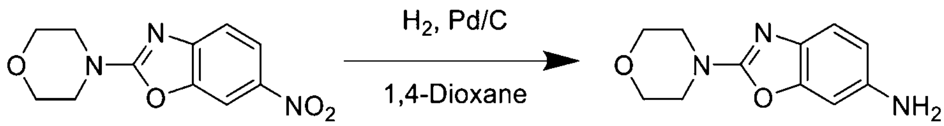


4-2 4-3

The reaction was performed in a similar manner to step 3-3 of Example 3, except that 2-morpholino-6-nitrobenzo[d]oxazole (200 mg, 0.80 mmol) obtained from step 4-2 was used in place of 2-morpholino-5-nitrobenzo[d]oxazole, to obtain 130 mg of the title compound (74% yield).

$^1H$ NMR (400 MHz, DMSO-$d_6$) δ 6.98 (d, $J$ = 8.3 Hz, 1H), 6.63 (d, $J$ = 2.0 Hz, 1H), 6.42 (dd, $J$ = 8.3, 2.1 Hz, 1H), 4.90 (s, 2H), 3.69 (t, $J$ = 4.8 Hz, 4H), 3.46 (t, $J$ = 4.8 Hz, 4H).

**Step 4-4: Preparation of (2-morpholinobenzo[d]oxazol-6-yl)carbonohydrazonoyl dicyanide**

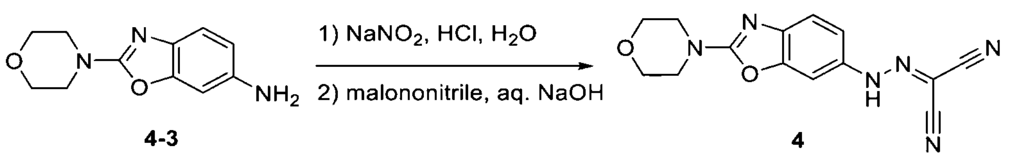


4-3 4

The reaction was performed in a similar manner to step 1-2 of Example 1, except that 2-morpholinobenzo[d]oxazol-6-amine (100 mg, 0.46 mmol) obtained from step 4-3 was used in place of 2-morpholinobenzo[d]thiazol-5-amine, to obtain 116 mg of the title compound (94% yield).

$^1H$ NMR (400 MHz, DMSO-$d_6$) δ 13.08 (s, 1H), 7.51 (d, *J* = 1.8 Hz, 1H), 7.39 - 7.27 (m, 2H), 3.76 - 3.68 (m, 4H), 3.63 - 3.53 (m, 4H).

**Example 5: Preparation of (1-methyl-2-morpholino-1H-benzo[d]imidazol-6-yl)carbonohydrazonoyl dicyanide (Compound 5)**

**Step 5-1: Preparation of 4-(6-nitro-1H-benzo[d]imidazol-2-yl)morpholine**

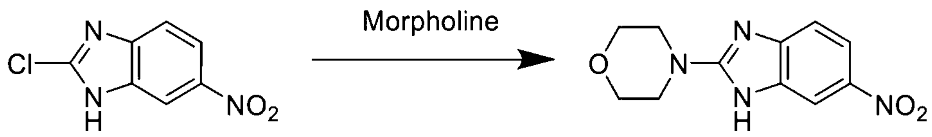


**5-1**

2-Chloro-6-nitro-1H-benzo[d]imidazole (500 mg, 2.53 mmol) was dissolved in morpholine (6 mL) and reacted at 180°C for 10 minutes under microwave irradiation. When the reaction was completed, the mixture was extracted with distilled water and ethyl acetate, and the organic layer was dried over anhydrous magnesium sulfate and then filtered. The filtrate was concentrated under reduced pressure, solidified with ether, and then filtered to obtain 379 mg of the title compound (60% yield).

$^1H$ NMR (400 MHz, DMSO-$d_6$) δ 12.03 (s, 1H), 7.99 (d, *J* = 2.3 Hz, 1H), 7.94 (dd, *J*= 8.7, 2.3 Hz, 1H), 7.31 (d, *J* = 8.7 Hz, 1H), 3.79 - 3.70 (m, 4H), 3.62 - 3.51 (m, 4H).

**Step 5-2: Preparation of 4-(1-methyl-6-nitro-1H-benzo[d]imidazol-2-yl)morpholine (5-2-1) and 4-(1-methyl-5-nitro-1H-benzo[d]imidazol-2-yl)morpholine (5-2-2)**

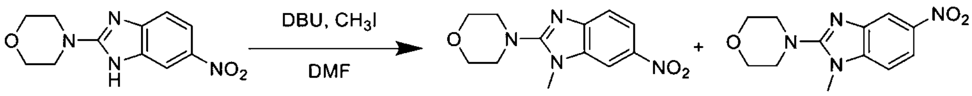


**5-1** **5-2** **5-3**

4-(6-Nitro-1H-benzo[d]imidazol-2-yl)morpholine (1 g, 4.03 mmol) obtained from step 5-1, DBU (1,8-Diazabicyclo [5.4.0]undec-7-ene, 1.84 mL, 12.08 mmol), and methyl iodide (752 μL, 12.08 mmol) were dissolved in dimethylformamide and reacted at 180°C for 4 hours under microwave irradiation. When the reaction was completed, the mixture was extracted with distilled water and ethyl acetate, and the organic layer was dried over anhydrous magnesium sulfate and then filtered. The filtrate was concentrated under reduced pressure and separated by column chromatography to obtain 100 mg of the title compound **5-2-1** (9% yield) and 118 mg of **5-2-2** (11% yield).

**5-2-1:** $^1H$ NMR (400 MHz, DMSO-$d_6$) δ 8.33 (d, *J* = 2.3 Hz, 1H), 8.04 (dd, *J* = 8.8, 2.3 Hz, 1H), 7.51 (d, *J* = 8.8 Hz, 1H), 3.82 - 3.77 (m, 4H), 3.73 (s, 3H), 3.44 - 3.37 (m, 4H),

**5-2-2:** $^1H$ NMR (400 MHz, DMSO-$d_6$) δ 8.25 (d, *J* = 2.2 Hz, 1H), 8.06 (dd, *J* = 8.8, 2.3 Hz, 1H), 7.58 (d, *J* = 8.8 Hz, 1H), 3.82 - 3.77 (m, 4H), 3.71 (s, 3H), 3.35 - 3.32 (m, 4H).

**Step 5-3: Preparation of 1-methyl-2-morpholino-1H-benzo[d]imidazol-6-amine**

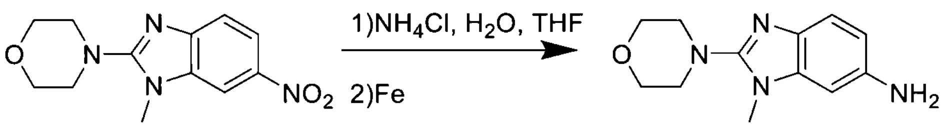


**5-2** **5-4**

4-(1-Methyl-6-nitro-1H-benzo[d]imidazol-2-yl)morpholine (80 mg, 0.30 mmol) obtained from step 5-2 and ammonium chloride (130 mg, 2.44 mmol) were dissolved in distilled water and tetrahydrofuran and stirred at room temperature for 30 minutes. Iron (136 mg, 2.44 mmol) was added to the reaction mixture, and the mixture was stirred at 60°C for 3 hours. When the reaction was completed, the mixture was filtered through filter paper and extracted with distilled water and ethyl acetate. Thereafter, the organic layer was dried over anhydrous magnesium sulfate and then filtered. The filtrate was concentrated under reduced pressure and separated by column chromatography to obtain 54 mg of the title compound (76% yield).

$^1$H NMR (400 MHz, DMSO-$d_6$) δ 7.08 (d, *J* = 8.3 Hz, 1H), 6.50 - 6.38 (m, 2H), 4.78 (s, 2H), 3.76 (t, *J* = 4.6 Hz, 4H), 3.46 (s, 3H), 3.10 (t, *J* = 4.7 Hz, 4H).

**Step 5-4: Preparation of (1-methyl-2-morpholino-1H-benzo[d]imidazol-6-yl)carbonohydrazonoyl dicyanide**

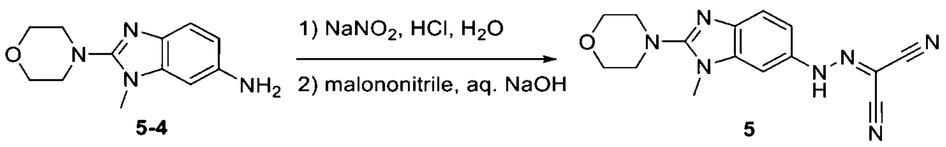


5-4 5

The reaction was performed in a similar manner to step 1-2 of Example 1, except that 1-methyl-2-morpholino-1H-benzo[d]imidazol-6-amine (50 mg, 0.21 mmol) obtained from step 5-3 was used in place of 2-morpholinobenzo[d]thiazol-5-amine, to obtain 50 mg of the title compound (74% yield).

$^1$H NMR (400 MHz, DMSO-$d_6$) δ 7.50 - 7.40 (m, 2H), 7.32 (dd, *J* = 8.5, 2.0 Hz, 1H), 3.79 (t, *J* = 4.7 Hz, 4H), 3.65 (s, 3H), 3.34 - 3.31 (m, 4H).

**Example 6: Preparation of (1-methyl-2-morpholino-1H-benzo[d]imidazol-5-yl)carbonohydrazonoyl dicyanide (Compound 6)**

**Step 6-1: Preparation of 1-methyl-2-morpholino-1H-benzo[d]imidazol-5-amine**

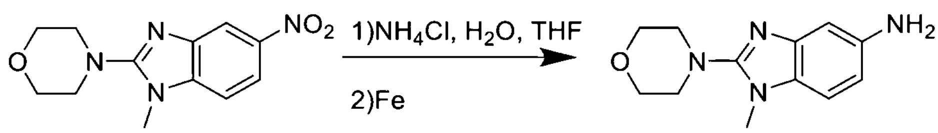


5-3 6-1

The reaction was performed in the same manner as step 5-3 of Example 5, except that 4-(1-methyl-5-nitro-1H-benzo[d]imidazol-2-yl)morpholine (40 mg, 0.17 mmol) obtained from step 5-2 of Example 5 was used in place of 4-(1-methyl-6-nitro-1H-benzo[d]imidazol-2-yl)morpholine, to obtain 67 mg of the title compound (75% yield).

$^1$H NMR (400 MHz, DMSO-$d_6$) δ 6.99 (d, *J* = 8.3 Hz, 1H), 6.62 (d, *J* = 2.1 Hz, 1H), 6.43 (dd, *J* = 8.3, 2.1 Hz, 1H), 4.58 (s, 2H), 3.76 (t, *J* = 4.7 Hz, 4H), 3.49 (s, 3H), 3.14 (t, *J* = 4.7 Hz, 4H).

**Step 6-2: Preparation of (1-methyl-2-morpholino-1H-benzo[d]imidazol-5-yl)carbonohydrazonoyl dicyanide**

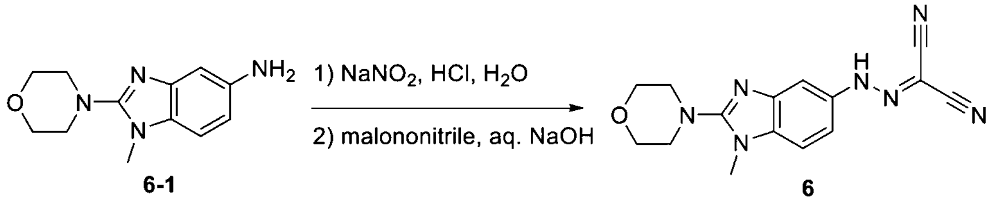


6-1 6

The reaction was performed in a similar manner to step 1-2 of Example 1, except that 1-methyl-2-morpholino-1H-benzo[d]imidazol-5-amine (50 mg, 0.21 mmol) obtained from step 6-1 was used in place of 2-morpholinobenzo[d]thiazol-5-amine, to obtain 56 mg of the title compound (88% yield).

$^1H$ NMR (400 MHz, DMSO-$d_6$) δ 13.08 (s, 1H), 7.52 - 7.37 (m, 2H), 7.30 (dd, *J* = 8.7, 2.0 Hz, 1H), 3.78 (t, *J* = 4.6 Hz, 4H), 3.63 (s, 3H).

**Example 7: Preparation of (2-morpholino-1H-benzo[d]imidazol-6-yl)carbonohydrazonoyl dicyanide (Compound 7)**

**Step 7-1: Preparation of 1-methyl-2-morpholino-1H-benzo[d]imidazol-6-amine**

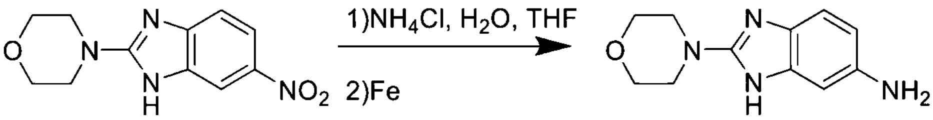


5-1 7-1

The reaction was performed in the same manner as step 5-3 of Example 5, except that 4-(1-methyl-5-nitro-1H-benzo[d]imidazol-2-yl)morpholine (400 mg, 1.61 mmol) obtained from step 5-2 of Example 5 was used in place of 4-(1-methyl-6-nitro-1H-benzo[d]imidazol-2-yl)morpholine, to obtain 66 mg of the title compound (19% yield).

$^1H$ NMR (400 MHz, DMSO-$d_6$) δ 10.94 (s, 1H), 6.88 (d, *J* = 8.2 Hz, 1H), 6.47 (d, *J* = 2.0 Hz, 1H), 6.27 (dd, *J* = 8.2, 2.1 Hz, 1H), 4.50 (s, 2H), 3.70 (t, *J* = 4.8 Hz, 4H), 3.35 (t, *J* = 4.8 Hz, 4H).

**Step 7-2: Preparation of (2-morpholino-1H-benzo[d]imidazol-6-yl)carbonohydrazonoyl dicyanide**

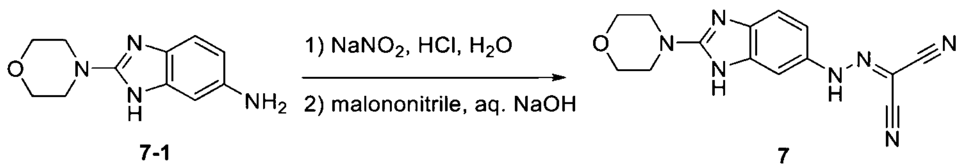


7-1 7

The reaction was performed in a similar manner to step 1-2 of Example 1, except that 1-methyl-2-morpholino-1H-benzo[d]imidazol-6-amine (60 mg, 0.27 mmol) obtained from step 7-1 was used in place of 2-morpholinobenzo[d]thiazol-5-amine, to obtain 38 mg of the title compound (47% yield).

$^1H$ NMR (400 MHz, DMSO-$d_6$) δ 12.57 (s, 1H), 7.31 - 7.23 (m, 2H), 7.20 (dd, *J* = 8.5, 1.9 Hz, 1H), 3.77 (t, *J* = 4.8 Hz, 4H), 3.54 (t, *J* = 4.8 Hz, 4H).

**Example 8: Preparation of (2-(4-methylpiperazin-1-yl)benzo[d]thiazol-6-yl)carbonohydrazonoyl dicyanide (Compound 8)**

**Step 8-1: Preparation of 2-(4-methylpiperazin-1-yl)benzo[d]thiazol-6-amine**

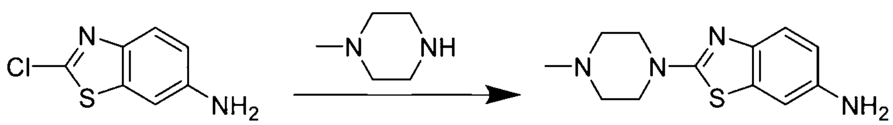

8-1

2-Chlorobenzo[d]thiazol-6-amine (300 mg, 1.62 mmol) was dissolved in 1-methylpiperazine (2.2 mL, 19.50 mmol) and reacted at 180°C for 10 minutes under microwave irradiation. Thereafter, distilled water was added and the mixture was stirred at room temperature for 1 hour, and then extracted with ethyl acetate, and the organic layer was dried over anhydrous magnesium sulfate and then filtered. The filtrate was concentrated under reduced pressure, solidified with ether, and then filtered to obtain 179 mg of the title compound (44% yield).

$^1H$ NMR (400 MHz, DMSO-$d_6$) δ 7.16 (d, $J$ = 8.5 Hz, 1H), 6.89 (d, $J$ = 2.3 Hz, 1H), 6.56 (dd, $J$ = 8.5, 2.3 Hz, 1H), 4.89 (s, 2H), 3.43 (t, $J$ = 5.1 Hz, 4H), 3.32 (s, 4H), 2.41 (t, $J$ = 5.1 Hz, 4H).

**Step 8-2: Preparation of (2-(4-methylpiperazin-1-yl)benzo[d]thiazol-6-yl)carbonohydrazonoyl dicyanide**

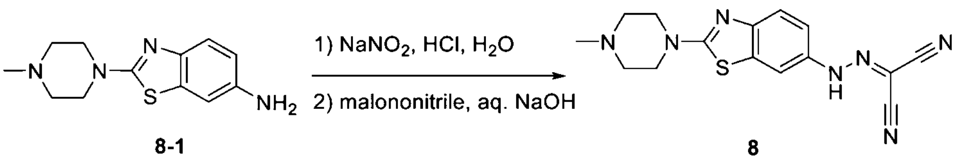


8-1 8

The reaction was performed in a similar manner to step 1-2 of Example 1, except that 2-(4-methylpiperazin-1-yl) benzo[d]thiazol-6-amine (100 mg, 0.40 mmol) obtained from step 8-1 was used in place of 2-morpholinobenzo[d]thiazol-5-amine, to obtain 103 mg of the title compound (78% yield).

$^1H$ NMR (400 MHz, DMSO-$d_6$) δ 10.46 (s, 1H), 7.74 (d, $J$ = 2.1 Hz, 1H), 7.41 (d, $J$ = 8.6 Hz, 1H), 7.30 (dd, $J$ = 8.6, 2.1 Hz, 1H), 3.77 (s, 4H), 3.24 (s, 4H), 2.77 (s, 3H).

**Example 9: Preparation of (2-(4-methoxypiperidin-1-yl)benzo[d]thiazol-6-yl)carbonohydrazonoyl dicyanide (Compound 9)**

**Step 9-1: Preparation of 2-(4-methoxypiperidin-1-yl)benzo[d]thiazol-6-amine**

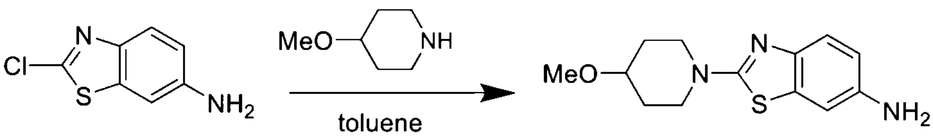


9-1

2-Chlorobenzo[d]thiazol-6-amine (200 mg, 1.08 mmol) was dissolved in toluene, and then 4-methoxypiperidine (1.34 mL, 10.83 mmol) was added, and the mixture was reacted at 180°C for 30 minutes under microwave irradiation. Thereafter, distilled water was added and the mixture was stirred at room temperature for 1 hour, and then extracted with ethyl acetate, and the organic layer was dried over anhydrous magnesium sulfate and then filtered. The filtrate was concentrated under reduced pressure and purified by column chromatography to obtain 270 mg of the title compound (95% yield).

$^1H$ NMR (400 MHz, DMSO-$d_6$) δ 7.15 (d, $J$ = 8.5 Hz, 1H), 6.88 (d, $J$ = 2.4 Hz, 1H), 6.55 (dd, $J$ = 8.5, 2.4 Hz, 1H), 4.88 (s, 2H), 3.76 - 3.61 (m, 2H), 3.43 (dq, $J$ = 8.2, 4.0 Hz, 1H), 3.29 - 3.23 (m, 5H), 1.50 (qd, $J$ = 8.9, 4.5 Hz, 2H)

**Step 9-2: Preparation of (2-(4-methoxypiperidin-1-yl)benzo[d]thiazol-6-yl)carbonohydrazonoyl dicyanide**

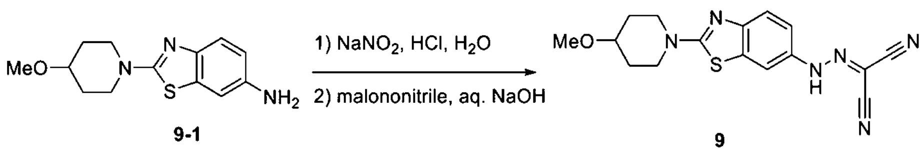


9-1 9

The reaction was performed in a similar manner to step 1-2 of Example 1, except that 2-(4-methoxypiperidin-1-yl) benzo[d]thiazol-6-amine (200 mg, 0.76 mmol) obtained from step 9-1 was used in place of 2-morpholinobenzo[d]thiazol-5-amine, to obtain 34 mg of the title compound (13% yield).

$^1H$ NMR (400 MHz, DMSO-$d_6$) δ 13.09 (s, 1H), 7.91 (d, $J$= 2.1 Hz, 1H), 7.50 - 7.39 (m, 2H), 3.88 (d, $J$ = 12.5 Hz, 2H), 3.69 (dt, $J$ = 6.7, 3.7 Hz, 2H), 2.82 (dd, $J$ = 12.7, 10.6 Hz, 2H), 1.17 (d, $J$ = 6.2 Hz, 6H).

**Example 10: Preparation of (2-(2,6-dimethylmorpholino)benzo[d]thiazol-6-yl)carbonohydrazonoyl dicyanide (Compound 10)**

**Step 10-1: Preparation of 2-(2,6-dimethylmorpholino)benzo[d]thiazol-6-amine**

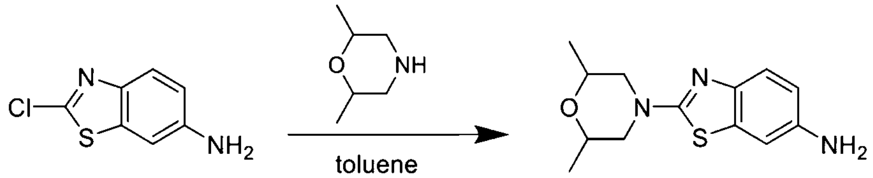


**10-1**

The reaction was performed in a similar manner to step 9-1 of Example 9, except that 2,6-dimethylmorpholine (1.33 mL, 10.83 mmol) was used in place of 4-methoxypiperidine, to obtain 175 mg of the title compound (60% yield).

$^1$H NMR (400 MHz, DMSO-$d_6$) δ 7.17 (d, *J* = 8.5 Hz, 1H), 6.90 (d, *J* = 2.3 Hz, 1H), 6.57 (dd, *J* = 8.5, 2.3 Hz, 1H), 4.91 (s, 2H), 3.75 (dd, *J* = 12.8, 2.4 Hz, 2H), 3.71 - 3.61 (m, 2H), 2.69 (dd, *J* = 12.5, 10.5 Hz, 2H), 1.15 (d, *J* = 6.2 Hz, 6H).

**Step 10-2: Preparation of (2-(2,6-dimethylmorpholino)benzo[d]thiazol-6-yl)carbonohydrazonoyl dicyanide**

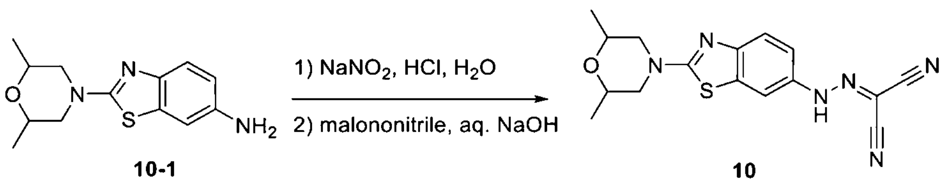


**10-1** **10**

The reaction was performed in a similar manner to step 1-2 of Example 1, except that 2-(2,6-dimethylmorpholino) benzo[d]thiazol-6-amine (100 mg, 0.54 mmol) obtained from step 10-1 was used in place of 2-morpholinobenzo[d] thiazol-5-amine, to obtain 81 mg of the title compound (44% yield).

$^1$H NMR (400 MHz, DMSO-$d_6$) δ 13.09 (s, 1H), 7.91 (d, *J* = 2.1 Hz, 1H), 7.61 - 7.24 (m, 2H), 3.88 (d, *J* = 12.5 Hz, 2H), 3.69 (td, *J* = 6.6, 3.1 Hz, 2H), 2.82 (dd, *J* = 12.7, 10.6 Hz, 2H), 1.17 (d, *J* = 6.2 Hz, 6H).

**Example 11: Preparation of (2-(3-oxa-8-azabicyclo[3.2.1]octan-8-yl)benzo[d]thiazol-6-yl)carbonohydrazonoyl dicyanide (Compound 11)**

**Step 11-1: Preparation of 2-(3-oxa-8-azabicyclo[3.2.1]octan-8-yl)benzo[d]thiazol-6-amine**

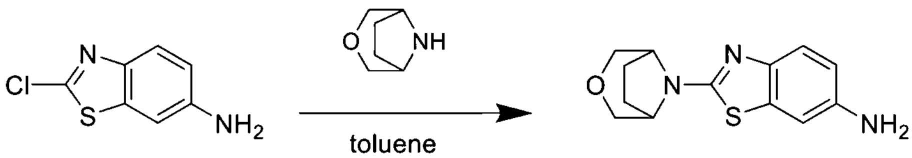


**11-1**

The reaction was performed in a similar manner to step 9-1 of Example 9, except that 3-oxa-8-azabicyclo[3.2.1] octane (613 mg, 0.54 mmol) was used in place of 4-methoxypiperidine, to obtain 262 mg of the title compound (91% yield).

$^1$H NMR (400 MHz, Chloroform-d) δ 7.38 (d, *J* = 8.5 Hz, 1H), 6.96 (d, *J* = 2.3 Hz, 1H), 6.69 (dd, *J* = 8.5, 2.4 Hz, 1H), 4.22 (s, 2H), 3.95 (d, *J* = 11.0 Hz, 2H), 3.62 (d, *J* = 11.0 Hz, 4H), 2.10 (s, 4H).

Step 11-2: Preparation of *N*-(2-(3-oxa-8-azabicyclo[3.2.1]octan-8-yl)benzo[d]thiazol-6-yl)carbonohydrazonoyl dicyanide **(Compound 11**)

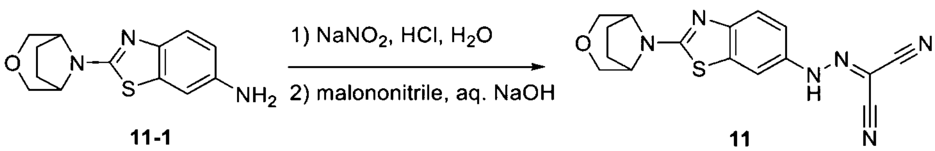


11-1

11

In the same manner as step 1-2, 2-(3-oxa-8-azabicyclo[3.2.1]octan-8-yl)benzo[d]thiazol-6-amine (150 mg, 0.57 mmol), sodium nitrite (59 mg, 0.86 mmol), and 1.0 M hydrochloric acid aqueous solution (1.7 mL, 1.72 mmol) were dissolved in distilled water, and then malononitrile (76 mg, 1.15 mmol) and an aqueous sodium hydroxide solution were used to obtain 90 mg of the title compound (46% yield).

$^1H$ NMR (400 MHz, DMSO-d) δ 13.10 (s, 1H), 7.92 (d, J = 2.2 Hz, 1H), 7.61 - 7.32 (m, 2H), 4.28 (s, 2H), 3.75 (d, J = 11.0 Hz, 2H), 3.61 (d, J = 11.0 Hz, 2H), 2.00 (s, 4H).

**Example 12: Preparation of N-(2-(2-methylmorpholino)benzo[d]thiazol-6-yl)carbonohydrazonoyl dicyanide (Compound 12)**

Step 12-1: Preparation of 2-(2-methylmorpholino)benzo[*d*]thiazol-6-amine **(Compound 12-1)**

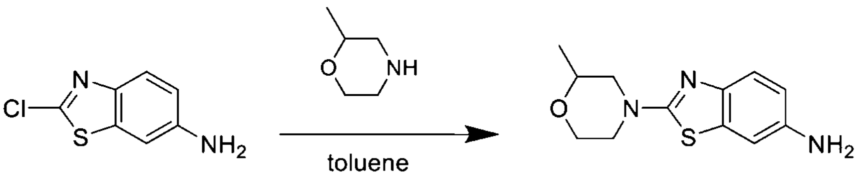


12-1

In the same manner as step 9-1, 2-chloro-6-benzothiazolamine (100 mg, 0.54 mmol) was dissolved in toluene, and then 2-methylmorpholine (0.58 mL, 5.42 mmol) was added to obtain 84 mg of the title compound (62% yield).

$^1H$ NMR (400 MHz, DMSO-$d_6$) δ 7.18 (d, J = 8.5 Hz, 1H), 6.91 (d, J = 2.3 Hz, 1H), 6.57 (dd, J = 8.5, 2.3 Hz, 1H), 4.91 (s, 2H), 3.95 - 3.86 (m, 1H), 3.77 (d, J = 12.4 Hz, 1H), 3.70 - 3.53 (m, 3H), 3.08 (td, J = 12.1, 3.6 Hz, 1H), 2.76 (dd, J = 12.5, 10.4 Hz, 1H), 1.15 (d, J = 6.2 Hz, 3H).

Step 12-2: Preparation of *N*-(2-(2-methylmorpholino)benzo[d]thiazol-6-yl)carbonohydrazonoyl dicyanide **(Compound 12)**

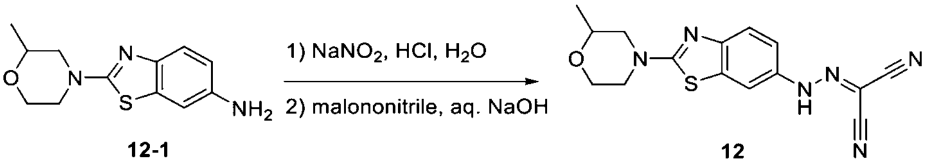


12-1

12

In the same manner as step 1-2, 2-(2-methylmorpholino)benzo[d]thiazol-6-amine (50 mg, 0.20 mmol), sodium nitrite (21 mg, 0.30 mmol), and 1.0 M hydrochloric acid aqueous solution (0.6 mL, 0.60 mmol) were dissolved in distilled water, and then malononitrile (26 mg, 0.40 mmol) and an aqueous sodium hydroxide solution were used to obtain 40 mg of the title compound (60% yield).

$^1H$ NMR (400 MHz, DMSO-*d*) δ 13.10 (s, 1H), 7.92 (d, J = 2.1 Hz, 1H), 7.52 - 7.39 (m, 2H), 3.99 - 3.87 (m, 2H), 3.80 (d, J = 12.7 Hz, 1H), 3.69 - 3.57 (m, 2H), 3.22 (td, J = 12.2, 3.6 Hz, 1H), 2.90 (dd, J = 12.7, 10.5 Hz, 1H), 1.17 (d, J = 6.2 Hz, 3H).

**Example 13: Preparation of N-(2-(3-methylmorpholino)benzo[d]thiazol-6-yl)carbonohydrazonoyl dicyanide (Compound 13)**

Step 13-1: Preparation of 2-(3-methylmorpholino)benzo[*d*]thiazol-6-amine **(Compound 13-1)**

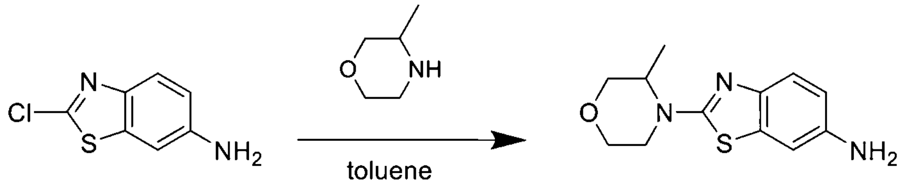


**13-1**

In the same manner as step 9-1, 2-chloro-6-benzothiazolamine (100 mg, 0.54 mmol) was dissolved in toluene, and 3-methylmorpholine (0.57 mL, 5.42 mmol) was added and reacted at 180°C for 12 hours under microwave irradiation to obtain 108 mg of the title compound (74% yield).

$^1H$ NMR (400 MHz, DMSO-$d_6$) δ 7.17 (d, J = 8.5 Hz, 1H), 6.90 (d, J = 2.3 Hz, 1H), 6.68 - 6.41 (m, 1H), 4.91 (s, 3H), 3.99 - 3.82 (m, 3H), 3.69 - 3.64 (m, 2H), 3.59 - 3.46 (m, 2H), 3.37 - 3.35 (m, 1H), 1.24 (d, J = 6.7 Hz, 3H).

Step 13-2: Preparation of *N*-(2-(3-methylmorpholino)benzo[d]thiazol-6-yl)carbonohydrazonoyl dicyanide **(Compound 13)**

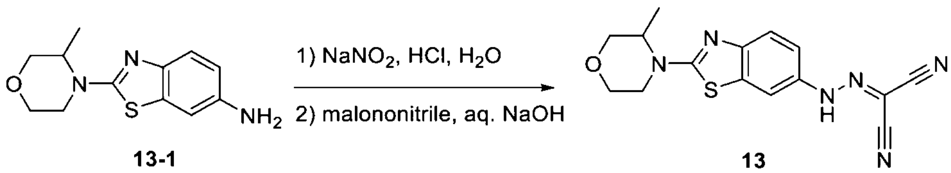


**13-1** **13**

In the same manner as step 1-2, 2-(3-methylmorpholino)benzo[d]thiazol-6-amine (50 mg, 0.20 mmol), sodium nitrite (21 mg, 0.30 mmol), and 1.0 M hydrochloric acid aqueous solution (0.6 mL, 0.60 mmol) were dissolved in distilled water, and then malononitrile (26 mg, 0.40 mmol) and an aqueous sodium hydroxide solution were used to obtain 14 mg of the title compound (21% yield).

$^1H$ NMR (400 MHz, DMSO-*d*) δ 13.10 (s, 1H), 7.91 (d, J = 2.1 Hz, 1H), 7.54 - 7.37 (m, 2H), 4.08 (d, J = 7.4 Hz, 1H), 4.00 - 3.86 (m, 1H), 3.76 - 3.62 (m, 3H), 3.59 - 3.50 (m, 1H), 3.44 (dd, J = 12.4, 3.5 Hz, 1H), 1.29 (d, J = 6.7 Hz, 3H).

**Example 14: Preparation of N-(2-(4-methylpiperazin-1yl)benzo[d]oxazol-6-yl)carbonohydrazonoyl dicyanide (Compound 14)**

Step 14-1: Preparation of 2-(4-methylpiperazin-1yl)-6-nitrobenzo[*d*]oxazole **(Compound 14-1)**

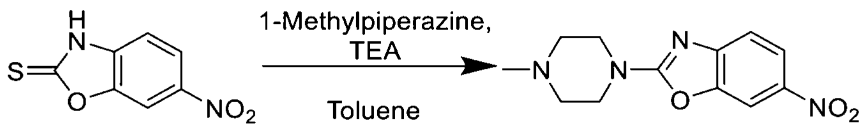


**4-1** **14-1**

In the same manner as step 3-2, 6-nitrobenzo[*d*]oxazole-2(3H)-thione (100 mg, 0.51 mmol), 1-methylpiperazine (85 μL, 0.76 mmol), triethylamine (213 μL, 1.53 mmol), and toluene were used to obtain 89 mg of the title compound (67% yield).

$^1H$ NMR (400 MHz, DMSO-$d_6$) δ 8.29 (d, J = 2.3 Hz, 1H), 8.15 (dd, J = 8.7, 2.3 Hz, 1H), 7.40 (d, J = 8.7 Hz, 1H), 3.69 (t, J = 5.1 Hz, 4H), 2.45 (t, J = 5.2 Hz, 4H), 2.24 (s, 3H).

Step 14-2: Preparation of 2-(4-methylpiperazin-1-yl)benzo[*d*]oxazol-6-amine **(Compound 14-2)**

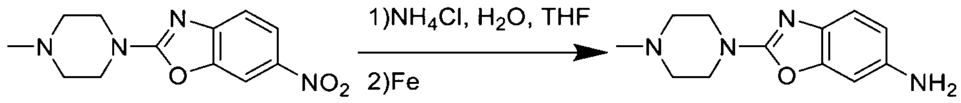


**14-1** **14-2**

In the same manner as step 5-3, 2-(4-methylpiperazin-1-yl)-6-nitrobenzo[d]oxazole (100 mg, 0.38 mmol) and ammonium chloride (163 mg, 3.05 mmol) were dissolved in distilled water and tetrahydrofuran, and then iron (170 mg, 3.05 mmol) was used in the reaction mixture to obtain 45 mg of the title compound (51 % yield).

$^1$H NMR (400 MHz, DMSO-$d_6$) δ 6.95 (d, J = 8.3 Hz, 1H), 6.61 (d, J = 2.1 Hz, 1H), 6.41 (dd, J = 8.3, 2.1 Hz, 1H), 4.87 (s, 2H), 3.48 (t, J = 5.1 Hz, 4H), 2.39 (t, J = 5.1 Hz, 4H), 2.21 (s, 3H).

Step 14-3: Preparation of *N*-(2-(4-methylpiperazin-1yl)benzo[*d*]oxazol-6-yl)carbonohydrazonoyl dicyanide (**Compound 14**)

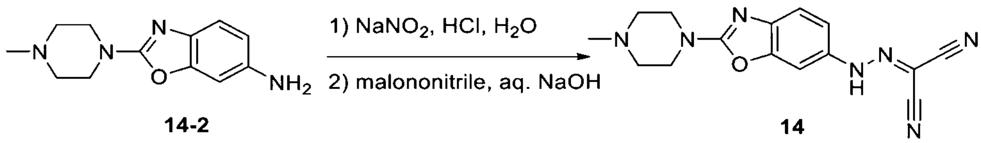


**14-2** **14**

In the same manner as step 1-2, 2-(4-methylpiperazin-1-yl)benzo[*d*]oxazol-6-amine (30 mg, 0.13 mmol), sodium nitrite (13 mg, 0.19 mmol), and 1.0 M hydrochloric acid aqueous solution (0.4 mL, 0.39 mmol) were dissolved in distilled water, and then malononitrile (17 mg, 0.26 mmol) and an aqueous sodium hydroxide solution were used to obtain 36 mg of the title compound (40% yield).

$^1$H NMR (400 MHz, DMSO-d) δ 10.05 (s, 1H), 7.31 (d, J = 1.7 Hz, 1H), 7.26 - 7.17 (m, 2H), 3.79 (s, 4H), 3.22 (s, 4H), 2.76 (s, 3H).

**Example 15: Preparation of N-(2-(2-methylmorpholino)benzo[d]oxazol-6-yl)carbonohydrazonoyl dicyanide (Compound 15)**

Step 15-1: Preparation of 2-(2-methylmorpholino)-6-nitrobenzo[*d*]oxazole **(Compound 15-1)**

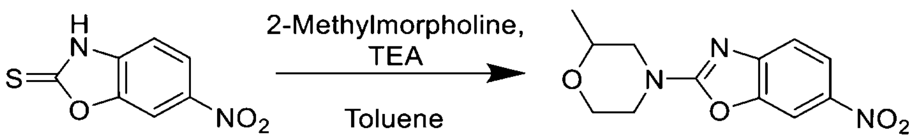


**4-1** **15-1**

In the same manner as step 3-2, 6-nitrobenzo[*d*]oxazole-2(3*H*)-thione (300 mg, 1.53 mmol), 2-methylmorpholine (247 μL, 2.29 mmol), triethylamine (639 μL, 4.59 mmol), and toluene were added and reacted at 180°C for 30 minutes under microwave irradiation to obtain 193 mg of the title compound (72% yield).

$^1$H NMR (400 MHz, Chloroform-d) δ 8.19 (dd, J = 8.7, 2.2 Hz, 1H), 8.14 (d, J = 2.2 Hz, 1H), 7.34 (d, J = 8.7 Hz, 1H), 4.23 - 4.01 (m, 3H), 3.83 - 3.64 (m, 2H), 3.35 (ddd, J = 13.1, 11.9, 3.7 Hz, 1H), 3.00 (dd, J = 13.0, 10.6 Hz, 1H), 1.28 (d, J = 6.2 Hz, 3H).

Step 15-2: Preparation of 2-(2-methylmorpholino)benzo[*d*]oxazol-6-amine **(Compound 15-2)**

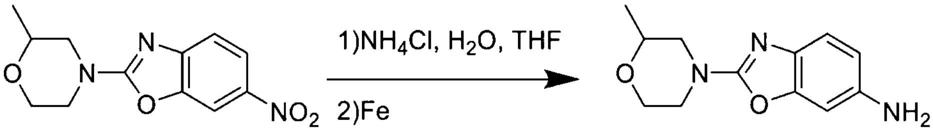


15-1 15-2

In the same manner as step 5-3, 2-(2-methylmorpholino)-6-nitrobenzo[*d*]oxazole (200 mg, 0.95 mmol) and ammonium chloride (406 mg, 7.60 mmol) were dissolved in distilled water and tetrahydrofuran, and then iron (424 mg, 7.60 mmol) was used in the reaction mixture to obtain 88 mg of the title compound (40% yield).

$^{1}H$ NMR (400 MHz, DMSO-$d_6$) δ 6.97 (d, J = 8.3 Hz, 1H), 6.62 (d, J = 2.1 Hz, 1H), 6.42 (dd, J = 8.3, 2.1 Hz, 1H), 4.89 (s, 2H), 3.87 (ddd, J = 10.9, 6.3, 2.5 Hz, 2H), 3.82 - 3.73 (m, 1H), 3.62 - 3.53 (m, 2H), 3.07 (td, J = 12.4, 3.6 Hz, 1H), 2.76 (dd, J = 12.7, 10.4 Hz, 1H), 1.14 (d, J = 6.2 Hz, 3H).

Step 15-3: Preparation of *N*-(2-(2-methylmorpholino)benzo[*d*]oxazol-6-yl)carbonohydrazonoyl dicyanide **(Compound 15)**

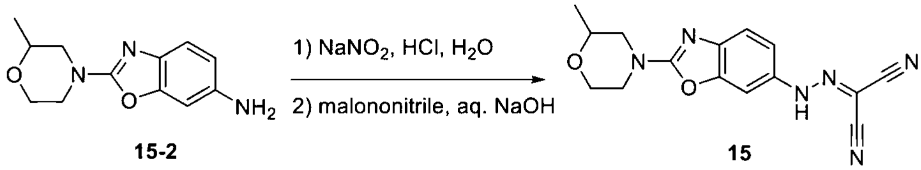


15-2 15

In the same manner as step 1-2, 2-(2-methylmorpholino)benzo[*d*]oxazol-6-amine (30 mg, 0.13 mmol), sodium nitrite (13 mg, 0.19 mmol), and 1.0 M hydrochloric acid aqueous solution (0.4 mL, 0.39 mmol) were dissolved in distilled water, and then malononitrile (17 mg, 0.26 mmol) and an aqueous sodium hydroxide solution were used to obtain 12 mg of the title compound (30% yield).

$^{1}H$ NMR (400 MHz, DMSO-*d*) δ 13.08 (s, 1H), 7.50 (d, J = 1.9 Hz, 1H), 7.38 - 7.28 (m, 2H), 4.15 - 3.76 (m, 3H), 3.71 - 3.53 (m, 2H), 3.20 (td, J = 12.5, 3.7 Hz, 1H), 2.89 (dd, J = 12.8, 10.5 Hz, 1H), 1.15 (d, J = 6.2 Hz, 3H).

**Example 16: Preparation of N-(2-(3-methylmorpholino)benzo[d]oxazol-6-yl)carbonohydrazonoyl dicyanide (Compound 16)**

Step 16-1: Preparation of 2-(3-methylmorpholino)-6-nitrobenzo[*d*]oxazole **(Compound 16-1)**

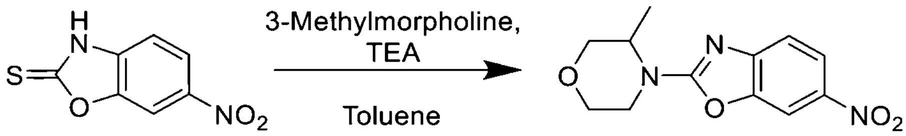


4-1 16-1

In the same manner as step 3-2, 6-nitrobenzo[*d*]oxazole-2(3H)-thione (200 mg, 1.02 mmol), 3-methylmorpholine (161 μL, 1.53 mmol), triethylamine (426 μL, 3.06 mmol), and toluene were used to obtain 193 mg of the title compound (72% yield).

$^{1}H$ NMR (400 MHz, Chloroform-d) δ 8.24 - 8.09 (m, 2H), 7.34 (d, J = 8.7 Hz, 1H), 4.43 - 4.29 (m, 1H), 4.09 - 3.87 (m, 2H), 3.80 (s, 1H), 3.73 - 3.52 (m, 2H), 1.47 (d, J = 6.9 Hz, 3H).

Step 16-2: Preparation of 2-(3-methylmorpholino)benzo[*d*]oxazol-6-amine **(Compound 16-2)**

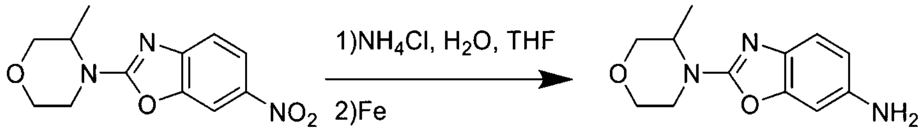


16-1 16-2

In the same manner as step 5-3, 2-(3-methylmorpholino)-6-nitrobenzo[*d*]oxazole (100 mg, 0.43 mmol) and ammonium chloride (293 mg, 5.47 mmol) were dissolved in distilled water and tetrahydrofuran, and then iron (305 mg, 5.47 mmol) was added to the reaction mixture, and after solvent removal, the product was used in the next step without purification.

Step 16-3: Preparation of *N*-(2-(3-methylmorpholino)benzo[*d*]oxazol-6-yl)carbonohydrazonoyl dicyanide **(Compound 16)**

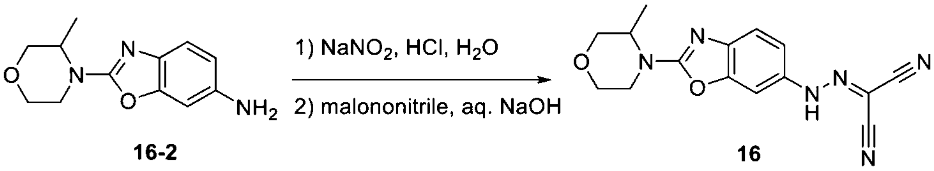


16-2 16

In the same manner as step 1-2, 2-(3-methylmorpholino)benzo[*d*]oxazol-6-amine (100 mg, 0.43 mmol), sodium nitrite (44 mg, 0.64 mmol), and 1.0 M hydrochloric acid aqueous solution (1.3 mL, 1.29 mmol) were dissolved in distilled water, and then malononitrile (57 mg, 0.86 mmol) and an aqueous sodium hydroxide solution were used to obtain 29 mg of the title compound (22% yield).

$^1$H NMR (400 MHz, Chloroform-d) δ 9.72 (s, 1H), 7.41 - 7.30 (m, 2H), 7.05 (dd, J = 8.4, 2.2 Hz, 1H), 4.29 (q, J = 7.1 Hz, 1H), 4.01 (dd, J = 11.0, 3.3 Hz, 1H), 3.92 - 3.85 (m, 1H), 3.79 (d, J = 2.2 Hz, 2H), 3.70 - 3.50 (m, 2H), 1.44 (d, J = 6.8 Hz, 3H).

**Example 17: Preparation of N-(2-(4-methoxypiperidin-1-yl)benzo[d]oxazol-6-yl)carbonohydrazonoyl dicyanide (Compound 17)**

Step 17-1: Preparation of 2-(4-methoxypiperidin-1-yl)-6-nitrobenzo[*d*]oxazole **(Compound 17-1)**

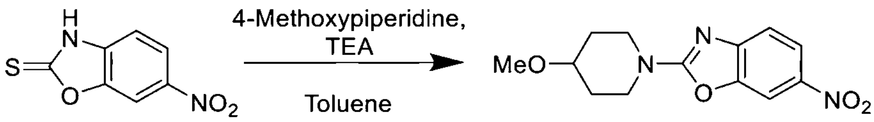


4-1 17-1

In the same manner as step 3-2, 6-nitrobenzo[*d*]oxazole-2(3*H*)-thione (200 mg, 1.02 mmol), 3-methoxypiperidine (189 μL, 1.53 mmol), triethylamine (426 μL, 3.06 mmol), and toluene were used to obtain 170 mg of the title compound (60% yield).

$^1$H NMR (400 MHz, DMSO-$d_6$) δ 8.28 (d, J = 2.2 Hz, 1H), 8.14 (dd, J = 8.7, 2.3 Hz, 1H), 7.38 (d, J = 8.7 Hz, 1H), 3.89 (ddd, J = 11.4, 6.8, 4.0 Hz, 2H), 3.52 (dddd, J = 19.1, 11.5, 8.1, 3.7 Hz, 3H), 3.30 (s, 3H), 2.04 - 1.89 (m, 2H), 1.60 (dtd, J = 12.5, 8.1, 4.0 Hz, 2H).

Step 17-2: Preparation of 2-(4-methoxypiperidin-1-yl)benzo[d]oxazol-6-amine **(Compound 17-2)**

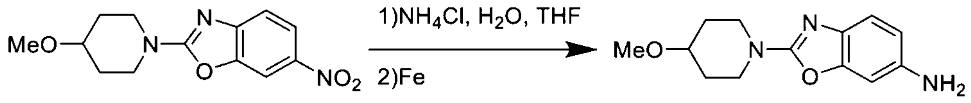


17-1 17-2

In the same manner as step 5-3, 2-(4-methoxypiperidin-1-yl)-6-nitrobenzo[*d*]oxazole (120 mg, 0.51 mmol) and ammonium chloride (220 mg, 4.11 mmol) were dissolved in distilled water and tetrahydrofuran, and then iron (230 mg, 4.11 mmol) was used in the reaction mixture to obtain 63 mg of the title compound (49% yield).

$^1$H NMR (400 MHz, Chloroform-*d*) δ 7.13 (d, J = 8.3 Hz, 1H), 6.66 (d, J = 2.2 Hz, 1H), 6.53 (dd, J = 8.2, 2.2 Hz, 1H), 3.91 (ddd, J = 13.1, 6.9, 3.9 Hz, 2H), 3.62 (s, 2H), 3.50 - 3.37 (m, 8H), 1.96 (ddt, J = 13.9, 7.7, 3.7 Hz, 2H), 1.70 (dtd, J = 12.6, 8.1, 3.9 Hz, 2H).

Step 17-3: Preparation of *N*-(2-(4-methoxypiperidin-1-yl)benzo[*d*]oxazol-6-yl)carbonohydrazonoyl dicyanide **(Compound 17)**

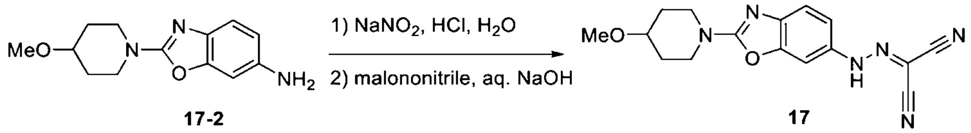


17-2 17

In the same manner as step 1-2, 2-(4-methoxypiperidin-1-yl)benzo[*d*]oxazol-6-amine (50 mg, 0.20 mmol), sodium nitrite (21 mg, 0.30 mmol), and 1.0 M hydrochloric acid aqueous solution (0.6 mL, 0.61 mmol) were dissolved in distilled water, and then malononitrile (27 mg, 0.40 mmol) and an aqueous sodium hydroxide solution were used to obtain 18 mg of the title compound (28% yield).

$^1$H NMR (400 MHz, DMSO-*d$_6$*) δ 13.07 (s, 1H), 7.49 (s, 1H), 7.34 - 7.25 (m, 2H), 3.93 - 3.75 (m, 2H), 3.55 - 3.34 (m, 3H), 3.29 (s, 3H), 2.02 - 1.87 (m, 2H), 1.58 - 1.53 (m, 2H).

**Example 18: Preparation of N-(2-(2,6-dimethylmorpholino)benzo[d]oxazol-6-yl)carbonohydrazonoyl dicyanide**

Step 18-1: Preparation of 2-(2,6-dimethylmorpholino)-6-nitrobenzo[*d*]oxazole **(Compound 18-1)**

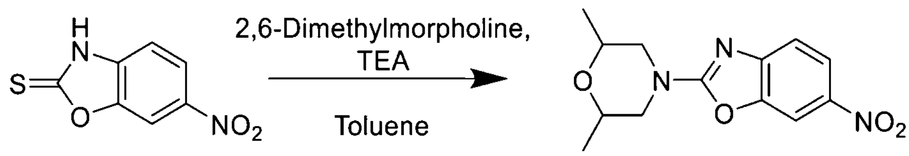


4-1 18-1

In the same manner as step 3-2, 6-nitrobenzo[*d*]oxazole-2(3*H*)-thione (200 mg, 1.02 mmol), 2,6-dimethylmorpholine (188 μL, 1.53 mmol), triethylamine (426 μL, 3.06 mmol), and toluene were used to obtain 227 mg of the title compound (80% yield).

$^1$H NMR (400 MHz, Chloroform-*d*) δ 8.19 (dd, J = 8.7, 2.2 Hz, 1H), 8.14 (d, J = 2.1 Hz, 1H), 7.33 (d, J = 8.7 Hz, 1H), 4.15 (dd, J = 13.5, 2.2 Hz, 2H), 3.74 (dtd, J = 12.5, 6.3, 2.6 Hz, 2H), 2.91 (dd, J = 13.1, 10.7 Hz, 2H), 1.29 (d, J = 6.2 Hz, 6H).

Step 18-2: Preparation of 2-(2,6-dimethylmorpholino)benzo[*d*]oxazol-6-amine **(Compound 18-2)**

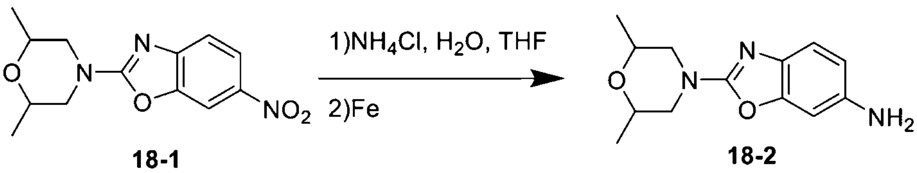


18-1 18-2

In the same manner as step 5-3, 2-(2,6-dimethylmorpholino)-6-nitrobenzo[*d*]oxazole (200 mg, 0.72 mmol) and ammonium chloride (309 mg, 5.77 mmol) were dissolved in distilled water and tetrahydrofuran, and then iron (322 mg, 5.77 mmol) was used in the reaction mixture to obtain 54 mg of the title compound (30% yield).

$^1$H NMR (400 MHz, Chloroform-*d*) δ 7.14 (d, J = 8.3 Hz, 1H), 6.67 (d, J = 2.2 Hz, 1H), 6.55 (dd, J = 8.3, 2.2 Hz, 1H), 4.00 (dd, J = 13.3, 2.3 Hz, 2H), 3.73 (dtd, J = 12.4, 6.2, 2.5 Hz, 2H), 3.60 (s, 2H), 2.78 (dd, J = 12.9, 10.6 Hz, 2H), 1.25 (d, J = 6.2 Hz, 6H).

Step 18-3: Preparation of *N*-(2-(2,6-dimethylmorpholino)benzo[*d*]oxazol-6-yl)carbonohydrazonoyl dicyanide **(Compound 18)**

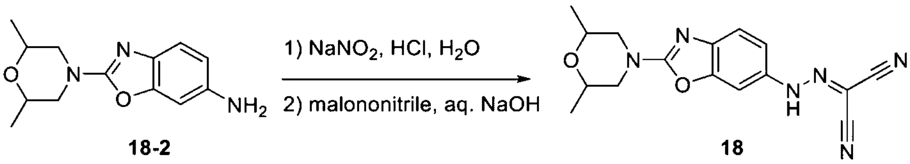


18-2 18

In the same manner as step 1-2, 2-(2,6-dimethylmorpholino)benzo[*d*]oxazol-6-amine (50 mg, 0.20 mmol), sodium nitrite (21 mg, 0.30 mmol), and 1.0 M hydrochloric acid aqueous solution (0.6 mL, 0.61 mmol) were dissolved in distilled water, and then malononitrile (27 mg, 0.40 mmol) and an aqueous sodium hydroxide solution were used to obtain 19 mg of the title compound (29% yield).

$^1$H NMR (400 MHz, Chloroform-*d*) δ 9.76 (s, 1H), 7.38 - 7.30 (m, 2H), 7.06 (dd, J = 8.4, 2.2 Hz, 1H), 4.18 - 4.00 (m, 3H), 3.74 (ddp, J = 12.4, 6.1, 3.1, 2.5 Hz, 2H), 2.86 (dd, J = 13.0, 10.7 Hz, 2H), 1.27 (d, J = 6.2 Hz, 8H).

**Example 19: Preparation of N-(5-chloro-2-morpholinobenzo[*d*]oxazol-6-yl)carbonohydrazonoyl dicyanide**

**Step 19-1: Preparation of 5-chloro-6-nitrobenzo[*d*]oxazole-2(3*H*)-thione (Compound 19-1)**

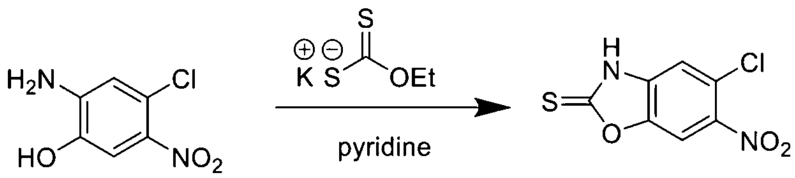


19-1

2-Amino-4-chloro-5-nitrophenol (200 mg, 1.06 mmol) was dissolved in pyridine, and potassium ethyl xanthogenate (187 mg, 1.17 mmol) was added, and the mixture was stirred at 120°C for 2 hours. When the reaction was completed, the mixture was extracted with distilled water and ethyl acetate, and the organic layer was dried over anhydrous magnesium sulfate and then filtered. The filtrate was concentrated under reduced pressure and used in the next step without purification.

Step 19-2: Preparation of 5-chloro-2-morpholino-6-nitrobenzo[*d*]oxazole **(Compound 19-2)**

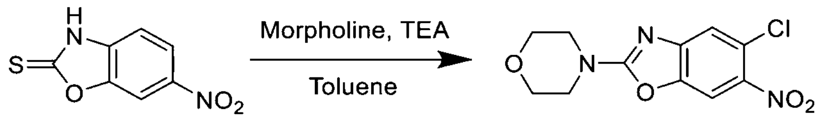


19-1 19-2

In the same manner as step 3-2, 5-chloro-6-nitrobenzo[d]oxazole-2(3H)-thione (100 mg, 0.43 mmol), morpholine (56 μL, 0.65 mmol), triethylamine (181 μL, 1.30 mmol), and toluene were used to obtain 30 mg of the title compound (24% yield).

$^1$H NMR (400 MHz, DMSO-$d_6$) δ 8.29 (s, 1H), 7.61 (s, 1H), 3.76 - 3.74 (m, 4H), 3.69 - 3.67 (m, 4H).

Step 19-3: Preparation of 5-chloro-2-morpholinobenzo[d]oxazol-6-amine **(Compound 19-3)**

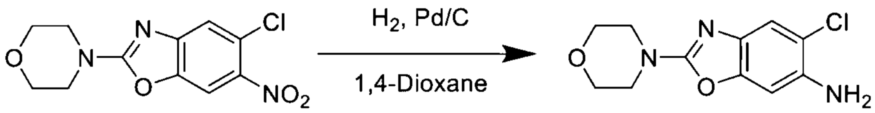


19-2 19-3

In the same manner as step 3-3, 5-chloro-2-morpholino-6-nitrobenzo[*d*]oxazole (50 mg, 0.18 mmol) and 10% Pd/C (37 mg, 0.03 mmol) were dissolved in 1,4-dioxane, and the title compound was used in the next step without purification through hydrogen.

Step 19-4: Preparation of *N*-(5-chloro-2-morpholinobenzo[*d*]oxazol-6-yl)carbonohydrazonoyl dicyanide **(Compound 19)**

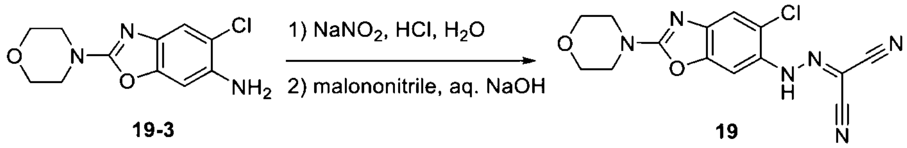


19-3 19

In the same manner as step 1-2, 5-chloro-2-morpholinobenzo[*d*]oxazol-6-amine (30 mg, 0.12 mmol), sodium nitrite (12 mg, 0.18 mmol), and 1.0 M hydrochloric acid aqueous solution (0.3 mL, 0.35 mmol) were dissolved in distilled water, and then malononitrile (16 mg, 0.24 mmol) and an aqueous sodium hydroxide solution were used to obtain 11 mg of the title compound (27% yield).

$^1$H NMR (400 MHz, Chloroform-*d*) δ 10.10 (s, 1H), 7.55 (s, 1H), 7.35 (s, 1H), 3.83 (t, *J* = 4.8 Hz, 4H), 3.71 (t, *J* = 4.9 Hz, 4H).

**Example 20: Preparation of N-(2-(3-oxa-8-azabicyclo[3.2.1]octan-8-yl)benzo[d]oxazol-6-yl)carbonohydrazonoyl dicyanide**

Step 20-1: Preparation of 2-(3-oxa-8-azabicyclo[3.2.1]octan-8-yl)-6-nitrobenzo[d]oxazole **(Compound 20-1)**

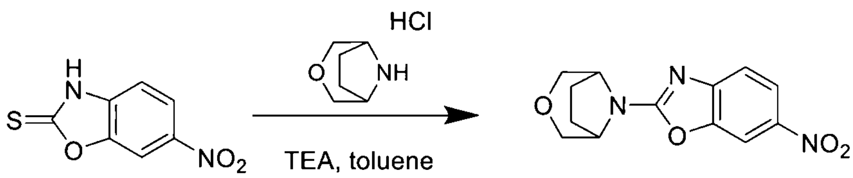


4-1 20-1

In the same manner as step 9-1, 6-nitrobenzo[*d*]oxazole-2(3*H*)-thione (1 g, 5.10 mmol), 3-oxa-8-azabicyclo [3.2.1]octane hydrochloride (1.14 g, 7.65 mmol), triethylamine (2.13 mL, 15.29 mmol), and toluene were used to obtain 799 mg of the title compound (57% yield).

$^1$H NMR (400 MHz, Chloroform-d) δ 8.20 (dd, J = 8.7, 2.2 Hz, 1H), 8.15 (d, J = 2.2 Hz, 1H), 7.35 (d, J = 8.7 Hz, 1H), 4.49 (dd, J = 4.6, 2.2 Hz, 2H), 3.87 (d, J = 11.2 Hz, 2H), 3.72 (d, J = 11.6 Hz, 2H), 2.25 - 2.09 (m, 4H).

Step 20-2: Preparation of 2-(3-oxa-8-azabicyclo[3.2.1]octan-8-yl)benzo[*d*]oxazol-6-amine **(Compound 20-2)**

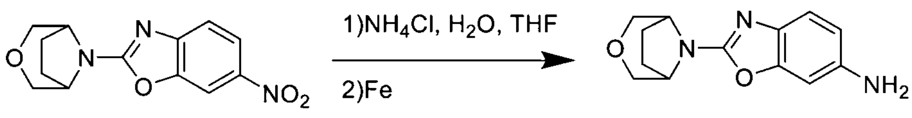


**20-1** **20-2**

In the same manner as step 5-3, 2-(3-oxa-8-azabicyclo[3.2.1]octan-8-yl)-6-nitrobenzo[*d*]oxazole (750 mg, 2.72 mmol) and ammonium chloride (1.17 g, 21.80 mmol) were dissolved in distilled water and tetrahydrofuran, and then iron (1.22 g, 21.80 mmol) was used in the reaction mixture to obtain 241 mg of the title compound (36% yield).

$^1$H NMR (400 MHz, Chloroform-d) δ 7.17 (d, J = 8.3 Hz, 1H), 6.68 (d, J = 2.2 Hz, 1H), 6.56 (dd, J = 8.2, 2.2 Hz, 1H), 4.34 (s, 2H), 3.89 (d, J = 11.0 Hz, 2H), 3.63 (d, J = 10.8 Hz, 5H), 2.13 - 2.02 (m, 4H).

Step 20-3: Preparation of *N*-(2-(3-oxa-8-azabicyclo[3.2.1]octan-8-yl)benzo[*d*]oxazol-6-yl)carbonohydrazonoyl dicyanide **(Compound 20)**

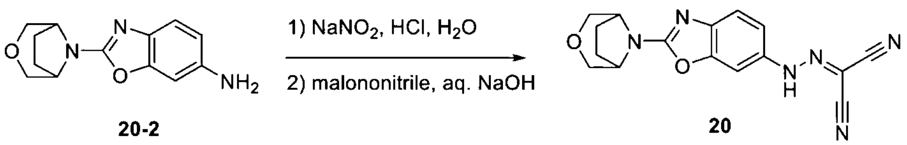


**20-2** **20**

In the same manner as step 1-2, 2-(3-oxa-8-azabicyclo[3.2.1]octan-8-yl)benzo[d]oxazol-6-amine (230 mg, 0.94 mmol), sodium nitrite (97 mg, 1.41 mmol), and 1.0 M hydrochloric acid aqueous solution (2.8 mL, 2.81 mmol) were dissolved in distilled water, and then malononitrile (124 mg, 1.88 mmol) and an aqueous sodium hydroxide solution were used to obtain 235 mg of the title compound (78% yield).

$^1$H NMR (400 MHz, DMSO-d6) δ 13.08 (s, 2H), 7.52 (s, 1H), 7.34 (s, 2H), 4.35 (s, 2H), 3.72 (d, J = 11.0 Hz, 2H), 3.61 (d, J = 11.1 Hz, 2H), 2.00 (d, J = 2.2 Hz, 4H).

**Example 21: Preparation of tert-butyl 4-(6-(2-(dicyanomethylene)hydrazinyl)benzo[d]oxazol-2-yl)piperazine-1-carboxylate**

Step 21-1: Preparation of *tert*-butyl 4-(6-nitrobenzo[*d*]oxazol-2-yl)piperazine-1-carboxylate **(Compound 21-1)**

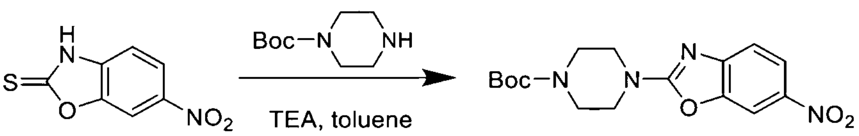


**4-1** **21-1**

In the same manner as step 9-1, 6-nitrobenzo[*d*]oxazole-2(3*H*)-thione (300 mg, 1.53 mmol), 1-Boc-piperazine (427 mg, 2.29 mmol), triethylamine (639 μL, 4.59 mmol), and toluene were used to obtain 246 mg of the title compound (53% yield).

$^1$H NMR (400 MHz, Chloroform-d) δ 8.19 (dd, J = 8.7, 2.2 Hz, 1H), 8.15 (d, J = 2.2 Hz, 1H), 7.34 (d, J = 8.7 Hz, 1H),

3.76 (dd, J = 6.6, 4.0 Hz, 4H), 3.61 (dd, J = 6.4, 4.0 Hz, 4H), 1.50 (s, 9H).

Step 21-2: Preparation of *tert*-butyl 4-(6-aminobenzo[*d*]oxazol-2-yl)piperazine-1-carboxylate **(Compound 21-2)**

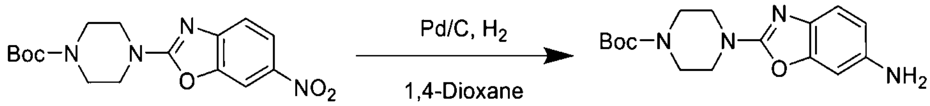


**21-1** **21-2**

In the same manner as step 3-3, *tert*-butyl 4-(6-nitrobenzo[*d*]oxazol-2-yl)piperazine-1-carboxylate (100 mg, 0.29 mmol) and 10% Pd/C (61 mg, 0.06 mmol) were dissolved in 1,4-dioxane, and 43 mg of the title compound (47% yield) was obtained through hydrogen.

$^1H$ NMR (400 MHz, Chloroform-d) δ 7.15 (d, J = 8.3 Hz, 1H), 6.68 (d, J = 2.2 Hz, 1H), 6.55 (dd, J = 8.3, 2.2 Hz, 1H), 3.64 - 3.52 (m, 10H), 1.49 (s, 9H).

Step 21-3: Preparation of *tert*-butyl 4-(6-(2-(dicyanomethylene)hydrazinyl)benzo[d]oxazol-2-yl)piperazine-1-carboxylate **(Compound 21)**

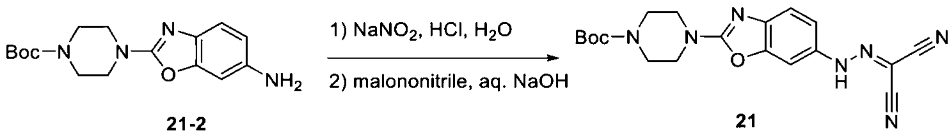


**21-2** **21**

In the same manner as step 1-2, *tert*-butyl 4-(6-aminobenzo[*d*]oxazol-2-yl)piperazine-1-carboxylate (30 mg, 0.09 mmol), sodium nitrite (10 mg, 0.14 mmol), and 1.0 M hydrochloric acid aqueous solution (0.3 mL, 0.28 mmol) were dissolved in distilled water, and then malononitrile (12 mg, 0.19 mmol) and an aqueous sodium hydroxide solution were used to obtain 36 mg of the title compound (96% yield).

$^1H$ NMR (400 MHz, Chloroform-d) δ 9.79 (s, 1H), 7.37 (d, J = 2.1 Hz, 1H), 7.33 (d, J = 8.4 Hz, 1H), 7.07 (dd, J = 8.4, 2.2 Hz, 1H), 3.69 (dd, J = 6.7, 3.8 Hz, 4H), 3.59 (dd, J = 6.5, 3.8 Hz, 4H), 1.50 (s, 9H).

**Example 22: Preparation of tert-butyl 4-(6-(2-(dicyanomethylene)hydrazinyl)benzo[d]thiazol-2-yl)piperazine-1-carboxylate**

Step 22-1: Preparation of *tert*-butyl 4-(6-aminobenzo[d]thiazol-2-yl)piperazine-1-carboxylate **(Compound 22-1)**

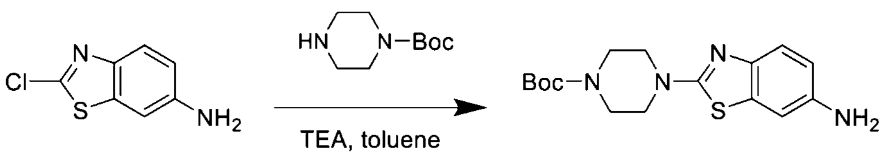


**22-1**

In the same manner as step 9-1, 2-chloro-6-benzothiazolamine (200 mg, 1.08 mmol), 1-Boc-piperazine (403 mg, 2.17 mmol), triethylamine (453 μL, 3.20 mmol), and toluene were used to obtain 223 mg of the title compound (62% yield).

$^1H$ NMR (400 MHz, Chloroform-d) δ 7.37 (d, J = 8.5 Hz, 1H), 6.95 (d, J = 2.4 Hz, 1H), 6.69 (dd, J = 8.5, 2.4 Hz, 1H), 3.62 - 3.48 (m, 10H), 1.48 (s, 9H).

Step 22-2: Preparation of *tert*-butyl 4-(6-(2-(dicyanomethylene)hydrazinyl)benzo[d]thiazol-2-yl)piperazine-1-carboxylate **(Compound 22)**

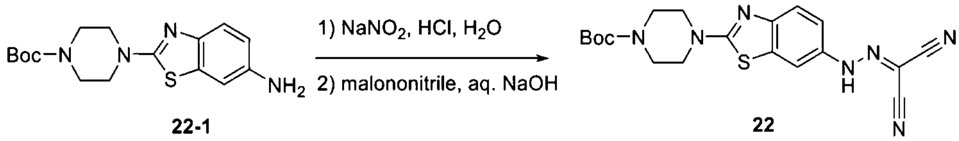


22-1 22

In the same manner as step 1-2, *tert*-butyl 4-(6-aminobenzo[*d*]thiazol-2-yl)piperazine-1-carboxylate (200 mg, 0.60 mmol), sodium nitrite (62 mg, 0.90 mmol), and 1.0 M hydrochloric acid aqueous solution (1.8 mL, 1.79 mmol) were dissolved in distilled water, and then malononitrile (79 mg, 1.20 mmol) and an aqueous sodium hydroxide solution were used to obtain 24 mg of the title compound (98% yield).

$^1$H NMR (400 MHz, Chloroform-d) δ 9.95 (s, 1H), 7.66 (d, J = 2.3 Hz, 1H), 7.54 (d, J = 8.7 Hz, 1H), 7.22 (dd, J = 8.7, 2.3 Hz, 1H), 3.68 - 3.50 (m, 8H), 1.49 (s, 8H).

**Example 23: Preparation of N-(2-(8-oxa-3-azabicyclo[3.2.1]octan-3-yl)benzo[d]thiazol-6-yl)carbonohydrazonoyl dicyanide**

Step 23-1: Preparation of 2-(8-oxa-3-azabicyclo[3.2.1]octan-3-yl)benzo[*d*]thiazol-6-amine **(Compound 23-1)**

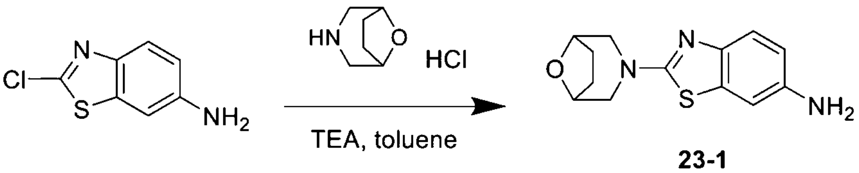


23-1

In the same manner as step 9-1, 2-chloro-6-benzothiazolamine (200 mg, 1.08 mmol), 8-oxa-3-azabicyclo[3.2.1] octane hydrochloride (324 mg, 2.17 mmol), triethylamine (453 μL, 3.25 mmol), and toluene were used to obtain 110 mg of the title compound (39% yield).

$^1$H NMR (400 MHz, Chloroform-d) δ 7.36 (d, J = 8.5 Hz, 1H), 6.95 (d, J = 2.4 Hz, 1H), 6.69 (dd, J = 8.5, 2.4 Hz, 1H), 4.47 (d, J = 2.7 Hz, 2H), 3.64 - 3.54 (m, 4H), 3.41 (dd, J = 12.2, 2.6 Hz, 2H), 2.07 - 1.81 (m, 4H).

Step 23-2: Preparation of *N*-(2-(8-oxa-3-azabicyclo[3.2.1]octan-3-yl)benzo[d]thiazol-6-yl)carbonohydrazonoyl dicyanide **(Compound 23)**

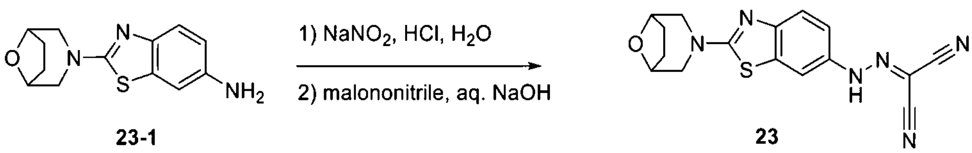


23-1 23

In the same manner as step 1-2, 2-(8-oxa-3-azabicyclo[3.2.1]octan-3-yl)benzo[*d*]thiazol-6-amine (100 mg, 0.38 mmol), sodium nitrite (40 mg, 0.57 mmol), and 1.0 M hydrochloric acid aqueous solution (1.1 mL, 1.15 mmol) were dissolved in distilled water, and then malononitrile (51 mg, 0.76 mmol) and an aqueous sodium hydroxide solution were used to obtain 117 mg of the title compound (91% yield).

$^1$H NMR (400 MHz, DMSO-d6) δ 13.11 (s, 1H), 7.90 (d, J = 2.0 Hz, 1H), 7.49 - 7.38 (m, 2H), 4.45 (d, J = 4.8 Hz, 2H), 3.63 (d, J = 12.2 Hz, 2H), 2.00 - 1.68 (m, 4H).

**Example 24: Preparation of N-(2-(piperazin-1-yl)benzo[d]thiazol-6-yl)carbonohydrazonoyl dicyanide**

Step 24-1: Preparation of 2-(piperazin-1-yl)benzo[*d*]thiazol-6-amine **(Compound 24-1)**

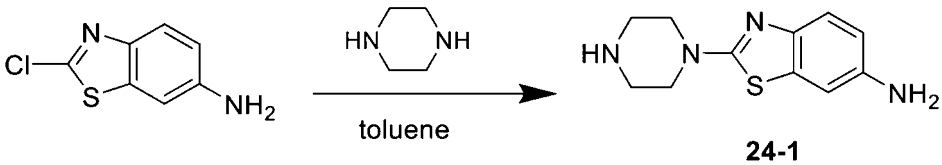


**24-1**

In the same manner as step 9-1, 2-chloro-6-benzothiazolamine (200 mg, 1.08 mmol), piperazine (187 mg, 2.17 mmol), and toluene were used to obtain 90 mg of the title compound (35% yield).

$^1$H NMR (400 MHz, Chloroform-d) δ 7.36 (d, J = 8.5 Hz, 1H), 6.95 (d, J = 2.4 Hz, 1H), 6.68 (dd, J = 8.5, 2.4 Hz, 1H), 3.63 - 3.46 (m, 6H), 3.03 - 2.93 (m, 4H).

Step 24-2: Preparation of *N*-(2-(piperazin-1-yl)benzo[d]thiazol-6-yl)carbonohydrazonoyl dicyanide **(Compound 24)**

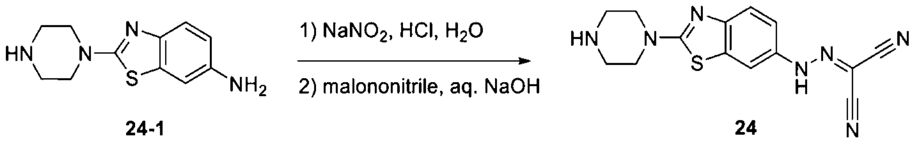


In the same manner as step 1-2, 2-(piperazin-1-yl)benzo[d]thiazol-6-amine (100 mg, 0.43 mmol), sodium nitrite (44 mg, 0.64 mmol), and 1.0 M hydrochloric acid aqueous solution (1.3 mL, 1.28 mmol) were dissolved in distilled water, and then malononitrile (56 mg, 0.85 mmol) and an aqueous sodium hydroxide solution were used to obtain 42 mg of the title compound (31% yield).

$^1$H NMR (400 MHz, Chloroform-d) δ 9.76 (s, 1H), 7.70 (d, J = 2.3 Hz, 1H), 7.59 (d, J = 8.7 Hz, 1H), 7.26 - 7.21 (m, 1H), 4.51 - 4.44 (m, 2H), 4.00 (dd, J = 6.5, 4.6 Hz, 2H), 3.94 (dd, J = 6.3, 4.5 Hz, 2H), 3.70 (dd, J = 6.5, 4.6 Hz, 2H).

**Example 25: Preparation of N-(2-(piperazin-1-yl)benzo[d]oxazol-6-yl)carbonohydrazonoyl dicyanide**

Step 25-1: Preparation of 6-nitro-2-(piperazin-1-yl)benzo[*d*]oxazole **(Compound 25-1)**

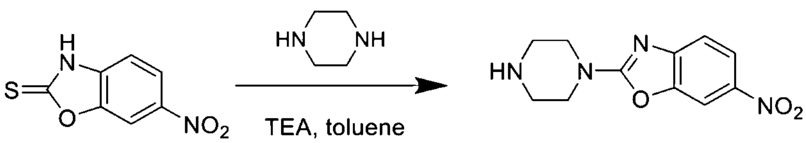


**4-1** **25-1**

In the same manner as step 9-1, 6-nitrobenzo[*d*]oxazole-2(3*H*)-thione (300 mg, 1.53 mmol), piperazine (198 mg, 2.29 mmol), triethylamine (639 μL, 4.59 mmol), and toluene were used to obtain 124 mg of the title compound (33% yield).

$^1$H NMR (400 MHz, DMSO-d6) δ 8.27 (d, J = 2.3 Hz, 1H), 8.14 (dd, J = 8.7, 2.3 Hz, 1H), 7.38 (d, J = 8.7 Hz, 1H), 3.61 (t, J = 5.1 Hz, 4H), 2.81 (t, J = 5.1 Hz, 4H), 2.65 (s, 2H).

Step 25-2: Preparation of 2-(piperazin-1-yl)benzo[*d*]oxazol-6-amine **(Compound 25-2)**

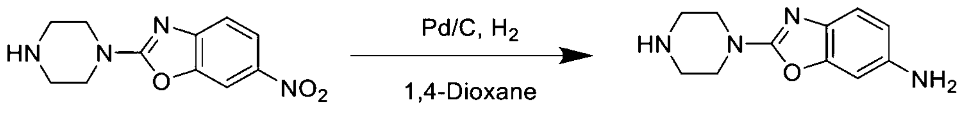


25-1 25-2

In the same manner as step 3-3, 6-nitro-2-(piperazin-1-yl)benzo[*d*]oxazole (100 mg, 0.40 mmol) and 10% Pd/C (86 mg, 0.08 mmol) were dissolved in 1,4-dioxane, and 50 mg of the title compound (57% yield) was obtained through hydrogen.

$^1H$ NMR (400 MHz, Chloroform-d) δ 7.14 (d, J = 8.3 Hz, 1H), 6.67 (d, J = 2.2 Hz, 1H), 6.54 (dd, J = 8.3, 2.2 Hz, 1H), 3.71 - 3.54 (m, 6H), 3.05 - 2.89 (m, 4H).

Step 25-3: Preparation of *N*-(2-(piperazin-1-yl)benzo[d]oxazol-6-yl)carbonohydrazonoyl dicyanide **(Compound 25)**

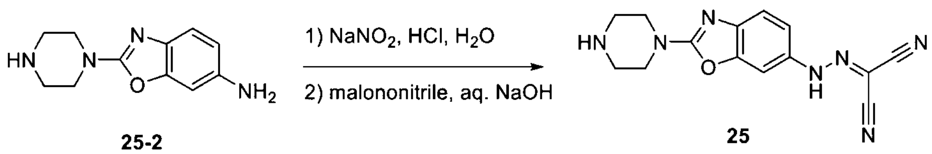


25-2 25

In the same manner as step 1-2, 2-(piperazin-1-yl)benzo[*d*]oxazol-6-amine (50 mg, 0.23 mmol), sodium nitrite (24 mg, 0.34 mmol), and 1.0 M hydrochloric acid aqueous solution (0.7 mL, 0.69 mmol) were dissolved in distilled water, and then malononitrile (30 mg, 0.46 mmol) and an aqueous sodium hydroxide solution were used to obtain 14 mg of the title compound (20% yield).

$^1H$ NMR (400 MHz, DMSO-d6) δ 13.10 (s, 1H), 7.54 (s, 1H), 7.35 (s, 1H), 4.45 - 4.39 (m, 2H), 3.90 (q, J = 5.1 Hz, 4H), 3.68 (dd, J = 6.6, 4.5 Hz, 2H).

**Example 26: Preparation of N-(2-(piperazin-1-yl)benzo[d]oxazol-6-yl)carbonohydrazonoyl dicyanide**

Step 25-1: Preparation of 2-(8-oxa-3-azabicyclo[3.2.1]octan-3-yl)-6-nitrobenzo[*d*]oxazole **(Compound 25-1)**

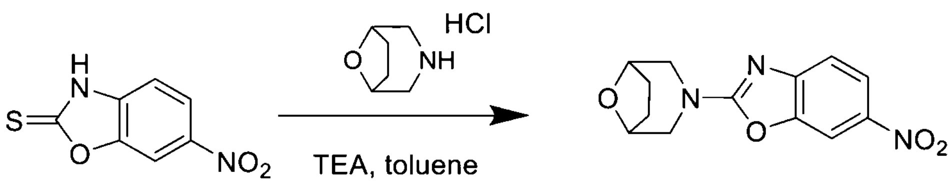


4-1 26-1

In the same manner as step 9-1, 6-nitrobenzo[*d*]oxazole-2(3*H*)-thione (300 mg, 1.53 mmol), 8-oxa-3-azabicyclo [3.2.1]octane hydrochloride (343 mg, 2.29 mmol), triethylamine (639 μL, 4.59 mmol), and toluene were used to obtain 142 mg of the title compound (34% yield).

$^1H$ NMR (400 MHz, Chloroform-d) δ 8.19 (dd, J = 8.7, 2.2 Hz, 1H), 8.14 (d, J = 2.2 Hz, 1H), 7.33 (d, J = 8.7 Hz, 1H), 4.52 (d, J = 2.6 Hz, 2H), 3.91 (d, J = 13.1 Hz, 2H), 3.53 (dd, J = 12.8, 2.5 Hz, 2H), 2.12 - 2.01 (m, 2H), 1.96 - 1.83 (m, 2H).

Step 26-2: Preparation of 2-(8-oxa-3-azabicyclo[3.2.1]octan-3-yl)benzo[d]oxazol-6-amine **(Compound 26-2)**

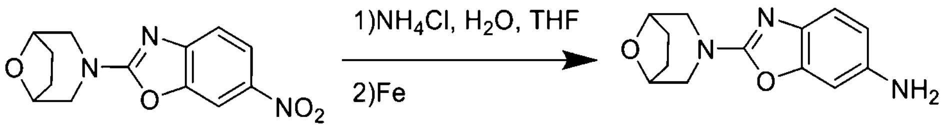


26-1 26-2

In the same manner as step 5-3, 2-(8-oxa-3-azabicyclo[3.2.1]octan-3-yl)-6-nitrobenzo[d]oxazole (120 mg, 0.44 mmol) and ammonium chloride (187 mg, 3.49 mmol) were dissolved in distilled water and tetrahydrofuran, and then iron (195 mg, 3.49 mmol) was used in the reaction mixture to obtain 33 mg of the title compound (30% yield).
$^1$H NMR (400 MHz, Chloroform-d) δ 7.14 (d, J = 8.2 Hz, 1H), 6.67 (d, J = 2.2 Hz, 1H), 6.54 (dd, J = 8.2, 2.2 Hz, 1H), 4.46 (d, J = 2.7 Hz, 1H), 3.85 - 3.67 (m, 2H), 3.66 - 3.47 (m, 2H), 3.40 (dd, J = 12.5, 2.5 Hz, 2H), 2.15 - 1.75 (m, 4H).

Step 26-3: Preparation of N-(2-(8-oxa-3-azabicyclo[3.2.1]octan-3-yl)benzo[*d*]oxazol-6-yl)carbonohydrazonoyl dicyanide **(Compound 26)**

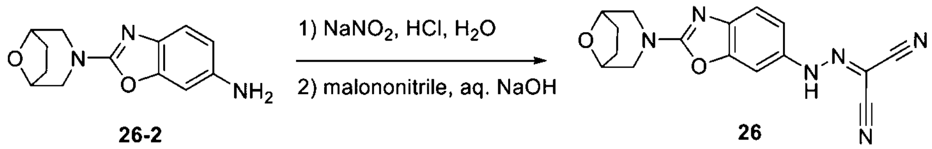


26-2 26

In the same manner as step 1-2, 2-(8-oxa-3-azabicyclo[3.2.1]octan-3-yl)benzo[d]oxazol-6-amine (20 mg, 0.08 mmol), sodium nitrite (8 mg, 0.12 mmol), and 1.0 M hydrochloric acid aqueous solution (0.2 mL, 0.24 mmol) were dissolved in distilled water, and then malononitrile (11 mg, 0.16 mmol) and an aqueous sodium hydroxide solution were used to obtain 6 mg of the title compound (24% yield).
$^1$H NMR (400 MHz, DMSO-d6) δ 13.09 (s, 1H), 7.50 (d, J = 2.0 Hz, 1H), 7.36 - 7.26 (m, 2H), 4.44 (s, 3H), 3.71 (d, J = 12.4 Hz, 3H), 1.98 - 1.65 (m, 6H).

**Example 27: Preparation of N-(2-(4-isopropylpiperazin-1-yl)benzo[d]thiazol-6-yl)carbonohydrazonoyl dicyanide**

Step 27-1: Preparation of 2-(4-isopropylpiperazin-1-yl)benzo[*d*]thiazol-6-amine **(Compound 27-1)**

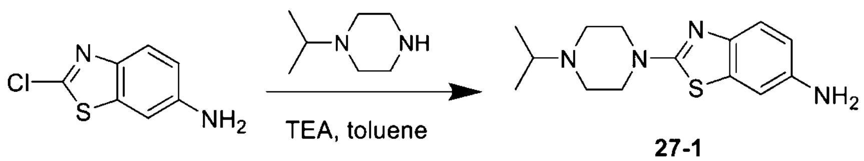


27-1

In the same manner as step 9-1, 2-chloro-6-benzothiazolamine (200 mg, 1.08 mmol), 1-isopropylpiperazine (310 μL, 2.17 mmol), triethylamine (249 μL, 3.25 mmol), and toluene were used to obtain 238 mg of the title compound (79% yield).
$^1$H NMR (400 MHz, Chloroform-d) δ 7.36 (d, J = 8.5 Hz, 1H), 6.95 (d, J = 2.3 Hz, 1H), 6.68 (dd, J = 8.5, 2.4 Hz, 1H), 3.58 (t, J = 5.2 Hz, 6H), 2.75 (p, J = 6.5 Hz, 1H), 2.63 (t, J = 5.1 Hz, 4H), 1.07 (d, J = 6.5 Hz, 6H).

Step 27-2: Preparation of *N*-(2-(4-isopropylpiperazin-1-yl)benzo[d]thiazol-6-yl)carbonohydrazonoyl dicyanide **(Compound 27)**

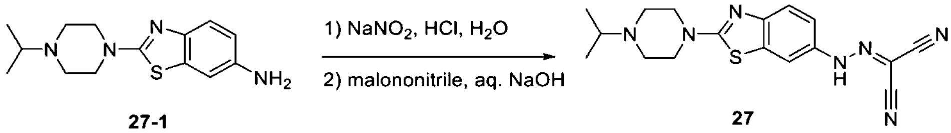


27-1 27

In the same manner as step 1-2, 2-(4-isopropylpiperazin-1-yl)benzo[*d*]thiazol-6-amine (200 mg, 0.72 mmol), sodium nitrite (75 mg, 1.08 mmol), and 1.0 M hydrochloric acid aqueous solution (2.2 mL, 2.17 mmol) were dissolved in distilled water, and then malononitrile (96 mg, 1.45 mmol) and an aqueous sodium hydroxide solution were used to obtain 51 mg of the title compound (20% yield).

$^1$H NMR (400 MHz, Chloroform-d) δ 7.64 (d, J = 2.3 Hz, 1H), 7.52 (d, J = 8.7 Hz, 1H), 7.19 (dd, J = 8.7, 2.4 Hz, 1H), 3.66 (t, J = 5.1 Hz, 4H), 2.78 (p, J = 6.5 Hz, 1H), 2.65 (t, J = 5.2 Hz, 4H), 1.08 (d, J = 6.5 Hz, 6H).

**Example 28: Preparation of N-(2-(piperazin-1-yl)benzo[d]oxazol-6-yl)carbonohydrazonoyl dicyanide**

Step 28-1: Preparation of 2-(4-isopropylpiperazin-1-yl)-6-nitrobenzo[d]oxazole **(Compound 28-1)**

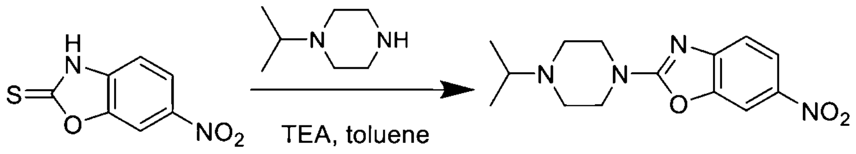


4-1 28-1

In the same manner as step 9-1, 6-nitrobenzo[*d*]oxazole-2(3*H*)-thione (300 mg, 1.53 mmol), 1-isopropylpiperazine (328 μL, 2.29 mmol), triethylamine (351 μL, 4.59 mmol), and toluene were used to obtain 267 mg of the title compound (49% yield).

$^1$H NMR (400 MHz, Chloroform-d) δ 8.18 (dd, J = 8.7, 2.2 Hz, 1H), 8.13 (d, J = 2.2 Hz, 1H), 7.31 (d, J = 8.7 Hz, 1H), 3.79 (t, 4H), 2.80 (p, J = 6.6 Hz, 1H), 2.66 (t, 4H), 1.08 (d, J = 6.5 Hz, 6H).

Step 28-2: Preparation of 2-(4-isopropylpiperazin-1-yl)benzo[d]oxazol-6-amine **(Compound 28-2)**

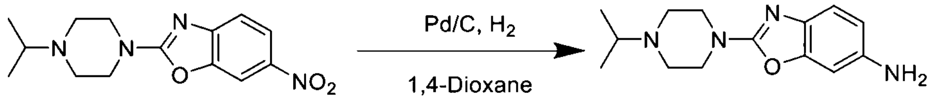


28-1 28-2

In the same manner as step 3-3, 2-(4-isopropylpiperazin-1-yl)-6-nitrobenzo[*d*]oxazole (200 mg, 0.69 mmol) and 10% Pd/C (147 mg, 0.14 mmol) were dissolved in 1,4-dioxane, and 148 mg of the title compound (82% yield) was obtained through hydrogen.

$^1$H NMR (400 MHz, Chloroform-d) δ 7.14 (d, J = 8.3 Hz, 1H), 6.67 (d, J = 2.2 Hz, 1H), 6.53 (dd, J = 8.3, 2.2 Hz, 1H), 3.69 - 3.61 (m, 4H), 3.57 (s, 2H), 2.75 (p, J = 6.6 Hz, 1H), 2.62 (t, J = 5.1 Hz, 4H), 1.07 (d, J = 6.5 Hz, 6H).

Step 28-3: Preparation of *N*-(2-(4-isopropylpiperazin-1-yl)benzo[*d*]oxazol-6-yl)carbonohydrazonoyl dicyanide **(Compound 28)**

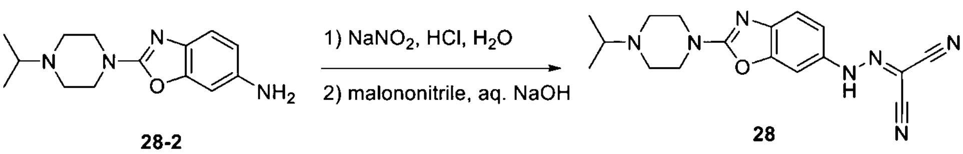


28-2 28

In the same manner as step 1-2, 2-(4-isopropylpiperazin-1-yl)benzo[d]oxazol-6-amine (100 mg, 0.38 mmol), sodium nitrite (40 mg, 0.58 mmol), and 1.0 M hydrochloric acid aqueous solution (1.1 mL, 1.15 mmol) were dissolved in distilled water, and then malononitrile (51 mg, 0.77 mmol) and an aqueous sodium hydroxide solution were used to obtain 123 mg of the title compound (95% yield).

$^1$H NMR (400 MHz, Acetone-d6) δ 7.51 (d, J = 2.1 Hz, 1H), 7.35 (dd, J = 8.5, 2.1 Hz, 1H), 7.26 (d, J = 8.5 Hz, 1H), 3.72 - 3.68 (m, 4H), 2.85 (p, J = 6.6 Hz, 1H), 2.74 - 2.67 (m, 4H), 1.08 (d, J = 6.6 Hz, 6H).

**Example 29: Preparation of tert-butyl (2S,6R)-4-(6-(2-(dicyanomethylene)hydrazinyl)benzo[d]thiazol-2-yl)-2,6-dimethylpiperazine-1-carboxylate**

Step 29-1: Preparation of *tert*-butyl (2S,6R)-4-(6-aminobenzo[d]thiazol-2-yl)-2,6-dimethylpiperazine-1-carboxylate **(Compound 29-1)**

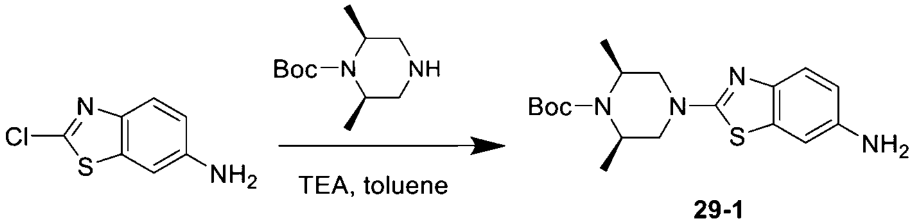


**29-1**

In the same manner as step 9-1, 2-chloro-6-benzothiazolamine (300 mg, 1.62 mmol), tert-butyl (2S,6R)-2,6-dimethylpiperazine-1-carboxylate (696 mg, 3.25 mmol), triethylamine (679 μL, 4.87 mmol), and toluene were used to obtain 76 mg of the title compound (13% yield).

$^1$H NMR (400 MHz, DMSO-d6) δ 7.16 (d, J = 8.5 Hz, 1H), 6.89 (d, J = 2.3 Hz, 1H), 6.56 (dd, J = 8.5, 2.3 Hz, 1H), 4.90 (s, 2H), 4.15 (p, J = 6.6 Hz, 2H), 3.78 (d, J = 12.9 Hz, 2H), 3.22 (dd, J = 12.9, 4.5 Hz, 2H), 1.43 (s, 8H), 1.19 (d, J = 6.8 Hz, 6H).

Step 29-2: Preparation of *tert*-butyl (2S,6R)-4-(6-(2-(dicyanomethylene)hydrazinyl)benzo[d]thiazol-2-yl)-2,6-dimethylpiperazine-1-carboxylate **(Compound 29)**

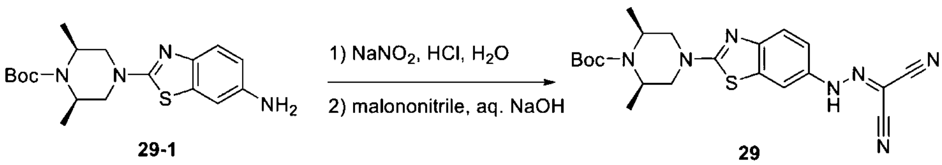


**29-1** **29**

In the same manner as step 1-2, *tert*-butyl (2S,6R)-4-(6-aminobenzo[d]thiazol-2-yl)-2,6-dimethylpiperazine-1-carboxylate (60 mg, 0.17 mmol), sodium nitrite (17 mg, 0.25 mmol), and 1.0 M hydrochloric acid aqueous solution (0.5 mL, 0.50 mmol) were dissolved in distilled water, and then malononitrile (22 mg, 0.33 mmol) and an aqueous sodium hydroxide solution were used to obtain 61 mg of the title compound (84% yield).

$^1$H NMR (400 MHz, Chloroform-d) δ 7.64 (d, J = 2.3 Hz, 1H), 7.52 (d, J = 8.7 Hz, 1H), 7.21 (dd, J = 8.7, 2.4 Hz, 1H), 4.41 - 4.25 (m, 2H), 3.94 (d, J = 13.0 Hz, 2H), 3.39 (dd, J = 13.1, 4.7 Hz, 2H), 1.50 (s, 9H), 1.32 (d, J = 6.9 Hz, 6H).

**Example 30: Preparation of N-(2-(4-isopropylpiperazin-1-yl)benzo[d]thiazol-6-yl)carbonohydrazonoyl dicyanide**

Step 30-1: Preparation of 1-(4-(6-aminobenzo[*d*]thiazol-2-yl)piperazin-1-yl)ethan-1-one **(Compound 30-1)**

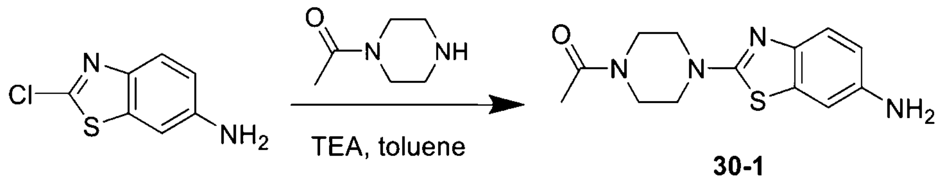


30-1

In the same manner as step 9-1, 2-chloro-6-benzothiazolamine (200 mg, 1.08 mmol), 1-acetylpiperazine (278 mg, 2.17 mmol), triethylamine (453 μL, 3.25 mmol), and toluene were used to obtain 208 mg of the title compound (69% yield).

$^1H$ NMR (400 MHz, Chloroform-d) δ 7.38 (d, J = 8.5 Hz, 1H), 6.97 (d, J = 2.3 Hz, 1H), 6.70 (dd, J = 8.5, 2.3 Hz, 1H), 3.76 (d, J = 5.5 Hz, 2H), 3.66 - 3.49 (m, 8H), 2.15 (s, 3H).

Step 30-2: Preparation of *N*-(2-(4-acetylpiperazin-1-yl)benzo[d]thiazol-6-yl)carbonohydrazonoyl dicyanide **(Compound 30)**

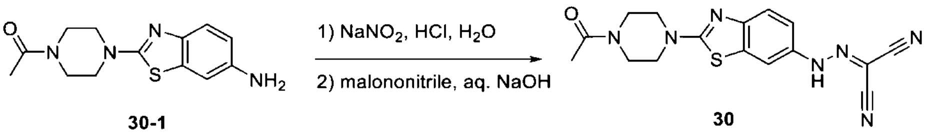


In the same manner as step 1-2, 1-(4-(6-aminobenzo[d]thiazol-2-yl)piperazin-1-yl)ethan-1-one (180 mg, 0.65 mmol), sodium nitrite (67 mg, 0.98 mmol), and 1.0 M hydrochloric acid aqueous solution (2.0 mL, 1.95 mmol) were dissolved in distilled water, and then malononitrile (86 mg, 1.30 mmol) and an aqueous sodium hydroxide solution were used to obtain 117 mg of the title compound (51% yield).

$^1H$ NMR (400 MHz, DMSO-d6) δ 13.10 (s, 1H), 7.91 (d, J = 2.2 Hz, 1H), 7.53 - 7.36 (m, 2H), 3.64 - 3.52 (m, 8H), 2.06 (s, 3H).

**Example 31: Preparation of N-(2-(4-acetylpiperazin-1-yl)benzo[d]oxazol-6-yl)carbonohydrazonoyl dicyanide**

Step 31-1: Preparation of 1-(4-(6-nitrobenzo[d]oxazol-2-yl)piperazin-1-yl)ethan-1-one **(Compound 31-1)**

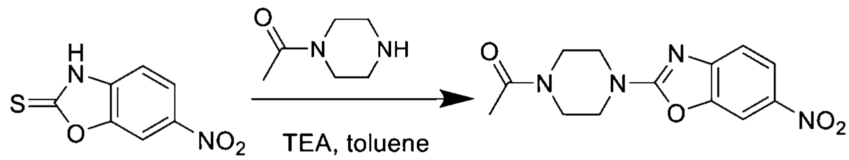


4-1 31-1

In the same manner as step 9-1, 6-nitrobenzo[*d*]oxazole-2(3H)-thione (300 mg, 1.53 mmol), 1-acetylpiperazine (392 mg, 3.06 mmol), triethylamine (639 μL, 4.59 mmol), and toluene were used to obtain 153 mg of the title compound (34% yield).

$^1H$ NMR (400 MHz, Chloroform-d) δ 8.23 - 8.15 (m, 2H), 7.36 (d, J = 8.6 Hz, 1H), 3.89 - 3.73 (m, 6H), 3.69 - 3.57 (m, 2H), 2.18 (s, 3H).

Step 31-2: Preparation of 1-(4-(6-aminobenzo[d]oxazol-2-yl)piperazin-1-yl)ethan-1-one **(Compound 31-2)**

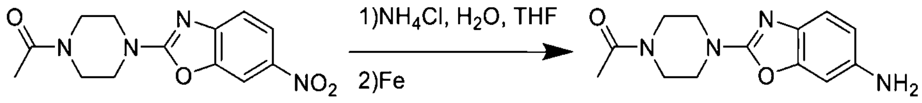


**31-1** **31-2**

In the same manner as step 5-3, 1-(4-(6-nitrobenzo[d]oxazol-2-yl)piperazin-1-yl)ethan-1-one (120 mg, 0.41 mmol) and ammonium chloride (177 mg, 3.31 mmol) were dissolved in distilled water and tetrahydrofuran, and then iron (185 mg, 3.31 mmol) was used in the reaction mixture to obtain 33 mg of the title compound (31% yield).

$^1H$ NMR (400 MHz, DMSO-d6) δ 6.98 (d, J = 8.3 Hz, 1H), 6.63 (d, J = 2.2 Hz, 1H), 6.42 (dd, J = 8.3, 2.2 Hz, 1H), 4.90 (s, 2H), 3.63 - 3.40 (m, 8H), 2.05 (s, 3H).

Step 31-3: Preparation of *N*-(2-(4-acetylpiperazin-1-yl)benzo[d]oxazol-6-yl)carbonohydrazonoyl dicyanide **(Compound 31)**

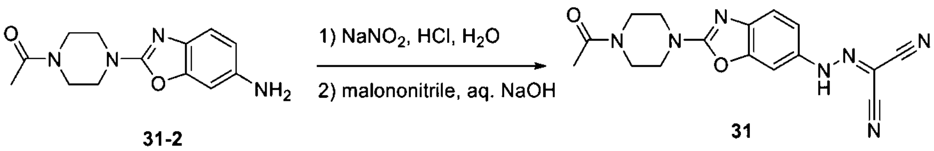


**31-2** **31**

In the same manner as step 1-2, 1-(4-(6-aminobenzo[d]oxazol-2-yl)piperazin-1-yl)ethan-1-one (20 mg, 0.08 mmol), sodium nitrite (8 mg, 0.12 mmol), and 1.0 M hydrochloric acid aqueous solution (0.2 mL, 0.23 mmol) were dissolved in distilled water, and then malononitrile (10 mg, 0.15 mmol) and an aqueous sodium hydroxide solution were used to obtain 25 mg of the title compound (98% yield).

$^1H$ NMR (400 MHz, Chloroform-d) δ 10.12 (s, 1H), 7.42 (d, J = 2.1 Hz, 1H), 7.35 (d, J = 8.4 Hz, 1H), 7.13 (dd, J = 8.4, 2.2 Hz, 1H), 3.84 - 3.69 (m, 6H), 3.70 - 3.58 (m, 2H), 2.18 (s, 3H).

**Example 32: Preparation of N-(1-ethyl-2-morpholino-1H-benzo[d]imidazol-6-yl)carbonohydrazonoyl dicyanide (Compound 32)**

Step 32-1: 4-(1-ethyl-6-nitro-1*H*-benzo[*d*]imidazol-2-yl)morpholine **(Compound 32-1)**

4-(1-ethyl-5-nitro-1*H*-benzo[*d*]imidazol-2-yl)morpholine **(Compound 32-2)**

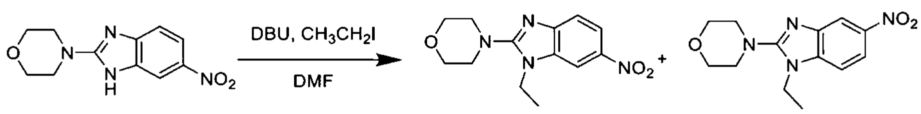


**5-1** **32-1** **32-2**

In the same manner as step 5-1, 4-(6-nitro-1*H*-benzo[*d*]imidazol-2-yl)morpholine (200 mg, 0.81 mmol), DBU (361 μL, 2.42 mmol), ethyl iodide (194 μL, 2.42 mmol), and dimethylformamide were used to obtain 43 mg of the title compound (32-1) (19% yield) and 30 mg of the title compound (32-2) (14% yield).

**Compound 32-1:** $^1H$ NMR (400 MHz, Chloroform-d) δ 8.21 - 8.11 (m, 2H), 7.58 (d, J = 8.7 Hz, 1H), 4.13 (q, J = 7.3 Hz, 2H), 3.94 - 3.86 (m, 4H), 3.48 - 3.38 (m, 4H), 1.53 (t, J = 7.3 Hz, 3H).

**Compound 32-2:** $^1H$ NMR (400 MHz, Chloroform-d) δ 8.48 (d, J = 2.2 Hz, 1H), 8.13 (dd, J = 8.9, 2.2 Hz, 1H), 7.28 - 7.25 (m, 1H), 4.19 - 4.07 (m, 2H), 3.94 - 3.86 (m, 4H), 3.40 - 3.34 (m, 4H), 1.50 (t, J = 7.3 Hz, 3H).

Step 32-2: Preparation of 1-ethyl-2-morpholino-1*H*-benzo[*d*]imidazol-6-amine **(Compound 32-3)**

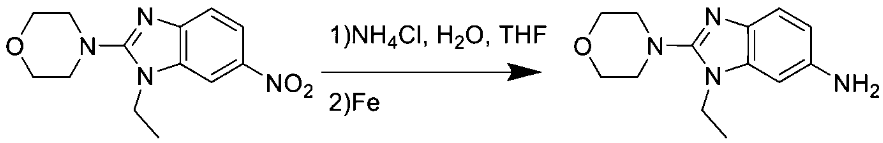


32-1

32-3

In the same manner as step 5-3, 4-(1-ethyl-6-nitro-1*H*-benzo[*d*]imidazol-2-yl)morpholine (70 mg, 0.25 mmol) and ammonium chloride (108 mg, 2.03 mmol) were dissolved in distilled water and tetrahydrofuran, and then iron (113 mg, 2.03 mmol) was used in the reaction mixture to obtain 34 mg of the title compound (54% yield).

$^1$H NMR (400 MHz, DMSO-d6) δ 7.10 (d, J = 8.4 Hz, 1H), 6.52 (dd, J = 2.1, 0.6 Hz, 1H), 6.42 (dd, J = 8.4, 2.1 Hz, 1H), 4.79 (s, 2H), 3.94 (q, J = 7.2 Hz, 2H), 3.81 - 3.68 (m, 4H), 3.12 - 2.99 (m, 4H), 1.36 (s, 2H), 1.30 (t, J = 7.2 Hz, 3H).

Step 32-3: Preparation of *N*-(1-ethyl-2-morpholino-1*H*-benzo[*d*]imidazol-6-yl)carbonohydrazonoyl dicyanide **(Compound 32)**

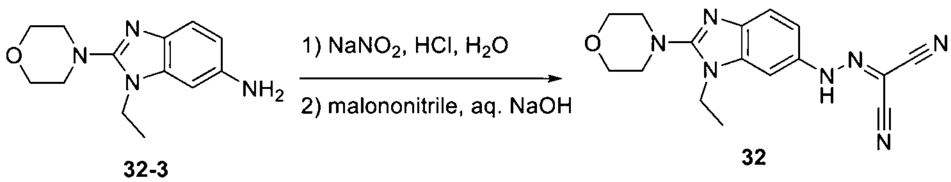


32-3

32

In the same manner as step 1-2, 1-ethyl-2-morpholino-1*H*-benzo[*d*]imidazol-6-amine (30 mg, 0.12 mmol), sodium nitrite (13 mg, 0.18 mmol), and 1.0 M hydrochloric acid aqueous solution (0.4 mL, 0.36 mmol) were dissolved in distilled water, and then malononitrile (16 mg, 0.24 mmol) and an aqueous sodium hydroxide solution were used to obtain 22 mg of the title compound (55% yield).

$^1$H NMR (400 MHz, DMSO-d6) δ 13.15 (s, 1H), 7.50 (s, 1H), 7.44 (d, J = 8.5 Hz, 1H), 7.35 - 7.27 (m, 1H), 4.12 (q, J = 7.1 Hz, 2H), 3.79 (t, J = 4.6 Hz, 4H), 3.29 - 3.23 (m, 4H), 1.37 (t, J = 7.3 Hz, 3H).

**Example 33: Preparation of N-(1-ethyl-2-morpholino-1H-benzo[d]imidazol-5-yl)carbonohydrazonoyl dicyanide (Compound 33)**

Step 33-1: Preparation of 1-ethyl-2-morpholino-1*H*-benzo[*d*]imidazol-5-amine **(Compound 33-1)**

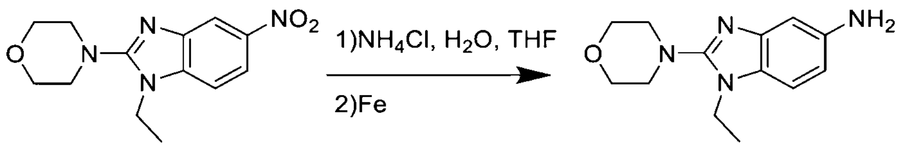


32-2

33-1

In the same manner as step 5-3, 4-(1-ethyl-5-nitro-1*H*-benzo[*d*]imidazol-2-yl)morpholine (80 mg, 0.29 mmol) and ammonium chloride (124 mg, 2.32 mmol) were dissolved in distilled water and tetrahydrofuran, and then iron (129 mg, 2.32 mmol) was used in the reaction mixture to obtain 48 mg of the title compound (68% yield).

$^1$H NMR (400 MHz, DMSO-d6) δ 7.05 (d, J = 8.4 Hz, 1H), 6.64 (d, J = 2.0 Hz, 1H), 6.45 (dd, J = 8.4, 2.1 Hz, 1H), 4.69 (s, 2H), 3.96 (q, J = 7.2 Hz, 2H), 3.80 - 3.67 (m, 4H), 3.17 - 3.05 (m, 4H), 1.36 (s, 2H), 1.30 (t, J = 7.2 Hz, 3H).

Step 33-2: Preparation of *N*-(1-ethyl-2-morpholino-1*H*-benzo[*d*]imidazol-5-yl)carbonohydrazonoyl dicyanide **(Compound 33)**

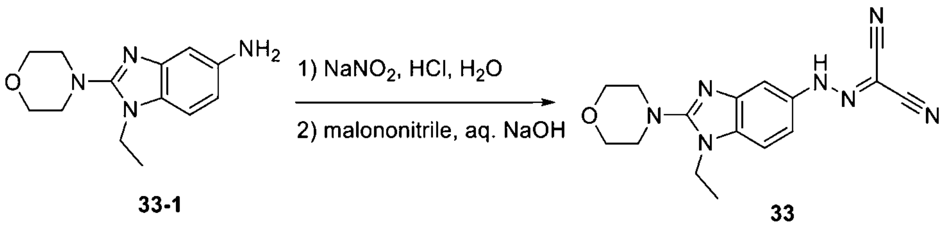


33-1 33

In the same manner as step 1-2, 1-ethyl-2-morpholino-1*H*-benzo[*d*]imidazol-5-amine (40 mg, 0.16 mmol), sodium nitrite (17 mg, 0.24 mmol), and 1.0 M hydrochloric acid aqueous solution (0.5 mL, 0.49 mmol) were dissolved in distilled water, and then malononitrile (21 mg, 0.32 mmol) and an aqueous sodium hydroxide solution were used to obtain 16 mg of the title compound (31 % yield).

$^1$H NMR (400 MHz, DMSO-d6) δ 13.06 (s, 1H), 7.47 (s, 2H), 7.31 (d, J = 8.5 Hz, 1H), 4.10 (q, J = 7.2 Hz, 2H), 3.81 - 3.74 (m, 4H), 3.27 (s, 4H), 1.36 (t, J = 7.2 Hz, 3H).

**Example 34: Preparation of N-(2-(3-oxa-8-azabicyclo[3.2.1]octan-8-yl)-1-methyl-1H-benzo[d]imidazol-6-yl)carbonohydrazonoyl dicyanide**

Step 34-1: Preparation of 8-(6-nitro-1*H*-benzo[*d*]imidazol-2-yl)-3-oxa-8-azabicyclo[3.2.1]octane **(Compound 34-1)**

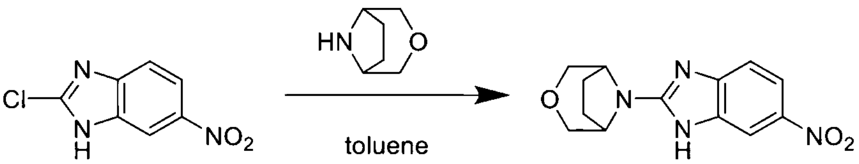


34-1

In the same manner as step 5-1, 2-chloro-6-nitro-1*H*-benzoimidazole (300 mg, 1.52 mmol), 3-oxa-8-azabicyclo [3.2.1]octane (344 mg, 3.04 mmol), and toluene were used to obtain 332 mg of the title compound (80% yield).

$^1$H NMR (400 MHz, DMSO-d6) δ 12.31 - 12.00 (m, 1H), 8.06 - 7.86 (m, 2H), 7.33 (d, J = 8.8 Hz, 1H), 4.39 (s, 2H), 3.72 (d, J = 10.9 Hz, 2H), 3.61 (d, J = 10.9 Hz, 2H), 2.05 - 1.92 (m, 4H).

Step 34-2:

8-(1-Methyl-6-nitro-1*H*-benzo[*d*]imidazol-2-yl)-3-oxa-8-azabicyclo[3.2.1]octane **(Compound 34-2)**
8-(1-Methyl-5-nitro-1*H*-benzo[*d*]imidazol-2-yl)-3-oxa-8-azabicyclo[3.2.1]octane **(Compound 34-3)**

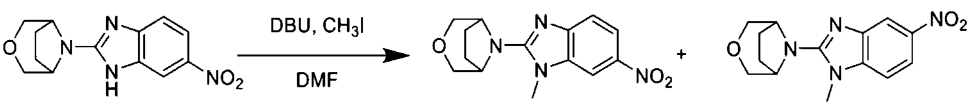


34-1 34-2 34-3

In the same manner as step 5-2, 8-(6-nitro-1*H*-benzo[*d*]imidazol-2-yl)-3-oxa-8-azabicyclo[3.2.1]octane (300 mg, 0.81 mmol), DBU (491 μL, 3.28 mmol), methyl iodide (204 μL, 3.28 mmol), and dimethylformamide were used to obtain 118 mg of the title compound (34-2) (37% yield) and 66 mg of the title compound (34-3) (21% yield).

**Compound 34-2:** $^1$H NMR (400 MHz, Chloroform-d) δ 8.17 - 8.06 (m, 2H), 7.50 (d, J = 8.6 Hz, 1H), 4.18 (s, 2H), 4.00 (d, J = 10.8 Hz, 2H), 3.75 - 3.70 (m, 5H), 2.14 (s, 4H).

**Compound 34-3:** $^1$H NMR (400 MHz, Chloroform-d) δ 8.42 (d, J = 2.2 Hz, 1H), 8.10 (dd, J = 8.8, 2.2 Hz, 1H), 7.18 (d, J = 8.8 Hz, 1H), 4.10 (s, 2H), 4.00 (d, J = 10.8 Hz, 2H), 3.76 - 3.68 (m, 5H), 2.20 - 2.07 (m, 4H).

Step 34-3: Preparation of 2-(3-oxa-8-azabicyclo[3.2.1]octan-8-yl)-1-methyl-1*H*-benzo[*d*]imidazol-6-amine **(Compound 34-4)**

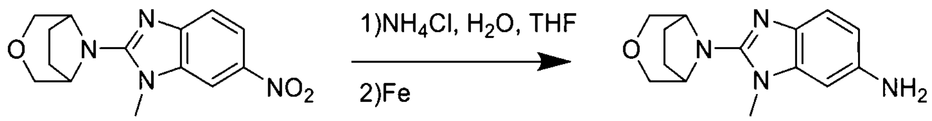


**34-2** **34-4**

In the same manner as step 5-3, 8-(1-methyl-6-nitro-1*H*-benzo[*d*]imidazol-2-yl)-3-oxa-8-azabicyclo[3.2.1]octane (200 mg, 0.69 mmol) and ammonium chloride (297 mg, 5.55 mmol) were dissolved in distilled water and tetrahydrofuran, and then iron (310 mg, 5.55 mmol) was used in the reaction mixture to obtain 170 mg of the title compound (97% yield).

$^1$H NMR (400 MHz, DMSO-d6) δ 7.03 (d, J = 8.4 Hz, 1H), 6.44 (d, J = 2.1 Hz, 1H), 6.38 (dd, J = 8.3, 2.1 Hz, 1H), 4.73 (s, 2H), 3.92 (s, 2H), 3.82 (d, J = 10.5 Hz, 2H), 3.60 - 3.55 (m, 2H), 3.49 (s, 3H), 2.02 - 1.81 (m, 4H).

Step 34-4: Preparation of *N*-(2-(3-oxa-8-azabicyclo[3.2.1]octan-8-yl)-1-methyl-1*H*-benzo[*d*]imidazol-6-yl)carbonohydrazonoyl dicyanide **(Compound 34)**

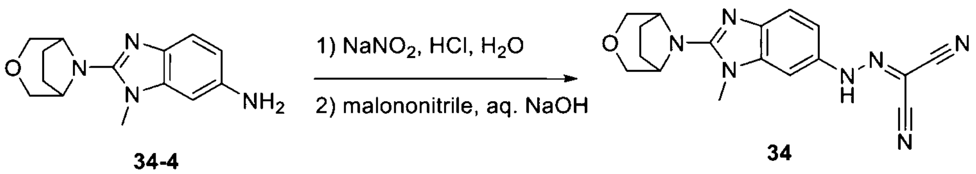


**34-4** **34**

In the same manner as step 1-2, 2-(3-oxa-8-azabicyclo[3.2.1]octan-8-yl)-1-methyl-1*H*-benzo[*d*]imidazol-6-amine (100 mg, 0.39 mmol), sodium nitrite (40 mg, 0.58 mmol), and 1.0 M hydrochloric acid aqueous solution (1.2 mL, 1.16 mmol) were dissolved in distilled water, and then malononitrile (51 mg, 0.77 mmol) and an aqueous sodium hydroxide solution were used to obtain 82 mg of the title compound (63% yield).

$^1$H NMR (400 MHz, DMSO-d6) δ 13.12 (s, 1H), 7.44 (d, J = 2.0 Hz, 1H), 7.37 (d, J = 8.5 Hz, 1H), 7.28 (dd, J = 8.6, 2.0 Hz, 1H), 4.19 (s, 2H), 3.85 (d, J = 10.8 Hz, 2H), 3.68 (s, 3H), 3.62 (dd, J = 11.0, 1.9 Hz, 2H), 2.06 - 1.85 (m, 4H).

**Example 35: Preparation of N-(2-(3-oxa-8-azabicyclo[3.2.1]octan-8-yl)-1-methyl-1H-benzo[d]imidazol-5-yl)carbonohydrazonoyl dicyanide**

Step 35-1: Preparation of 2-(3-oxa-8-azabicyclo[3.2.1]octan-8-yl)-1-methyl-1*H*-benzo[*d*]imidazol-5-amine **(Compound 35-1)**

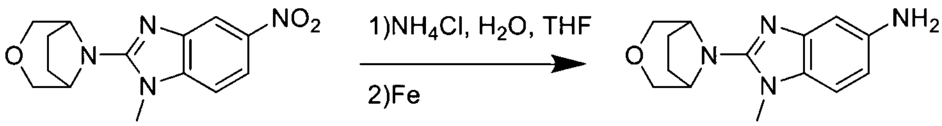


**34-3** **35-1**

In the same manner as step 5-3, 8-(1-methyl-5-nitro-1*H*-benzo[*d*]imidazol-2-yl)-3-oxa-8-azabicyclo[3.2.1]octane (130 mg, 0.45 mmol) and ammonium chloride (193 mg, 3.61 mmol) were dissolved in distilled water and tetrahydrofuran, and then iron (201 mg, 3.61 mmol) was used in the reaction mixture to obtain 37 mg of the title compound (32% yield).

$^1$H NMR (400 MHz, DMSO-d6) δ 6.96 (d, J = 8.3 Hz, 1H), 6.57 (d, J = 2.0 Hz, 1H), 6.39 (dd, J = 8.3, 2.1 Hz, 1H), 4.54 (s, 2H), 3.99 (s, 2H), 3.82 (d, J = 10.5 Hz, 2H), 3.63 - 3.55 (m, 2H), 3.52 (s, 3H), 2.03 - 1.81 (m, 4H).

Step 35-2 Preparation of *N*-(2-(3-oxa-8-azabicyclo[3.2.1]octan-8-yl)-1-methyl-1H-benzo[*d*]imidazol-5-yl)carbonohydrazonoyl dicyanide **(Compound 35)**

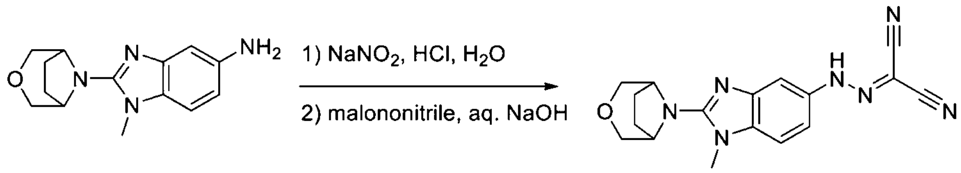


35-1 35

In the same manner as step 1-2, 2-(3-oxa-8-azabicyclo[3.2.1]octan-8-yl)-1-methyl-1H-benzo[d]imidazol-5-amine (20 mg, 0.08 mmol), sodium nitrite (8 mg, 0.12 mmol), and 1.0 M hydrochloric acid aqueous solution (0.2 mL, 0.23 mmol) were dissolved in distilled water, and then malononitrile (10 mg, 0.15 mmol) and an aqueous sodium hydroxide solution were used to obtain 15 mg of the title compound (57% yield).

$^1$H NMR (400 MHz, DMSO-d6) δ 13.04 (s, 1H), 7.47 - 7.37 (m, 2H), 7.31 - 7.24 (m, 1H), 4.21 (s, 2H), 3.85 (d, J = 10.8 Hz, 2H), 3.67 (s, 3H), 3.62 (d, J = 10.8 Hz, 2H), 2.06 - 1.90 (m, 4H).

**Example 36: Preparation of N-(2-(3-oxa-8-azabicyclo[3.2.1]octan-8-yl)benzo[d]thiazol-6-yl)-N-methylcarbonohydrazonoyl dicyanide**

Step 36-1: Preparation of N-(2-(3-oxa-8-azabicyclo[3.2.1]octan-8-yl)benzo[d]thiazol-6-yl)-N-methylcarbonohydrazonoyl dicyanide

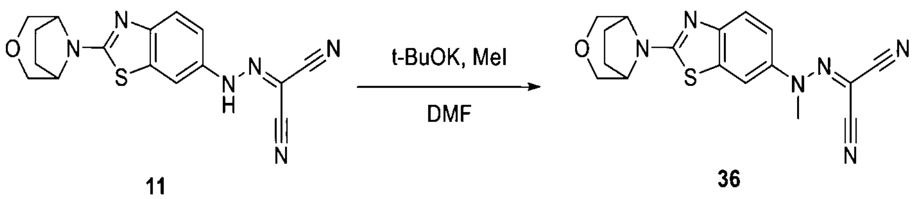


11 36

Under nitrogen, N-(2-(3-oxa-8-azabicyclo[3.2.1]octan-8-yl)benzo[d]thiazol-6-yl)carbonohydrazonoyl dicyanide (30 mg, 0.09 mmol) was dissolved in dimethylformamide solvent, and then potassium tert-butoxide (15 mg, 0.13 mmol) was added at room temperature, followed by iodomethane (28 μL, 0.44 mmol). The reaction mixture was stirred at 60°C for 4 hours. When the reaction was completed, the mixture was extracted with distilled water and ethyl acetate, and the organic layer was dried over anhydrous magnesium sulfate and then filtered. The filtrate was concentrated under reduced pressure, and the concentrate was purified by column chromatography to obtain 8 mg of the title compound (26% yield).

$^1$H NMR (400 MHz, DMSO-d6) δ 8.00 (d, J = 2.4 Hz, 1H), 7.52 (d, J = 8.7 Hz, 1H), 7.42 (dd, J = 8.8, 2.4 Hz, 1H), 4.31 (s, 2H), 4.08 (s, 3H), 3.74 (d, J = 11.0 Hz, 2H), 3.62 (d, J = 10.8 Hz, 2H), 2.01 (s, 4H).

**Example 37: Preparation of N-acetyl-N-(2-morpholinobenzo[d]thiazol-6-yl)carbonohydrazonoyl dicyanide**

Step 37-1: Preparation of N-acetyl-N-(2-morpholinobenzo[d]thiazol-6-yl)carbonohydrazonoyl dicyanide

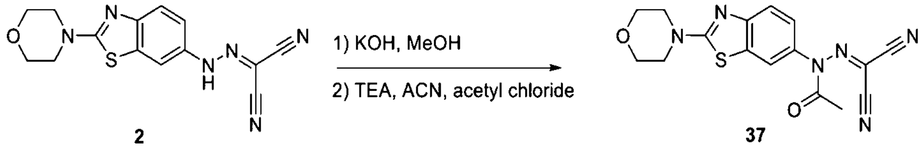


2 37

N-(2-morpholinobenzo[d]thiazol-6-yl)carbonohydrazonoyl dicyanide (150 mg, 0.48 mmol) and potassium hydroxide (30 mg, 0.53 mmol) were dissolved in methanol and stirred at room temperature for 3 hours. After the reaction was

completed, the solvent was concentrated under reduced pressure and solidified with ether. The produced solid (170 mg, 0.48 mmol) and triethylamine (34 μL, 0.24 mmol) were dissolved in acetonitrile solvent, and then acetyl chloride (86 μL, 1.21 mmol) was added, and the mixture was stirred at room temperature for 4 hours. After the reaction was completed, the mixture was extracted with distilled water and ethyl acetate, and the organic layer was dried over anhydrous magnesium sulfate and then filtered. The filtrate was concentrated under reduced pressure, solidified with ether, and filtered. The filtrate was concentrated again under reduced pressure, and then solidified with hexane to obtain 64 mg of the title compound (17% yield).

$^1$H NMR (400 MHz, Chloroform-d) δ 7.63 (d, J = 8.6 Hz, 1H), 7.42 (d, J = 2.1 Hz, 1H), 7.07 (dd, J = 8.5, 2.2 Hz, 1H), 3.84 (t, J = 4.9 Hz, 4H), 3.67 (t, J = 4.9 Hz, 4H), 2.60 (s, 3H).

**Example 38: Preparation of N-(2-(morpholine-4-carboxamido)benzo[d]thiazol-6-yl)carbonohydrazonoyl dicyanide**

Step 38-1: Preparation of phenyl(6-nitrobenzo[d]thiazol-2-yl)carbamate

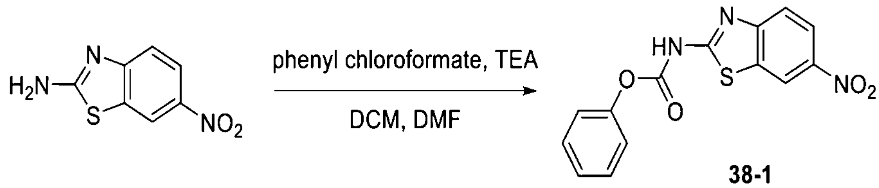


38-1

2-Amino-6-nitrobenzothiazole (500 mg, 2.56 mmol) and triethylamine (1.07 mL, 7.68 mmol) were dissolved in dichloromethane and dimethylformamide solvent, and then phenyl chloroformate (386 μL, 3.07 mmol) was added. The reaction mixture was stirred at room temperature for 6 hours. When the reaction was completed, the produced solid was filtered to obtain 802 mg of the title compound (99% yield).

$^1$H NMR (400 MHz, DMSO-d6) δ 13.07 (s, 1H), 9.07 (d, J = 2.4 Hz, 1H), 8.29 (dd, J = 9.1, 2.6 Hz, 1H), 7.90 (d, J = 8.9 Hz, 1H), 7.49 (t, J = 7.9 Hz, 2H), 7.35 - 7.28 (m, 3H).

Step 38-2: Preparation of N-(6-nitrobenzo[d]thiazol-2-yl)morpholine-4-carboxamide

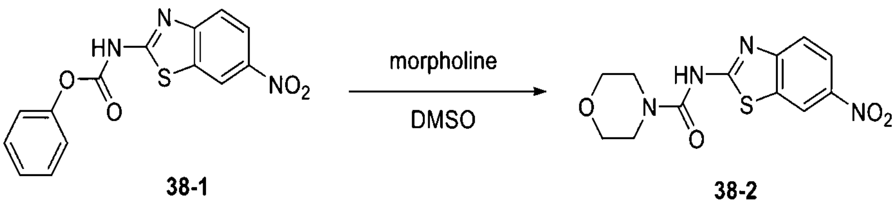


38-1 38-2

Phenyl(6-nitrobenzo[d]thiazol-2-yl)carbamate (200 mg, 0.63 mmol) was dissolved in dimethyl sulfoxide solvent, and then morpholine (277 μL, 3.17 mmol) was added. The reaction mixture was stirred at 50°C for 2 hours. When the reaction was completed, the mixture was extracted with an aqueous sodium bicarbonate solution and ethyl acetate, and the organic layer was dried over anhydrous magnesium sulfate and then filtered. The filtrate was concentrated under reduced pressure, and the concentrate was purified by column chromatography to obtain 66 mg of the title compound (34% yield).

$^1$H NMR (400 MHz, DMSO-d6) δ 12.04 (s, 1H), 8.91 (d, J = 2.4 Hz, 1H), 8.23 (dd, J = 8.9, 2.5 Hz, 1H), 7.69 (d, J = 8.9 Hz, 1H), 3.67 - 3.52 (m, 8H).

Step 38-3: Preparation of N-(6-aminobenzo[d]thiazol-2-yl)morpholine-4-carboxamide

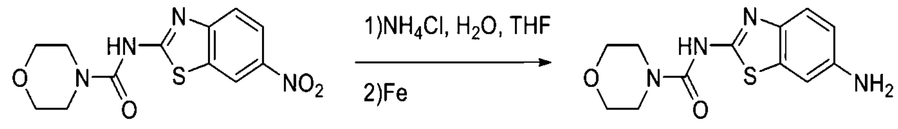


38-2 38-3

In the same manner as step 5-3, N-(6-nitrobenzo[d]thiazol-2-yl)morpholine-4-carboxamide (50 mg, 0.16 mmol) and ammonium chloride (69 mg, 1.30 mmol) were dissolved in distilled water and tetrahydrofuran, and then iron (72 mg, 1.30 mmol) was used in the reaction mixture to obtain 34 mg of the title compound (74% yield).

$^1$H NMR (400 MHz, DMSO-d6) δ 10.91 (s, 1H), 7.22 (s, 1H), 6.91 (s, 1H), 6.63 (dd, J = 8.5, 2.2 Hz, 1H), 5.03 (s, 2H), 3.63 - 3.45 (m, 8H).

Step 38-4: Preparation of *N*-(2-(morpholine-4-carboxamido)benzo[d]thiazol-6-yl)carbonohydrazonoyl dicyanide **(Compound 38)**

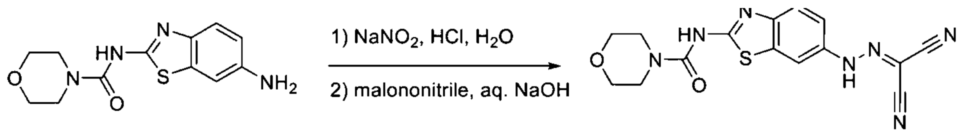


38-3 38

In the same manner as step 1-2, N-(6-aminobenzo[d]thiazol-2-yl)morpholine-4-carboxamide (30 mg, 0.11 mmol), sodium nitrite (11 mg, 0.16 mmol), and 1.0 M hydrochloric acid aqueous solution (0.3 mL, 0.32 mmol) were dissolved in distilled water, and then malononitrile (14 mg, 0.22 mmol) and an aqueous sodium hydroxide solution were used to obtain 35 mg of the title compound (92% yield).

$^1$H NMR (400 MHz, Acetone-d6) δ 12.00 (s, 1H), 7.98 (d, J = 2.2 Hz, 1H), 7.62 (d, J = 8.8 Hz, 1H), 7.55 (dd, J = 8.8, 2.2 Hz, 1H), 3.73 - 3.62 (m, 8H).

**Example 39: Preparation of N-(2-(2,6-dimethylmorpholine-4-carboxamido)benzo[d]thiazol-6-yl)carbonohydrazonoyl dicyanide**

Step 39-1: Preparation of 2,6-dimethyl-N-(6-nitrobenzo[d]thiazol-2-yl)morpholine-4-carboxamide

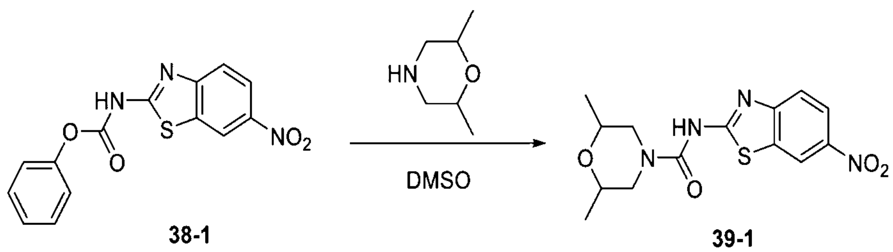


38-1 39-1

In the same manner as step 38-2, phenyl(6-nitrobenzo[d]thiazol-2-yl)carbamate (200 mg, 0.63 mmol) was dissolved in dimethyl sulfoxide solvent, and then 2,6-dimethylmorpholine (391 μL, 3.17 mmol) was used to obtain 75 mg of the title compound (35% yield).

$^1$H NMR (600 MHz, Chloroform-d) δ 9.08 (s, 1H), 8.69 (d, J = 2.4 Hz, 1H), 8.31 - 8.23 (m, 1H), 7.67 (d, J = 8.9 Hz, 1H), 3.93 (s, 2H), 3.63 (dt, J = 6.9, 3.8 Hz, 2H), 2.72 (t, J = 11.9 Hz, 2H), 1.21 (d, J = 6.2 Hz, 6H).

Step 39-2: Preparation of N-(6-aminobenzo[d]thiazol-2-yl)-2,6-dimethylmorpholine-4-carboxamide

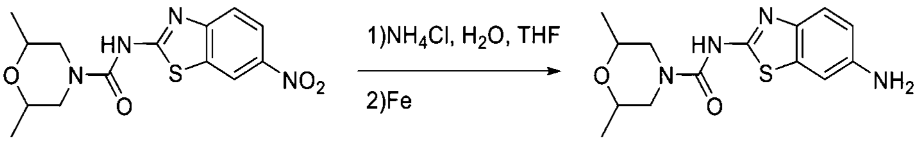


39-1 39-2

In the same manner as step 5-3, 2,6-dimethyl-N-(6-nitrobenzo[d]thiazol-2-yl)morpholine-4-carboxamide (50 mg, 0.15 mmol) and ammonium chloride (64 mg, 1.19 mmol) were dissolved in distilled water and tetrahydrofuran, and then iron (66 mg, 1.19 mmol) was used in the reaction mixture to obtain 44 mg of the title compound (97% yield).

$^1H$ NMR (600 MHz, DMSO-d6) δ 7.19 (s, 1H), 6.86 (d, J = 20.8 Hz, 1H), 6.60 (dd, J = 8.6, 2.2 Hz, 1H), 5.01 (s, 2H), 4.12 (s, 2H), 3.51 - 3.40 (m, 2H), 2.48 (s, 3H), 1.07 (d, J = 6.2 Hz, 6H).

Step 39-3: Preparation of *N*-(2-(2,6-dimethylmorpholine-4-carboxamido)benzo[d]thiazol-6-yl)carbonohydrazonoyl dicyanide **(Compound 39)**

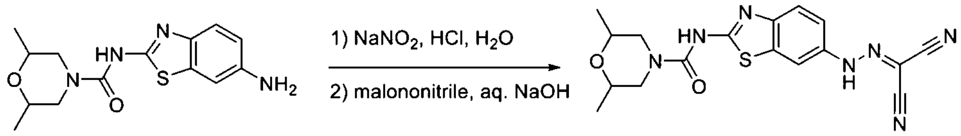


39-2 39

In the same manner as step 1-2, N-(6-aminobenzo[d]thiazol-2-yl)-2,6-dimethylmorpholine-4-carboxamide (40 mg, 0.13 mmol), sodium nitrite (13 mg, 0.20 mmol), and 1.0 M hydrochloric acid aqueous solution (0.4 mL, 0.39 mmol) were dissolved in distilled water, and then malononitrile (17 mg, 0.26 mmol) and an aqueous sodium hydroxide solution were used to obtain 18 mg of the title compound (35% yield).

$^1H$ NMR (400 MHz, Acetone-d6) δ 11.95 (s, 1H), 10.14 (s, 1H), 7.98 (d, J = 2.2 Hz, 1H), 7.62 (d, J = 8.8 Hz, 1H), 7.55 (dd, J = 8.7, 2.2 Hz, 1H), 4.23 (dd, J = 13.4, 1.9 Hz, 2H), 3.62 (dqd, J = 14.9, 6.1, 2.3 Hz, 2H), 2.70 - 2.56 (m, 2H), 1.17 (d, J = 6.2 Hz, 6H).

**Example 40: Preparation of N-(2-(8-oxa-3-azabicyclo[3.2.1]octane-3-carboxamido)benzo[d]thiazol-6-yl)carbonohydrazonoyl dicyanide**

Step 40-1: Preparation of N-(6-nitrobenzo[d]thiazol-2-yl)-8-oxa-3-azabicyclo[3.2.1]octane-3-carboxamide

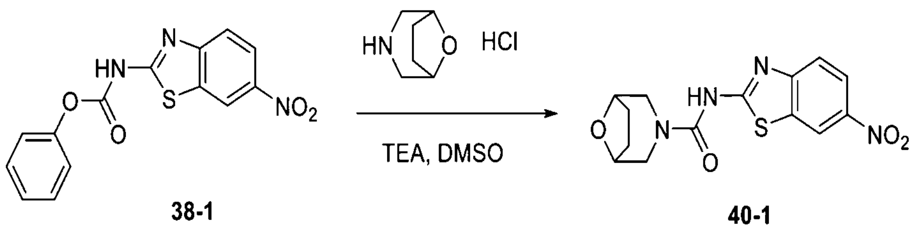


38-1 40-1

In the same manner as step 38-2, phenyl(6-nitrobenzo[d]thiazol-2-yl)carbamate (200 mg, 0.63 mmol) was dissolved in dimethyl sulfoxide solvent, and then 8-oxa-3-azabicyclo[3.2.1]octane hydrochloride (474 mg, 3.17 mmol) and triethylamine (442 μL, 3.17 mmol) were used to obtain 90 mg of the title compound (42% yield).

$^1H$ NMR (600 MHz, DMSO-d6) δ 11.65 (s, 1H), 8.89 (s, 1H), 8.21 (dd, J = 8.9, 2.4 Hz, 1H), 7.68 (s, 1H), 4.34 (d, J = 2.6 Hz, 2H), 3.85 (s, 2H), 3.08 (d, J = 11.7 Hz, 2H), 1.83 - 1.62 (m, 4H).

Step 40-2: Preparation of N-(6-nitrobenzo[d]thiazol-2-yl)-8-oxa-3-azabicyclo[3.2.1]octane-3-carboxamide

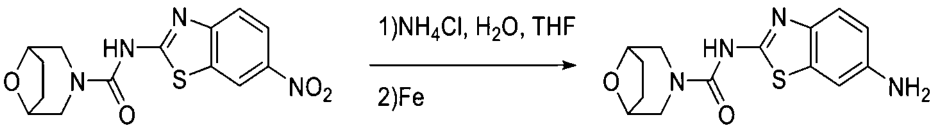


40-1 40-2

In the same manner as step 5-3, N-(6-nitrobenzo[d]thiazol-2-yl)-8-oxa-3-azabicyclo[3.2.1]octane-3-carboxamide (70 mg, 0.21 mmol) and ammonium chloride (90 mg, 1.67 mmol) were dissolved in distilled water and tetrahydrofuran, and then iron (93 mg, 1.67 mmol) was added to the reaction mixture, and after solvent removal, the product was used in the next step without purification.

Step 40-3: Preparation of *N*-(2-(8-oxa-3-azabicyclo[3.2.1]octane-3-carboxamido)benzo[d]thiazol-6-yl)carbonohydrazonoyl dicyanide **(Compound 40)**

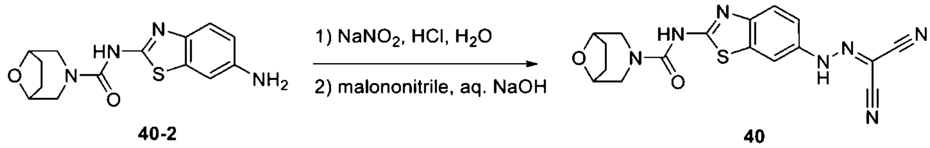


40-2 40

In the same manner as step 1-2, N-(6-nitrobenzo[d]thiazol-2-yl)-8-oxa-3-azabicyclo[3.2.1]octane-3-carboxamide (50 mg, 0.16 mmol), sodium nitrite (17 mg, 0.25 mmol), and 1.0 M hydrochloric acid aqueous solution (0.5 mL, 0.49 mmol) were dissolved in distilled water, and then malononitrile (22 mg, 0.33 mmol) and an aqueous sodium hydroxide solution were used to obtain 43 mg of the title compound (69% yield).

$^1H$ NMR (600 MHz, DMSO-d6) δ 13.12 (s, 1H), 11.18 (s, 1H), 7.96 (s, 1H), 7.63 (s, 1H), 7.49 (s, 1H), 4.33 (s, 2H), 3.81 (s, 2H), 3.06 (s, 2H), 1.84 - 1.73 (m, 3H).

**Example 41: Preparation of (R)-N-(2-(3-fluoropyrrolidin-1-yl)benzo[d]oxazol-6-yl)carbonohydrazonoyl dicyanide (Compound 41)**

Step 41-1: Preparation of *(R)*-2-(3-fluoropyrrolidin-1-yl)-6-nitrobenzo[d]oxazole **(Compound 41-1)**

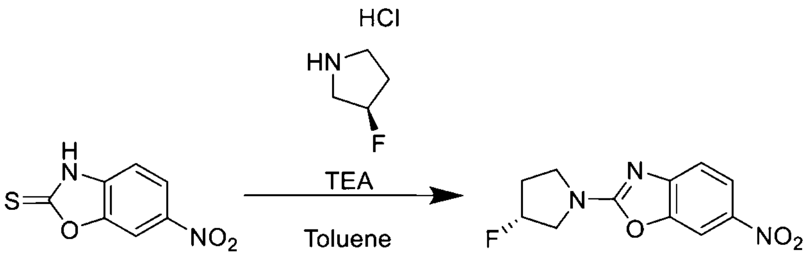


4-1 41-1

In the same manner as step 3-2, 6-nitrobenzo[*d*]oxazole-2(3*H*)-thione (300 mg, 1.53 mmol), *(R)*-(-)-3-fluoropyrrolidine hydrochloride (576 mg, 4.59 mmol), triethylamine (1.07 mL, 7.64 mmol), and toluene were added and reacted at 180°C for 30 minutes under microwave irradiation to obtain 253 mg of the title compound (66% yield).

$^1H$ NMR (400 MHz, DMSO-$d_6$) δ 8.32 (d, J = 2.2 Hz, 1H), 8.16 (dd, J = 8.7, 2.2 Hz, 1H), 7.40 (d, J = 8.7 Hz, 1H), 5.50 (d, J = 53 Hz, 1H), 3.93 - 3.80 (m, 3H), 3.74 - 3.67 (m, 1H), 2.38 - 2.16 (m, 2H).

Step 41-2: Preparation of *(R)*-2-(3-fluoropyrrolidin-1-yl)benzo[d]oxazol-6-amine **(Compound 41-2)**

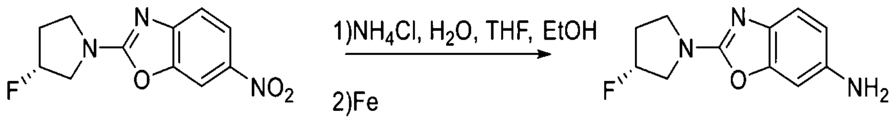


41-1 41-2

In the same manner as step 5-3, *(R)*-2-(3-fluoropyrrolidin-1-yl)-6-nitrobenzo[d]oxazole (200 mg, 0.79 mmol) and ammonium chloride (213 mg, 3.98 mmol) were dissolved in distilled water, tetrahydrofuran, and ethanol, and then iron (222 mg, 3.98 mmol) was used in the reaction mixture to obtain 114 mg of the title compound (65% yield).

$^1H$ NMR (400 MHz, DMSO-$d_6$) δ 6.96 (d, J = 8.3 Hz 1H), 6.65 (d, J = 2 Hz, 1H), 6.42 (dd, J = 8.3, 2 Hz, 1H), 5.43 (d, J = 53 Hz, 1H), 4.84 (s, 2H), 3.74 - 3.67 (m, 2H), 3.59 - 3.50 (m, 2H), 2.29 - 2.09 (m, 2H).

Step 41-3: Preparation of *(R)*-N-(2-(3-fluoropyrrolidin-1-yl)benzo[d]oxazol-6-yl)carbonohydrazonoyl dicyanide **(Compound 41)**

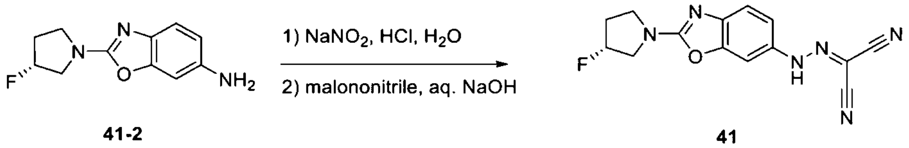


41-2 41

In the same manner as step 1-2, *(R)*-2-(3-fluoropyrrolidin-1-yl)benzo[d]oxazol-6-amine (100 mg, 0.45 mmol), sodium nitrite (77 mg, 0.90 mmol), and 1.0 M hydrochloric acid aqueous solution (1.36 mL, 1.36 mmol) were dissolved in distilled water, and then malononitrile (60 mg, 0.90 mmol) and an aqueous sodium hydroxide solution were used to obtain 80 mg of the title compound (59% yield).

$^1H$ NMR (600 MHz, DMSO-d6) δ 13.05 (s, 1H), 7.50 (d, J = 1.6 Hz 1H), 7.34 - 7.28 (m, 2H), 5.47 (d, J = 53 Hz, 1H), 3.85 - 3.73 (m, 2H), 3.66 - 3.59 (m, 2H), 2.31 - 2.13 (m, 2H).

**Example 42: Preparation of (S)-N-(2-(3-fluoropiperidin-1-yl)benzo[d]oxazol-6-yl)carbonohydrazonoyl dicyanide (Compound 42)**

Step 42-1: Preparation of *(S)*-2-(3-fluoropiperidin-1-yl)-6-nitrobenzo[d]oxazole **(Compound 42-1)**

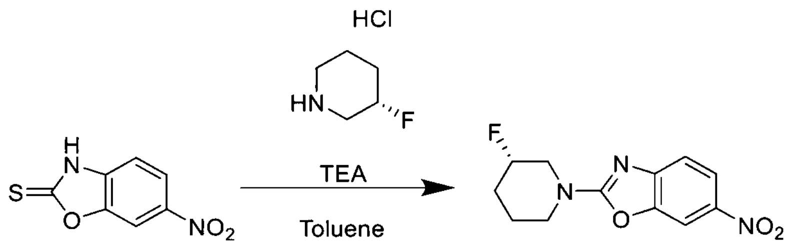


4-1 42-1

In the same manner as step 3-2, 6-nitrobenzo[*d*]oxazole-2(3*H*)-thione (300 mg, 1.53 mmol), *(S)*-3-fluoropiperidine hydrochloride (640 mg, 4.59 mmol), triethylamine (639 μL, 4.59 mmol), and toluene were added and reacted at 180°C for 30 minutes under microwave irradiation to obtain 238.9 mg of the title compound (59% yield).

$^1H$ NMR (400 MHz, DMSO-$d_6$) δ 8.30 (d, J = 2.2 Hz, 1H), 8.15 (dd, J = 8.7, 2.3 Hz, 1H), 7.40 (d, J = 8.7 Hz, 1H), 4.94 (d, J = 48.3 Hz, 1H), 4.18 - 4.11 (m, J = 1H), 4.03 (dt, J = 13.2, 4.28 Hz, 1H), 3.69 (qd, J = 14.2, 2.0 Hz, 1H), 3.47 - 3.41 (m, 1H), 1.99 - 1.79 (m, 3H), 1.70 - 1.63 (m, 1H).

Step 42-2: Preparation of *(S)*-2-(3-fluoropyrrolphedin-1-yl)benzo[d]oxazol-6-amine **(Compound 42-2)**

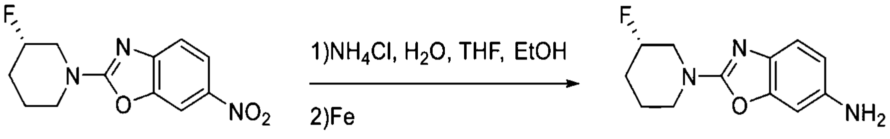


**42-1** **42-2**

In the same manner as step 5-3, *(S)*-2-(3-fluoropyrrolphedin-1-yl)-6-nitrobenzo[d]oxazole (200 mg, 0.79 mmol) and ammonium chloride (323 mg, 6.03 mmol) were dissolved in distilled water, tetrahydrofuran, and ethanol, and then iron (337 mg, 6.03 mmol) was used in the reaction mixture to obtain 153 mg of the title compound (87% yield).

$^1$H NMR (400 MHz, DMSO-$d_6$) δ 6.94 (d, J = 8.3 Hz 1H), 6.61 (d, J = 2.0 Hz, 1H), 6.40 (dd, J = 8.2, 2.1 Hz, 1H), 4.88 - 4.76 (m, 3H), 3.89 - 3.82 (m, 1H), 3.75 (dt, J = 12.9, 4.5 1H), 3.55 (qd, J = 13.8, 2.4, 1H), 1.90 - 1.79 (m, 3H), 1.58 - 1.53 (m, 1H) .

Step 42-3: Preparation of *(S)*-*N*-(2-(3-fluoropiperidin-1-yl)benzo[d]oxazol-6-yl)carbonohydrazonoyl dicyanide **(Compound 42)**

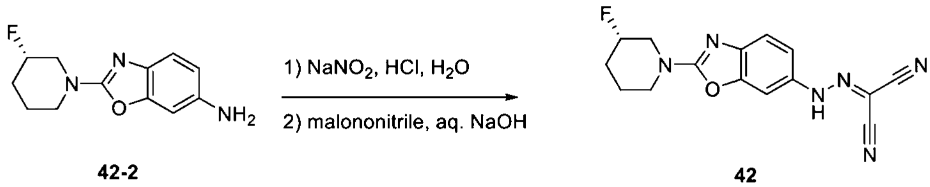


**42-2** **42**

In the same manner as step 1-2, *(S)*-2-(3-fluoropiperidin-1-yl)benzo[d]oxazol-6-amine (120 mg, 0.51 mmol), sodium nitrite (52.8 mg, 0.77 mmol), and 1.0 M hydrochloric acid aqueous solution (3.1 mL, 3.06 mmol) were dissolved in distilled water, and then malononitrile (67.4 mg, 1.02 mmol) and an aqueous sodium hydroxide solution were used to obtain 81.1 mg of the title compound (51% yield).

$^1$H NMR (600 MHz, DMSO-d6) δ 13.08 (s, 1H), 7.50 (d, J = 1.8 Hz 1H), 7.34 - 7.27 (m, 2H), 4.89 (d, J = 47 Hz, 1H), 4.06 - 3.99 (m, 1H), 3.91 (dt, J = 13.2, 4.08, 1H), 3.62 (qd, J = 14, 2.0, 1H), 1.94 - 1.78 (m, 3H), 1.63 - 1.58 (m, 1H).

**Example 43: Preparation of (S)-N-(2-(3-fluoropyrrolidin-1-yl)benzo[d]oxazol-6-yl)carbonohydrazonoyl dicyanide (Compound 43)**

Step 41-1: Preparation of *(S)*-2-(3-fluoropyrrolidin-1-yl)-6-nitrobenzo[d]oxazole **(Compound 43-1)**

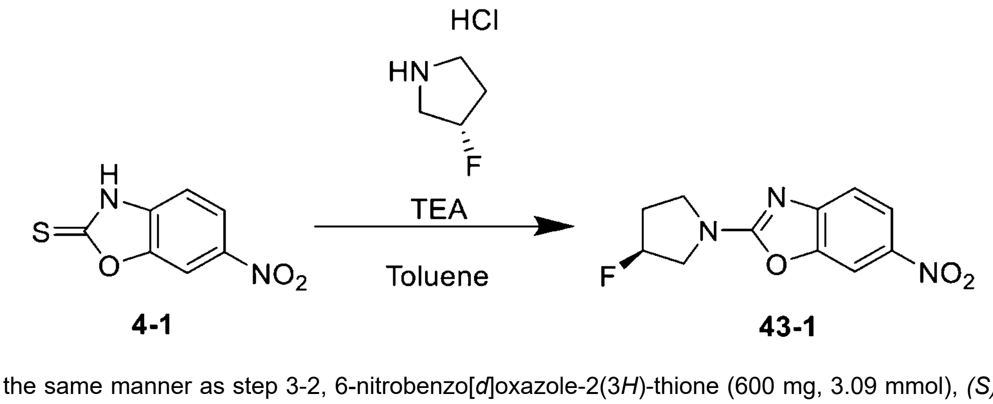


**4-1** **43-1**

In the same manner as step 3-2, 6-nitrobenzo[*d*]oxazole-2(3*H*)-thione (600 mg, 3.09 mmol), *(S)*-(-)-3-fluoropyrrolidine hydrochloride (583 mg, 4.64 mmol), triethylamine (1.26 mL, 9.28 mmol), and toluene were added and reacted at 180°C for 30 minutes under microwave irradiation to obtain 300 mg of the title compound (38.7% yield).

$^1$H NMR (400 MHz, DMSO-$d_6$) δ 8.33 (d, J = 2.2 Hz, 1H), 8.17 (dd, J = 8.7, 2.3 Hz, 1H), 7.43 (d, J = 8.7 Hz, 1H), 5.51 (d, J = 53 Hz, 1H), 3.93 - 3.81 (m, 3H), 3.74 - 3.67 (m, 1H), 2.36 - 2.17 (m, 2H).

Step 43-2: Preparation of *(S)*-2-(3-fluoropyrrolidin-1-yl)benzo[d]oxazol-6-amine **(Compound 43-2)**

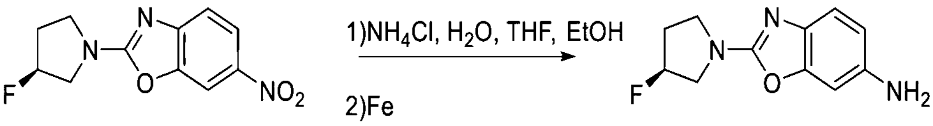


**43-1** **43-2**

In the same manner as step 5-3, *(S)*-2-(3-fluoropyrrolidin-1-yl)-6-nitrobenzo[d]oxazole (283 mg, 1.12 mmol) and ammonium chloride (301 mg, 5.63 mmol) were dissolved in distilled water, tetrahydrofuran, and ethanol, and then iron (315 mg, 5.63 mmol) was used in the reaction mixture to obtain 110 mg of the title compound (44% yield).

$^1$H NMR (400 MHz, DMSO-$d_6$) δ 6.96 (d, J = 8.2 Hz 1H), 6.66 (d, J = 1.96 Hz, 1H), 6.42 (dd, J = 8.2, 2.0 Hz, 1H), 5.44 (d, J = 53 Hz, 1H), 4.85 (s, 2H), 3.78 - 3.68 (m, 2H), 3.59 - 3.55 (m, 1H), 2.29 - 2.09 (m, 2H).

Step 43-3: Preparation of *(S)*-*N*-(2-(3-fluoropyrrolidin-1-yl)benzo[d]oxazol-6-yl)carbonohydrazonoyl dicyanide **(Compound 43)**

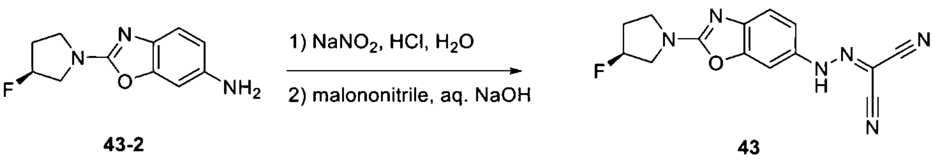


**43-2** **43**

In the same manner as step 1-2, *(S)*-2-(3-fluoropyrrolidin-1-yl)benzo[d]oxazol-6-amine (105 mg, 0.47 mmol), sodium nitrite (81 mg, 0.95 mmol), and 1.0 M hydrochloric acid aqueous solution (1.42 mL, 1.42 mmol) were dissolved in distilled water, and then malononitrile (63 mg, 0.95 mmol) and an aqueous sodium hydroxide solution were used to obtain 58 mg of the title compound (40.8% yield).

$^1$H NMR (600 MHz, DMSO-d6) δ 13.08 (s, 1H), 7.53 (d, J = 1.7 Hz, 1H), 7.36 - 7.30 (m, 2H), 5.49 (d, J = 53 Hz, 1H), 3.87 - 3.74 (m, 3H), 3.68 - 3.61 (m, 1H), 2.33 - 2.14 (m, 2H).

**Example 44: Preparation of N-(2-(3-fluoropiperidin-1-yl)benzo[d]oxazol-6-yl)carbonohydrazonoyl dicyanide (Compound 44)**

Step 44-1: Preparation of 2-(3-fluoropiperidin-1-yl)-6-nitrobenzo[d]oxazole **(Compound 44-1)**

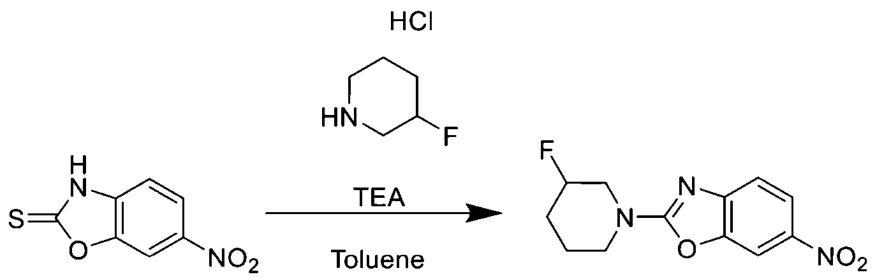


**4-1** **44-1**

In the same manner as step 3-2, 6-nitrobenzo[*d*]oxazole-2(3*H*)-thione (500 mg, 2.55 mmol), 3-fluoropiperidine hydrochloride (712 mg, 5.10 mmol), triethylamine (1.4 mL, 10.2 mmol), and toluene were added and reacted at 180°C for 30 minutes under microwave irradiation to obtain 363 mg of the title compound (54% yield).

$^1$H NMR (400 MHz, DMSO-$d_6$) δ 8.30 (S, 1H), 8.16 (d, J = 8.7 Hz, 1H), 7.40 (d, J = 8.7 Hz, 1H), 4.95 (d, J = 46.8 Hz, 1H), 4.16 (t, J = 11.92, 1H), 4.04 (d, J = 13 Hz, 1H), 3.69 (dd, J = 32.9, 13.9 Hz, 1H), 3.47 - 3.41 (m, 1H), 1.96 - 1.67 (m, 4H).

Step 44-2: Preparation of 2-(3-fluoropyrrolphedin-1-yl)benzo[d]oxazol-6-amine **(Compound 44-2)**

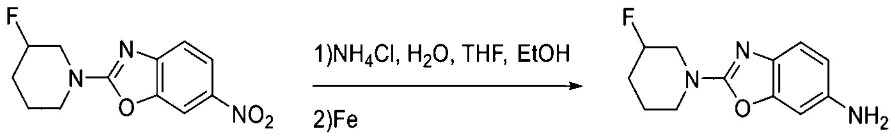


44-1 44-2

In the same manner as step 5-3, 2-(3-fluoropyrrolphedin-1-yl)-6-nitrobenzo[d]oxazole (300 mg, 1.13 mmol) and ammonium chloride (484 mg, 9.05 mmol) were dissolved in distilled water, tetrahydrofuran, and ethanol, and then iron (505 mg, 9.05 mmol) was used in the reaction mixture to obtain 231 mg of the title compound (87% yield).

$^1$H NMR (400 MHz, DMSO-$d_6$) δ 6.95 (d, J = 8.2 Hz 1H), 6.62 (d, J = 2.0 Hz, 1H), 6.41 (dd, J = 8.3, 2.1 Hz, 1H), 4.89 - 4.76 (m, 3H), 3.90 - 3.83 (m, 1H), 3.78 - 3.73 (m, 1H), 3.56 (qd, J = 13.8, 2.4, 1H), 1.91 - 1.79 (m, 3H), 1.59 - 1.54 (m, 1H).

Step 44-3: Preparation of *N*-(2-(3-fluoropiperidin-1-yl)benzo[d]oxazol-6-yl)carbonohydrazonoyl dicyanide **(Compound 44)**

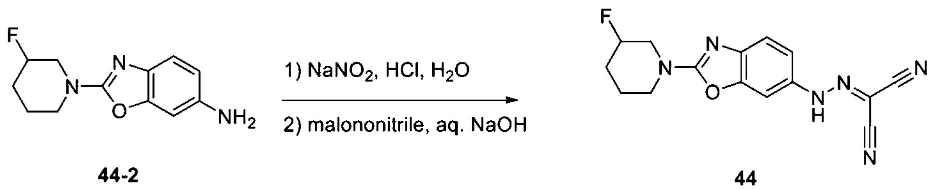


44-2 44

In the same manner as step 1-2, 2-(3-fluoropiperidin-1-yl)benzo[d]oxazol-6-amine (200 mg, 0.85 mmol), sodium nitrite (88 mg, 1.28 mmol), and 1.0 M hydrochloric acid aqueous solution (5.1 mL, 5.1 mmol) were dissolved in distilled water, and then malononitrile (112 mg, 1.70 mmol) and an aqueous sodium hydroxide solution were used to obtain 111 mg of the title compound (42% yield).

$^1$H NMR (600 MHz, DMSO-$d_6$) δ 13.06 (s, 1H), 7.50 (d, J = 1.8 Hz 1H), 7.34 - 7.27 (m, 2H), 4.89 (d, J = 47 Hz, 1H), 4.06 - 3.99 (m, 1H), 3.93 - 3.89 (m, 1H), 3.62 (qd, J = 14, 2.0, 1H), 1.94 - 1.81 (m, 3H), 1.63 - 1.58 (m, 1H).

**Example 45: Preparation of N-(2-(pyrrolidin-1-yl)benzo[d]oxazol-6-yl)carbonohydrazonoyl dicyanide (Compound 45)**

Step 45-1: Preparation of 2-(pyrrolidin-1-yl)-6-nitrobenzo[d]oxazole **(Compound 45-1)**

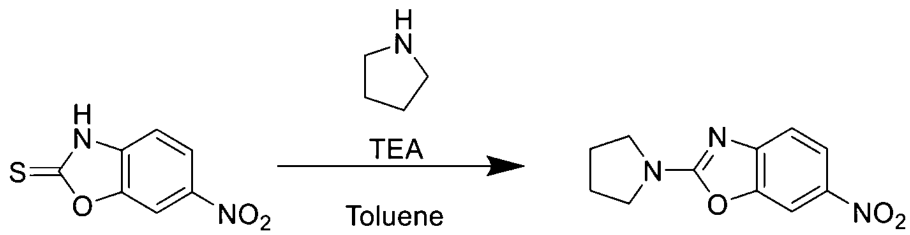


4-1 45-1

In the same manner as step 3-2, 6-nitrobenzo[d]oxazole-2(3*H*)-thione (300 mg, 1.53 mmol), pyrrolidine (255 μL, 3.06 mmol), triethylamine (852 μL, 6.12 mmol), and toluene were added and refluxed at 120°C for 2 hours to obtain 119 mg of the title compound (33% yield).

$^1$H NMR (400 MHz, DMSO-$d_6$) δ 8.28 (S, 1H), 8.14 (dd, J = 8.7 Hz, 2.28, 1H), 7.37 (d, J = 8.7 Hz, 1H), 3.62 (t, J = 6.64 Hz, 4H), 2.01 - 1.98 (m, 4H).

Step 45-2: Preparation of 2-(pyrrolidin-1-yl)benzo[d]oxazol-6-amine **(Compound 45-2)**

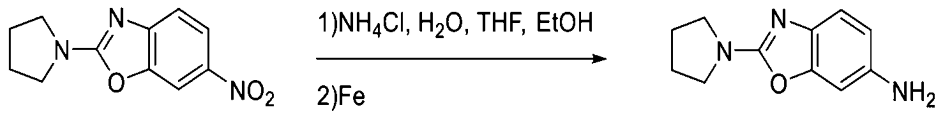


45-1 45-2

In the same manner as step 5-3, 2-(pyrrolidin-1-yl)-6-nitrobenzo[d]oxazole (100 mg, 0.43 mmol) and ammonium chloride (183 mg, 3.43 mmol) were dissolved in distilled water, tetrahydrofuran, and ethanol, and then iron (192 mg, 3.43 mmol) was used in the reaction mixture to obtain 72 mg of the title compound (82% yield).

$^1H$ NMR (400 MHz, $CDCl_3$-*$d_1$*) δ 7.44 (d, J = 8.2 Hz 1H), 6.69 (d, J = 2.1 Hz, 1H), 6.54 (dd, J = 8.2, 2.1 Hz, 1H), 3.63 - 3.60 (m, 6H), 2.04 - 2.00 (m, 4H).

Step 45-3: Preparation of *N*-(2-(pyrrolidin-1-yl)benzo[d]oxazol-6-yl)carbonohydrazonoyl dicyanide **(Compound 45)**

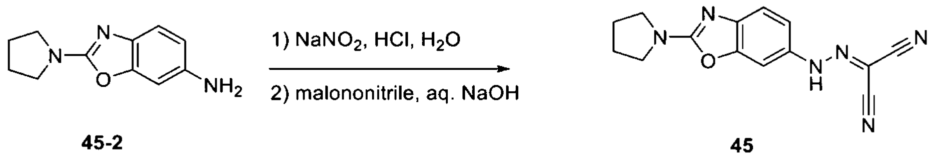


45-2 45

In the same manner as step 1-2, 2-(pyrrolidin-1-yl)benzo[d]oxazol-6-amine (60 mg, 0.30 mmol), sodium nitrite (31 mg, 0.44 mmol), and 1.0 M hydrochloric acid aqueous solution (1.8 mL, 1.77 mmol) were dissolved in distilled water, and then malononitrile (39 mg, 0.59 mmol) and an aqueous sodium hydroxide solution were used to obtain 56 mg of the title compound (67% yield).

$^1H$ NMR (600 MHz, DMSO-*$d_6$*) δ 13.09 (s, 1H), 7.50 (d, J = 1.8 Hz 1H), 7.34 - 7.26 (m, 2H), 3.58 - 3.55 (m, 4H), 2.00 - 1.96 (m, 4H).

**Example 46: Preparation of N-(2-(piperidin-1-yl)benzo[d]oxazol-6-yl)carbonohydrazonoyl dicyanide (Compound 46)**

Step 46-1: Preparation of 2-(piperidin-1-yl)-6-nitrobenzo[d]oxazole **(Compound 46-1)**

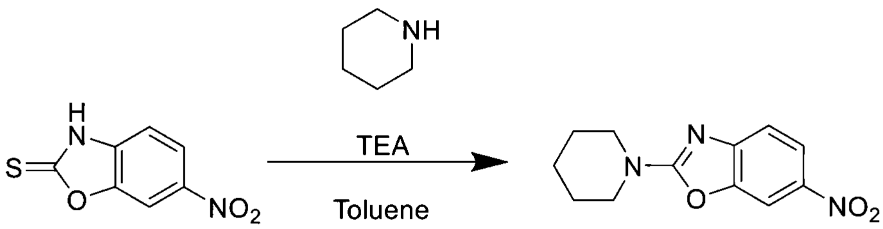


4-1 46-1

In the same manner as step 3-2, 6-nitrobenzo[*d*]oxazole-2(3*H*)-thione (300 mg, 1.53 mmol), piperidine (302 μL, 3.06 mmol), triethylamine (852 μL, 6.12 mmol), and toluene were added and refluxed at 170°C for 19 hours to obtain 233 mg of the title compound, which was used in the next reaction without separation.

Step 46-2: Preparation of 2-(piperidin-1-yl)benzo[d]oxazol-6-amine **(Compound 46-2)**

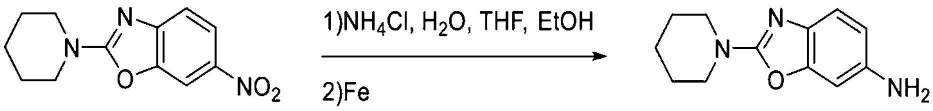


46-1 46-2

In the same manner as step 5-3, 2-(piperidin-1-yl)-6-nitrobenzo[d]oxazole (200 mg, 0.81 mmol) and ammonium chloride (346 mg, 6.47 mmol) were dissolved in distilled water, tetrahydrofuran, and ethanol, and then iron (361 mg, 6.47 mmol) was used in the reaction mixture to obtain 126 mg of the title compound (72% yield).

$^1$H NMR (400 MHz, DMSO-*d*$_6$) δ 6.94 (d, J = 8.2 Hz 1H), 6.62 (d, J = 2.0 Hz, 1H), 6.41 (dd, J = 8.2, 2.1 Hz, 1H), 4.85 (s, 2H), 3.50 - 3.49 (m, 4H), 1.66 - 1.55 (m, 6H).

Step 46-3: Preparation of *N*-(2-(piperidin-1-yl)benzo[d]oxazol-6-yl)carbonohydrazonoyl dicyanide **(Compound 46)**

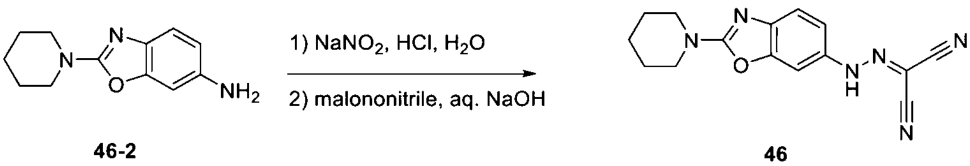


46-2 46

In the same manner as step 1-2, 2-(piperidin-1-yl)benzo[d]oxazol-6-amine (110 mg, 0.51 mmol), sodium nitrite (52 mg, 0.76 mmol), and 1.0 M hydrochloric acid aqueous solution (3.04 mL, 3.04 mmol) were dissolved in distilled water, and then malononitrile (67 mg, 1.01 mmol) and an aqueous sodium hydroxide solution were used to obtain 56 mg of the title compound (37% yield).

$^1$H NMR (600 MHz, DMSO-*d*$_6$) δ 13.0 (s, 1H), 7.49 (d, J = 1.8 Hz 1H), 7.34 - 7.26 (m, 2H), 3.62 - 3.61 (m, 4H), 1.66 - 1.58 (m, 6H).

**Example 47: Preparation of (R)-N-(2-(3-fluoropiperidin-1-yl)benzo[d]oxazol-6-yl)carbonohydrazonoyl dicyanide (Compound 47)**

Step 47-1: Preparation of (R)-2-(3-fluoropiperidin-1-yl)-6-nitrobenzo[d]oxazole **(Compound 47-1)**

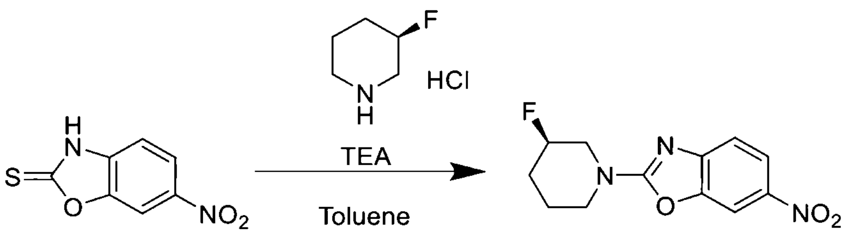


4-1 47-1

In the same manner as step 3-2, 6-nitrobenzo[*d*]oxazole-2(3*H*)-thione (500 mg, 2.55 mmol), (R)-3-fluoropiperidine hydrochloride (712 mg, 5.10 mmol), triethylamine (1.4 mL, 10.2 mmol), and toluene were added and reacted at 160°C for 30 minutes under microwave irradiation to obtain 402.7 mg of the title compound (61.6% yield).

$^1$H NMR (400 MHz, DMSO-*d*$_6$) δ 8.31 (d, J = 2.24 Hz 1H), 8.16 (dd, J = 8.68, 2.28 Hz, 1H), 7.41 (d, J = 8.72 Hz, 1H), 5.02 - 4.88 (m, 1H), 4.20 - 4.03 (m, 2H), 3.76 - 3.63 (m, 1H), 3.48 - 3.41 (m, 1H), 2.00 - 1.81 (m, 3H), 1.69 - 1.65 (m, 1H).

Step 47-2: Preparation of (R)-2-(3-fluoropiperidin-1-yl)benzo[d]oxazol-6-amine **(Compound 47-2)**

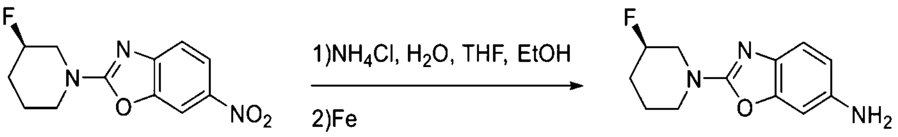


47-1 47-2

In the same manner as step 5-3, (R)-2-(3-fluoropiperidin-1-yl)-6-nitrobenzo[d]oxazole (350 mg, 1.32 mmol) and ammonium chloride (5+65 mg, 10.6 mmol) were dissolved in distilled water, tetrahydrofuran, and ethanol, and then iron (590 mg, 10.6 mmol) was used in the reaction mixture to obtain 254.9 mg of the title compound (82% yield).

$^1H$ NMR (400 MHz, DMSO-$d_6$) δ 6.95 (d, J = 8.2 Hz 1H), 6.62 (d, J = 1.9 Hz, 1H), 6.41 (dd, J = 8.5, 2.1 Hz, 1H), 4.90 - 4.77 (m, 3H), 3.91 - 3.74 (m, 2H), 3.62 - 3.50 (m, 1H), 1.91 - 1.79 (m, 3H), 1.60 - 1.55 (m, 1H).

Step 47-3: Preparation of (R)-*N*-(2-(3-fluoropiperidin-1-yl)benzo[d]oxazol-6-yl)carbonohydrazonoyl dicyanide **(Compound 47)**

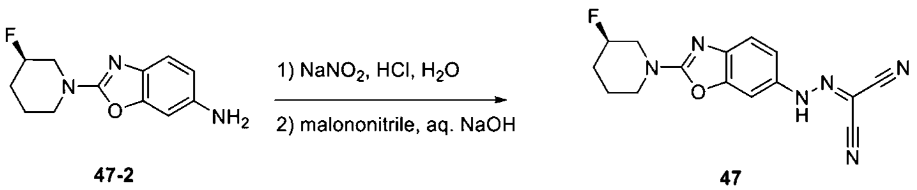


47-2 47

In the same manner as step 1-2, (R)-2-(3-fluoropiperidin-1-yl)benzo[d]oxazol-6-amine (200 mg, 0.85 mmol), sodium nitrite (90 mg, 1.3 mmol), and 1.0 M hydrochloric acid aqueous solution (5.10 mL, 5.10 mmol) were dissolved in distilled water, and then malononitrile (112 mg, 1.7 mmol) and an aqueous sodium hydroxide solution were used to obtain 173 mg of the title compound (65.2% yield).

$^1H$ NMR (400 MHz, DMSO-$d_6$) δ 13.1 (s, 1H), 7.51 (d, J = 1.8 Hz 1H), 7.35 - 7.28 (m, 2H), 4.96 - 4.84 (m, 1H), 4.07 - 3.91 (m, 2H), 3.69 - 3.57 (m, 1H), 3.42 - 3.39 (m, 1H), 1.95 - 1.80 (m, 3H), 1.65 - 1.61 (m, 1H).

**Example 48: Preparation of N-(2-(morpholine-4-carbonyl)-1H-pyrrolo[2,3-b]pyridin-5-yl)carbonohydrazonoyl dicyanide (Compound 48)**

Step 48-2: Preparation of morpholino(1-(phenylsulfonyl)-1*H*-pyrrolo[2,3-*b*]pyridin-2-yl)methanone **(Compound 48-2)**

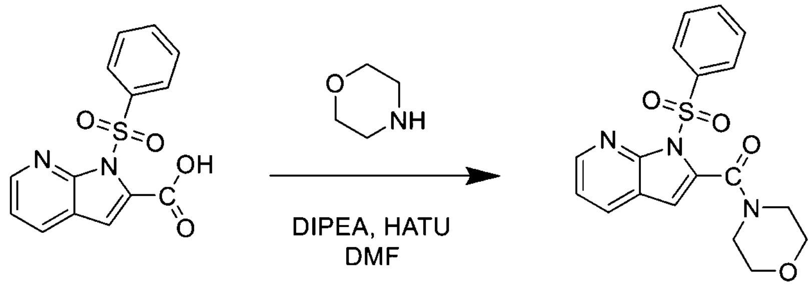


48-1 48-2

Under nitrogen, N,N-diisopropylamine (DIPEA, 3.50 mL, 19.8 mmol) and HATU (Hexafluorophosphate Azabenzotriazole Tetramethyl Uronium, 4.90 g, 12.9 mmol) were added to 1-(phenylsulfonyl)-1H-pyrrolo[2,3-b]pyridine-2-carboxylic acid (3 g, 9.90 mmol), and the mixture was stirred at room temperature for 20 minutes. Thereafter, morpholine (1.70 mL, 19.8 mmol) was added, and the mixture was stirred at room temperature for 5 hours to obtain 3.00 g of the title compound (83.11 % yield).

$^1H$ NMR (400 MHz, DMSO-$d_6$) δ 8.43 (d, J = 4.7 Hz, 1H), 8.23 (d, J = 8.0 Hz, 2H), 8.06 (d, J = 7.8 Hz, 1H), 7.73 (t, J = 7.3 Hz, 1H), 7.64 (t, J = 7.6 Hz, 2H), 7.34 (dd, J = 7.8 Hz, 4.8 Hz, 1H), 6.93 (s, 1H), 3.70 (s, 4H), 3.65 3.64 (m, 2H), 3.49 (m,

2H).

Step 48-3: Preparation of morpholino(5-nitro-1-(phenylsulfonyl)-1*H*-pyrrolo[2,3-*b*]pyridin-2-yl)methanone **(Compound 48-3)**

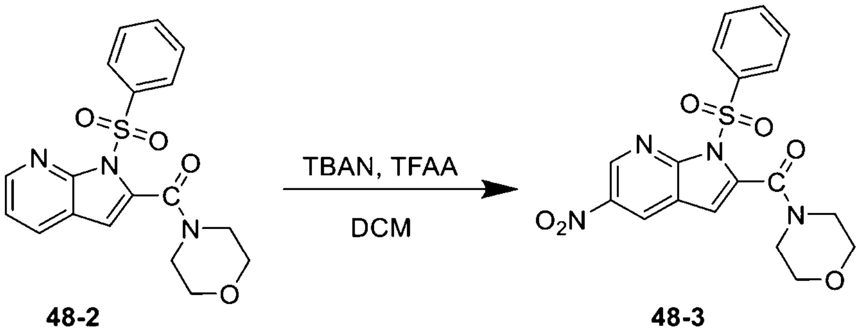


48-2 48-3

Under nitrogen, morpholino(1-phenylsulfonyl)-1*H*-pyrrolo[2,3-*b*]pyridin-2-yl)methanone (2 g, 5.40 mmol) and tetrabutylammonium nitrate (2.1 g, 7.0 mmol) were dissolved in dichloromethane and stirred at 0°C for 1.5 hours. Thereafter, anhydrous trifluoroacetic acid anhydride (TFAA, 978 μL, 7.0 mmol) was added, and the mixture was stirred at 0°C for 10 minutes, and then stirred at room temperature for an additional 18.5 hours. After the reaction was completed, the mixture was purified by column chromatography to obtain 1.6 g of the title compound (73% yield).

$^1H$ NMR (400 MHz, DMSO-$d_6$) δ 9.26 (d, J = 2.4 Hz, 1H), 8.97 (d, J = 2.5 Hz, 1H), 8.28 (d, J = 7.7 Hz, 2H), 7.80 (t, J = 7.4 Hz, 1H), 7.70 (t, J = 7.7 Hz, 2H), 7.12 (s, 1H), 3.72 (s, 4H), 3.67 3.66 (m, 2H), 3.53 (m, 2H).

Step 48-4: Preparation of morpholino(5-nitro-1*H*-pyrrolo[2,3-*b*]pyridin-2-yl)methanone **(Compound 48-4)**

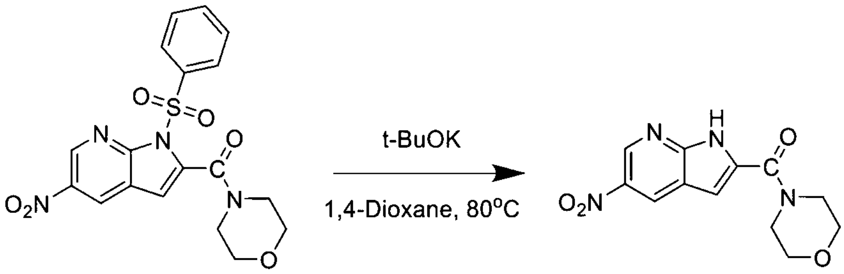


48-3 48-4

Under nitrogen, morpholino(5-nitro-1-(phenylsulfonyl)-1*H*-pyrrolo[2,3-*b*]pyridin-2-yl)methanone (1.4 g, 3.40 mmol) and solid potassium tert-butoxide (1.1 g, 10 mmol) were dissolved in dioxane and stirred at 80°C for 13 hours to obtain 325 mg of the title compound (35% yield).

$^1H$ NMR (400 MHz, DMSO-$d_6$) δ 13.0 (s, 1H), 9.18 (d, J = 2.2 Hz, 1H), 8.96 (d, J = 1.8 Hz, 1H), 7.03 (s, 1H), 3.68 (d, J = 11.9 Hz, 8H).

Step 48-5: Preparation of tert-butyl 2-(morpholine-4-carbonyl)-5-nitro-1*H*-pyrrolo[2,3-*b*]pyridine-1-carboxylate **(Compound 48-5)**

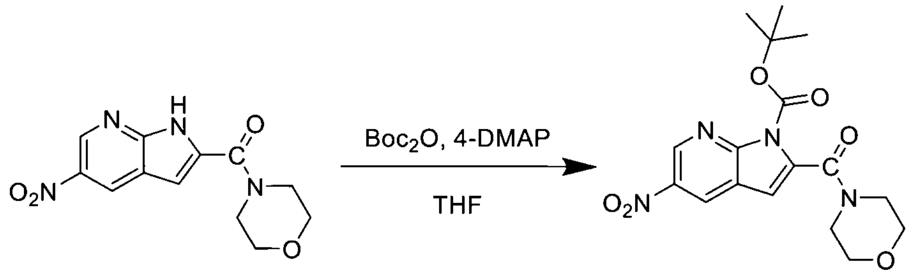


**48-4** **48-5**

Under nitrogen, morpholino(5-nitro-1H-pyrrolo[2,3-b]pyridin-2-yl)methanone (300 mg, 1.09 mmol), di-tert-butyl dicarbonate (568 mg, 2.60 mmol), and 4-dimethylaminopyridine (26 mg, 0.2 mmol) were dissolved in tetrahydrofuran solution and stirred at room temperature for 22.5 hours. The mixture was purified by column chromatography to obtain 186.7 mg of the title compound (46% yield).

$^1$H NMR (400 MHz, DMSO-$d_6$) δ 9.28 (d, J = 2.4 Hz, 1H), 8.99 (d, J = 2.4 Hz, 1H), 7.08 (s, 1H), 3.70-3.52 (m, 8H), 1.56 (s, 9H).

Step 48-6: Preparation of tert-butyl 5-amino-2-(morpholine-4-carbonyl)-1H-pyrrolo[2,3-b]pyridine-1-carboxylate (Compound 48-6)

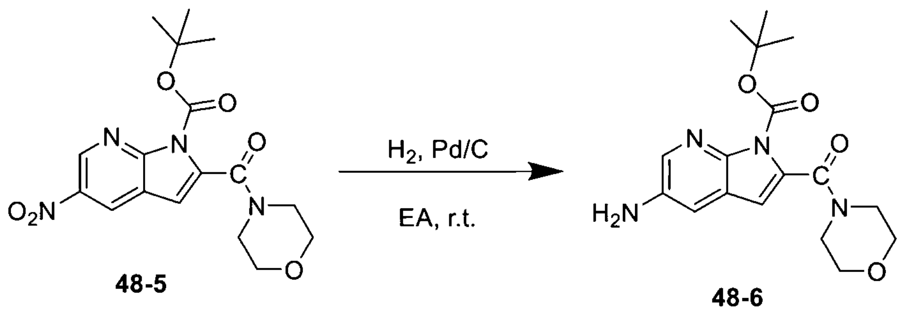


**48-5** **48-6**

Under nitrogen, tert-butyl 2-(morpholine-4-carbonyl)-5-nitro-1H-pyrrolo[2,3-b]pyridine-1-carboxylate (150 mg, 0.40 mmol) was dissolved in ethyl acetate, and then palladium on carbon catalyst 10% (47 mg, 0.065 mmol) was added. The mixture was stirred at room temperature for 10 hours under hydrogen gas to obtain 120.2 mg of the title compound (86% yield).

$^1$H NMR (400 MHz, DMSO-$d_6$) δ 7.86 (d, J = 2.4 Hz, 1H), 7.12 (d, J = 2.4 Hz, 1H), 6.60 (s, 1H), 5.16 (s, 2H), 3.67-3.44 (m, 8H), 1.51 (s, 9H).

Step 48-7: Preparation of tert-butyl 5-(2-(dicyanomethylene)hydrazinyl)-2-(morpholine-4-carbonyl)-1H-pyrrolo[2,3-b]pyridine-1-carboxylate (Compound 48-7)

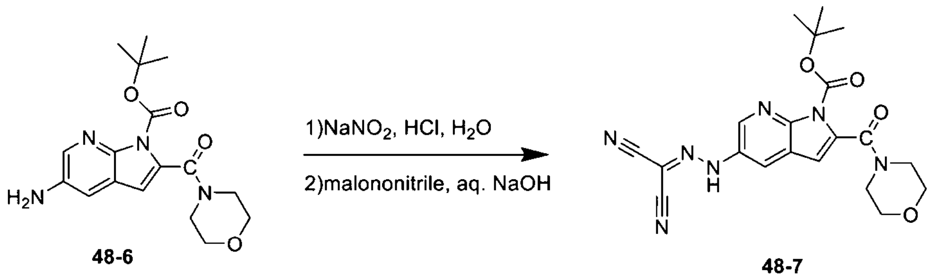


**48-6** **48-7**

Under nitrogen, tert-butyl 5-amino-2-(morpholine-4-carbonyl)-1H-pyrrolo[2,3-b]pyridine-1-carboxylate (50 mg, 0.14 mmol) and sodium nitrite (15 mg, 0.22 mmol) were dissolved in ethanol, and then 1.0 M hydrochloric acid aqueous

solution (0.4 mL, 0.36 mmol) was added at 0°C. The reaction mixture was stirred at 0°C for 1 hour to form a diazonium salt. After the diazonium salt was formed, malononitrile (14 mg, 0.22 mmol) was added, and the mixture was stirred at room temperature for 3.5 hours to obtain 46.6 mg of the title compound (79% yield).

$^{1}$H NMR (400 MHz, DMSO-$d_6$) δ 8.57 (s, 1H), 8.05 (s, 1H), 6.87 (s, 1H), 3.69-3.49 (m, 8H), 1.55 (s, 9H).

Step 48-8: Preparation of N-(2-(morpholine-4-carbonyl)-1*H*-pyrrolo[2,3-b]pyridin-5-yl)carbonohydrazonoyl dicyanide (Compound 48)

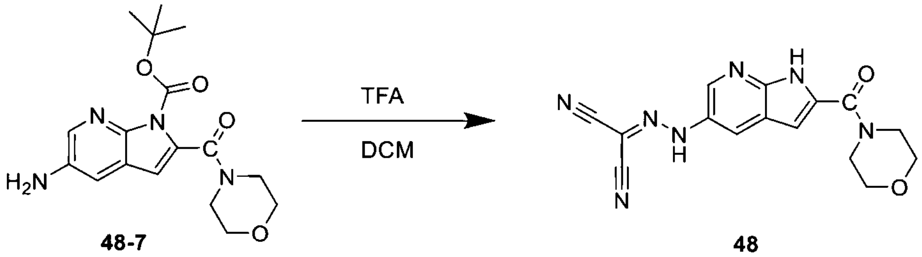


48-7 48

Under nitrogen, tert-butyl 5-(2-(dicyanomethylene)hydrazinyl)-2-(morpholine-4-carbonyl)-1*H*-pyrrolo[2,3-*b*]pyridine-1-carboxylate (40 mg, 0.09 mmol) was dissolved in dichloromethane, and then trifluoroacetic acid (145 μL, 1.89 mmol) was added. The reaction mixture was stirred at room temperature for 1.5 hours to obtain 17.3 mg of the title compound (59.4% yield).

$^{1}$H NMR (400 MHz, DMSO-$d_6$) δ 13.2 (s, 1H), 12.2 (s, 1H), 8.51 (s, 1H), 8.04 (s, 1H), 6.81 (s, 1H) 3.70-3.66 (m, 8H)

**Example 49: Preparation of N-(2-(morpholinooxazolo[4,5-b]pyridin-6-yl)carbonohydrazonoyl dicyanide (Compound 49)**

Step 49-1: Preparation of oxazolo[4,5-b]pyridine-2(3H)-thione (Compound 49-1)

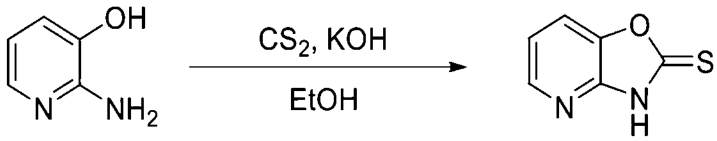


**49-1**

2-Aminopyridin-3-ol (5 g, 45.4 mmol) and potassium hydroxide (7.6 g, 136 mmol) were dissolved in ethanol, and then carbon disulfide (54.6 mL, 908 mmol) was added. The reaction mixture was stirred at 75°C for 24 hours to obtain 5.026 g of the title compound (73% yield).

1H NMR (400 MHz, DMSO-$d_6$) δ 14.49 (s, 1H), 8.24 (d, J = 5.2 Hz, 1H), 7.89 (dd, J = 8.1, 1.5 Hz, 1H), 7.29 (dd, J = 7.9, 5.1 Hz, 1H).

Step 49-2: Preparation of 2-(methylthio)oxazolo[4,5-*b*]pyridine **(Compound 49-2)**

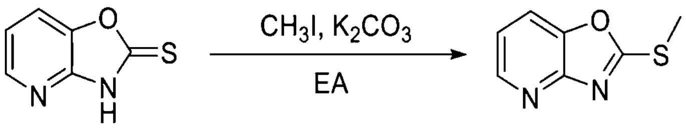


**49-1** **49-2**

Oxazolo[4,5-*b*]pyridine-2(3*H*)-thione (4.8 g, 32 mmol) and potassium carbonate (6.1 g, 44.8 mmol) were dissolved in ethyl acetate, and then methyl iodide (5.0 mL, 78 mmol) was added. The reaction mixture was stirred at room temperature for 12.5 hours to obtain 4.533 g of the title compound (85% yield).
1H NMR (400 MHz, DMSO-$d_6$) δ 8.44 (dd, J = 4.9, 1.5 Hz, 1H), 8.09 (dd, J = 8.2, 1.5 Hz, 1H), 7.35 (ddd, J = 8.4, 4.9, 1.3 Hz, 1H), 2.81 (d, J = 1.2 Hz, 3H).

Step 49-3: Preparation of 2-morpholinooxazolo[4,5-*b*]pyridine **(Compound** 49-**3)**

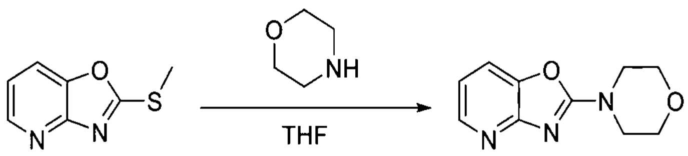


**49-2** **49-3**

2-(Methylthio)oxazolo[4,5-b]pyridine (4.5 g, 27.1 mmol) was dissolved in tetrahydrofuran, and then morpholine (11.6 mL, 135 mmol) was added. The reaction mixture was stirred at 80°C for 13 hours to obtain 3.910 g of the title compound (70.3% yield).
1H NMR (400 MHz, DMSO-$d_6$) δ 8.15 (dd, J = 5.3, 1.5 Hz, 1H), 7.79 - 7.72 (m, 1H), 7.02 (dd, J = 8.1, 5.4 Hz, 1H), 3.74 (t, J = 4.7 Hz, 4H), 3.66 (dd, J = 5.7, 3.8 Hz, 4H).

Step 49-4: Preparation of 2-morpholino-6-nitrooxazolo[4,5-*b*]pyridine **(Compound 49-4)**

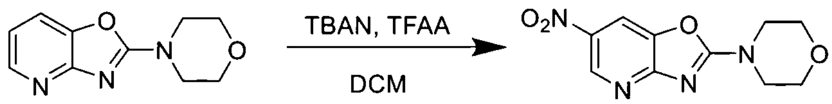


**49-3** **49-4**

Under nitrogen, 2-morpholinooxazolo[4,5-*b*]pyridine (3.6 g, 5.80 mmol) and tetrabutylammonium nitrate (2.6 g, 8.70 mmol) were dissolved in dichloromethane and stirred at room temperature for 3 hours. Thereafter, anhydrous trifluoroacetic acid anhydride (TFAA, 1.6 mL, 11.6 mmol) was added, and the mixture was stirred at 0°C for 10 minutes, and then stirred at room temperature for an additional 31 hours. After the reaction was completed, the mixture was purified by column chromatography to obtain 620 mg of the title compound (14% yield).
1H NMR (400 MHz, DMSO-$d_6$) δ 9.11 (d, J = 2.4 Hz, 1H), 8.56 (d, J = 2.3 Hz, 1H), 3.76 (s, 8H).

Step 49-5: Preparation of 2-morpholinooxazolo[4,5-*b*]pyridin-6-amine **(Compound 49-5)**

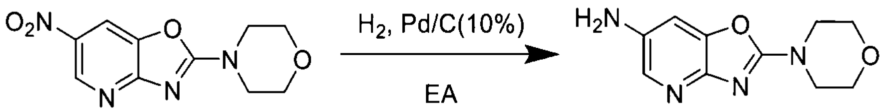


**49-4** **49-5**

2-Morpholino-6-nitrooxazolo[4,5-*b*]pyridine (620 mg, 2.50 mmol) was dissolved in ethyl acetate, and then palladium on carbon catalyst 10% (590 mg, 0.5 mmol) was added. The mixture was stirred at room temperature for 7 hours under hydrogen gas. After the reaction was completed, the mixture was purified by column chromatography to obtain 620 mg of the title compound (20% yield).

1H NMR (400 MHz, DMSO-$d_6$) δ 7.58 (d, *J*= 2.3 Hz, 1H), 7.01 (d, *J*= 2.3 Hz, 1H), 5.05 (s, 2H), 3.72 (t, *J* = 4.9 Hz, 4H), 3.55 (t, *J* = 4.9 Hz, 4H).

Step 49-6: Preparation of *N*-(2-(morpholinooxazolo[4,5-*b*]pyridin-6-yl)carbonohydrazonoyl dicyanide **(Compound 49-6)**

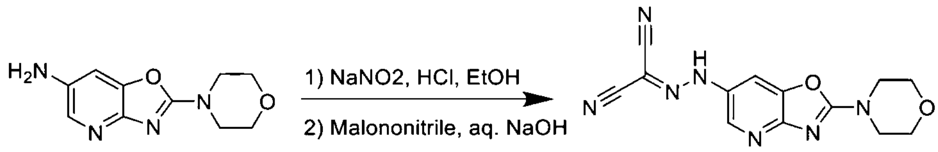


**49-5** **49-6**

2-Morpholinooxazolo[4,5-*b*]pyridin-6-amine (50 mg, 0.23 mmol) and sodium nitrite (24 mg, 0.34 mmol) were dissolved in ethanol, and then 1.0 M hydrochloric acid aqueous solution (0.7 mL, 0.68 mmol) was added at room temperature. The reaction mixture was stirred at room temperature for 1 hour to form a diazonium salt. After the diazonium salt was formed, malononitrile (22.5 mg, 0.34 mmol) was added, and the mixture was stirred at room temperature for 1 hour and purified by column chromatography to obtain 13.2 mg of the title compound (19% yield).
1H NMR (400 MHz, DMSO-$d_6$) δ 13.6 (s, 1H), 8.32 (s, 1H), 7.83 (s, 1H), 3.75 (t, J = 4.6 Hz, 4H), 3.67 (d, J = 4.7 Hz, 4H).

**Comparative Examples 1 to 5: Preparation of benzo[d]thiazol-6-yl)carbonohydrazonoyl dicyanide containing heteroaryl or acyclic substituents**

The following 6 compounds were synthesized according to the method disclosed in the applicant's prior patent application No. 10-2020-0039398 and used as Comparative Compounds 1 to 6.

Comparative Compound 1: N-(2-methylbenzo[d]thiazol-6-yl)carbonohydrazonoyl dicyanide
Comparative Compound 2: N-(2-phenylbenzo[d]thiazol-6-yl) carbonohydrazonoyl dicyanide
Comparative Compound 3: N-(2-(4-fluorophenyl)benzo[d]thiazol-6-yl) carbonohydrazonoyl dicyanide
Comparative Compound 4: N-(2-(4-(dimethylamino)phenyl)benzo[d]thiazol-6-yl) carbonohydrazonoyl dicyanide
Comparative Compound 5: N-(2-(thiophen-2-yl)benzo[d]thiazol-6-yl) carbonohydrazonoyl dicyanide
Comparative Compound 6: N-(2-(6-methylpyridin-3-yl) benzo[d]thiazol-6-yl) carbonohydrazonoyl dicyanide

[Table 2]

| | | | |
|---|---|---|---|
| Comparative Compound 1 | 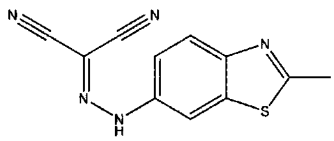 | Comparative Compound 2 | 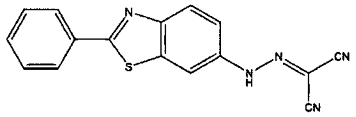 |
| Comparative Compound 3 | 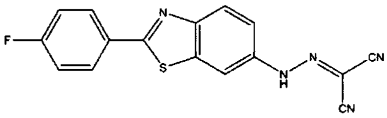 | Comparative Compound 4 | 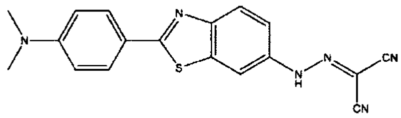 |
| Comparative Compound 5 | 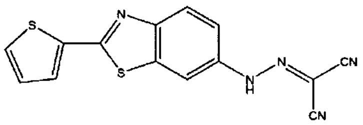 | Comparative Compound 6 | 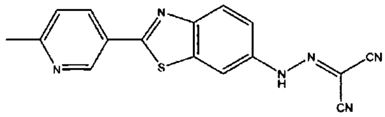 |

**[Formulation Examples]**

The novel compounds represented by Formula 1 according to the disclosure may be formulated in various forms depending on the purpose. The following illustrates several formulation methods containing the compounds represented by Formula 1 according to the disclosure as active ingredients, but the disclosure is not limited thereto.

**Formulation Example 1: Preparation of tablets by direct compression**

5.0 mg of the active ingredient prepared according to Examples 1 to 46 was sieved, and then 14.1 mg of lactose, 0.8 mg of crospovidone USNF, and 0.1 mg of magnesium stearate were mixed and compressed to prepare tablets.

**Formulation Example 2: Preparation of tablets by wet granulation**

5.0 mg of the active ingredient prepared according to Examples 1 to 46 was sieved and mixed with 16.0 mg of lactose and 4.0 mg of starch. After dissolving 0.3 mg of polysorbate 80 in pure water, an appropriate amount of this solution was added to the mixture to granulate. After drying, the granules were sieved, mixed with 2.7 mg of colloidal silicon dioxide and 2.0 mg of magnesium stearate, and the granules were compressed to prepare tablets.

**Formulation Example 3: Preparation of powder and capsules**

5.0 mg of the active ingredient prepared according to Examples 1 to 46 was sieved and mixed with 14.8 mg of lactose, 10.0 mg of polyvinylpyrrolidone, and 0.2 mg of magnesium stearate. The mixture was filled into hard No. 5 gelatin capsules using an appropriate device to prepare capsules.

**Formulation Example 4: Preparation of injections**

An injection was prepared containing 100 mg of the active ingredient prepared according to Examples 1 to 46, and additionally including 180 mg of mannitol, 26 mg of $Na_2HPO_4 \cdot 12H_2O$, and 2974 mg of distilled water.

**Experimental Example 1: Screening for tau aggregation inhibitors using Tau-BiFC cell model**

To screen for new tau aggregation inhibitors, a Tau-BiFC cell model that facilitates observation of tau oligomer formation in living cells was used. Tau-BiFC cells were dispensed into a 384-well plate, and the next day, the compounds prepared according to Examples 1 to 46 were treated at concentrations of 1, 3, and 10 μM, respectively, together with Forskolin (treatment concentration 30 μM), which is a compound that induces tau aggregation by activating the tau phosphorylation enzyme PKA. After 48 hours, nuclei within cells were stained using Hoechst (treatment concentration 2 μg/mL), and BiFC fluorescence intensity was automatically quantified using Operetta (Perkin Elmer) to count the stained nuclei in each well from the entire plate. The group treated only with Forskolin, which induces tau aggregation, was set as the reference point with 100% tau aggregation state, and the effect was confirmed by the formula "BiFC fluorescence intensity by the compounds synthesized according to the Examples of the disclosure/(fluorescence intensity of the control group treated only with Forskolin that induces tau aggregation - fluorescence intensity of the untreated control group) × 100". In addition, the degree of cytotoxicity induced by the newly synthesized compounds was also calculated using the formula "(number of stained nuclei in the compound-treated group/number of stained nuclei in the Forskolin-treated group) × 100", with the group treated only with Forskolin as the reference point at 100% viability. From the treatment results, substances that inhibit intracellular tau aggregation were identified through screening from a series of candidates based on the criteria of 70% or more tau aggregation inhibition and 100% cell viability at a compound treatment concentration of 10 μM.

**Experimental Example 2: Confirmation of concentration-dependent tau aggregation inhibitory effect of novel compounds**

To confirm the tau aggregation inhibitory effect according to the concentration of the compounds screened in Experimental Example 1, the screened compounds were treated to Tau-BiFC cells at concentrations of 0.03, 0.1, 0.3, 1, 3, 10, and 30 μM, respectively, together with Forskolin (treatment concentration 30 μM), which is a tau aggregation-inducing substance. After 48 hours, cell images were examined to analyze the tau aggregation reaction and degree of cytotoxicity. The $IC_{50}$ and toxicity of the compounds were analyzed by nonlinear regression analysis using Prism software (GraphPad). The calculated results for representative compounds are shown in Table 3 below: Tau aggregation inhibitory effect and cell viability of novel compounds.

[Table 3]

| | Tau aggregation inhibitory effect (Tau-BiFC cell line) | | | | |
|---|---|---|---|---|---|
| Compound No. | $IC_{50}$ (nM) | Cell viability (% @10μM) | Compound No. | $IC_{50}$ (nM) | Cell viability (% @10 μM) |
| 1 | 12 | 100 | 2 | 4 | 100 |
| 3 | 20 | 100 | 4 | 56 | 100 |
| 5 | 32 | 100 | 6 | 24 | 104 |
| 7 | 64 | 100 | 8 | 32 | 101 |
| 9 | 36 | 100 | 10 | 24 | 109 |
| 11 | 32 | 100 | 12 | 32 | 102 |
| 13 | 20 | 99 | 14 | 48 | 100 |
| 15 | 48 | 100 | 16 | 49 | 100 |
| 17 | 88 | 100 | 18 | 128 | 100 |
| 19 | 453 | 100 | 20 | 11 | 96 |
| 23 | 48 | 92 | 24 | 168 | 95 |
| 25 | 36 | 94 | 26 | 10 | 99 |
| 27 | 44 | 99 | 28 | 16 | 99 |
| 29 | 36 | 97 | 30 | 60 | 99 |
| 31 | 88 | 100 | 32 | 12 | 98 |
| 33 | 4 | 97 | 34 | 12 | 100 |

(continued)

| | Tau aggregation inhibitory effect (Tau-BiFC cell line) | | | | |
|---|---|---|---|---|---|
| Compound No. | $IC_{50}$ (nM) | Cell viability (% @10μM) | Compound No. | $IC_{50}$ (nM) | Cell viability (% @10 μM) |
| 35 | 12 | 100 | 36 | 136 | 97 |
| 37 | 24 | 85 | 38 | 68 | 97 |
| 39 | 44 | 98 | 40 | 316 | 98 |
| 41 | 260 | 100 | 42 | 20 | 100 |
| 43 | 28 | 100 | 44 | 12 | 100 |
| 45 | 4 | 98 | 46 | 16 | 100 |
| 47 | 117 | 100 | Comparative compound 1 | 500 | 100 |
| Comparative compound 2 | 2,800 | 65 | Comparative compound 3 | 3,200 | 50 |
| Comparative compound 4 | 3,100 | 80 | Comparative compound 5 | 7,300 | 70 |

**Experimental Example 3: Screening for TDP-43 aggregation inhibitors using TDP-43-BiFC cell model**

To screen for new TDP-43 aggregation inhibitors, a TDP-43-BiFC cell model that facilitates observation of TDP-43 aggregate formation in living cells was used. Specifically, TDP-43-BiFC cells were dispensed into a 384-well plate, and the next day, the compounds prepared according to Examples 1 to 46 were treated at concentrations of 3 and 10 μM, respectively, together with Forskolin (treatment concentration 30 μM), which is a compound that induces TDP-43 aggregation by activating the phosphorylation enzyme PKA. After 48 hours, nuclei within cells were stained using Hoechst (treatment concentration 1 μg/mL), and BiFC fluorescence intensity was automatically quantified using Operetta (PerkinElmer) to count the stained nuclei in each well from the entire plate. The group treated only with Forskolin, which induces TDP-43 aggregation, was set as the reference point with 100% TDP-43 aggregation state, and the effect was confirmed by the formula "(BiFC fluorescence intensity by the compounds synthesized according to the Examples of the disclosure - fluorescence intensity of the untreated control group)/(fluorescence intensity of the control group treated only with Forskolin that induces TDP-43 aggregation - fluorescence intensity of the untreated control group) × 100". In addition, the degree of cytotoxicity induced by the newly synthesized compounds was also calculated using the formula "(number of stained nuclei in the compound-treated group/number of stained nuclei in the Forskolin-treated group) × 100", with the group treated only with Forskolin as the reference point at 100% viability. From the treatment results, substances that inhibit intracellular TDP-43 aggregation were identified through screening from a series of candidates based on the criteria of 80% or more TDP-43 aggregation inhibition and 60% cell viability at a compound treatment concentration of 3 μM.

**Experimental Example 4: Confirmation of concentration-dependent TDP-43 aggregation inhibitory effect of novel compounds**

Furthermore, to confirm the TDP-43 aggregation inhibitory effect according to the concentration of the compounds screened in Experimental Example 3, the screened compounds were treated to Tau-BiFC cells at concentrations of 0.003, 0.01, 0.03, 0.1, 0.3, 1, 3, and 10 μM, respectively, together with Forskolin (treatment concentration 30 μM), which is a TDP-43 aggregation-inducing substance. After 48 hours, cell images were examined to analyze the TDP-43 aggregation reaction and degree of cytotoxicity. The $IC_{50}$ and toxicity of the compounds were analyzed by nonlinear regression analysis using Prism software (GraphPad). The calculated results for representative compounds are shown in Table 4 below: TDP-43 protein aggregation inhibitory effect and cell viability of novel compounds.

[Table 4]

| Compound No. | TDP-43 protein aggregation inhibitory effect (TDP-43 BiFC cells) | | | Compound No. | TDP-43 protein aggregation inhibitory effect (TDP-43 BiFC cells) | | |
|---|---|---|---|---|---|---|---|
| | Aggregati on inhibitory effect (%) (@3 uM) | $IC_{50}$ (nM) | Cell viability (%) (@ 3 uM) | | Aggregation inhibitory effect (%) (@3 uM) | $IC_{50}$ (nM) | Cell viability (%) (@ 3 uM) |
| **1** | 78.1 | | 87.5 | **2** | 100 | | 87.5 |
| **3** | 79.6 | | 102.3 | **4** | 100 | | 104.2 |
| **5** | 94.7 | | 109.2 | **6** | 97.4 | | 98.1 |
| **8** | 100 | | 83.0 | **9** | 100 | | 76.2 |
| **10** | 100 | | 83.8 | **11** | 100 | | 80.9 |
| **12** | 100 | | 73.0 | **13** | 100 | | 63.6 |
| **14** | 100 | | 101.4 | **15** | 97.5 | | 105.2 |
| **16** | 100 | | 97.8 | **17** | 100 | | 96.9 |
| **18** | 98.6 | | 102.1 | **19** | 92.6 | | 97.0 |
| **20** | 91.7 | 140 | 75.4 | **22** | 66.5 | | 130.7 |
| **23** | 99.2 | 107 | 81.1 | **24** | 97.9 | 53 | 83.1 |
| **25** | 98.2 | 53 | 76.7 | **26** | 94.8 | 248 | 76.1 |
| **27** | 48.2 | | 104.8 | **28** | 57.1 | | 113.4 |
| **29** | 47.2 | | 96.2 | **30** | 88.0 | 146 | 83.1 |
| **31** | 41.4 | 2,90 1 | 82.7 | **32** | 95.4 | 99 | 59.7 |
| **33** | 93.9 | 124 | 55.0 | **34** | 82.4 | 10 | 59.5 |
| **35** | 62.9 | 192 | 67.4 | **37** | 91.2 | 0.7 | 61.3 |
| **38** | 49.6 | | 111.9 | **39** | 58.7 | | 117.7 |
| **40** | 67.7 | | 95.6 | **41** | 87.8 | | 71.5 |
| **42** | 64.7 | | 62.4 | **43** | 92.9 | | 89.0 |
| **44** | 71.1 | | 85.0 | **45** | 81.1 | 371 | 73.6 |
| **46** | 81.4 | 320 | 73.6 | **Comparative compound 1** | 72.9 | | 79.0 |
| **Comparative compound 6** | 69.5 | | 60.8 | | | | |

**Experimental Example 5: Confirmation of liver microsomal stability of novel compounds**

Liver microsomal stability by the compounds prepared according to Examples 1 to 46 was confirmed. Specifically, four types of liver microsomes (human, dog, rat, and mouse each at 0.5 mg/mL), 0.1 M phosphate buffer solution (pH 7.4), and the compounds at 1 μM concentration were added and pre-incubated at 37°C for 5 minutes, and then NADPH generation system solution was added and incubated at 37°C for an additional 30 minutes. Thereafter, an acetonitrile solution containing an internal standard (chloropropamide) was added to terminate the reaction, and after centrifugation for 5 minutes (14,000 rpm, 4°C), the supernatant was injected into an LC-MS/MS system to analyze the substrate drug, thereby evaluating the metabolic stability of 8 compounds.

The amount of remaining substrate through the reaction was analyzed using a Shimadzu Nexera XR system and TSQ vantage (Thermo). A Kinetex XB-C18 (2.1 × 100 mm, 2.6 μm, particle size; Phenomenex, USA) was used as the HPLC column, and (A) distilled water containing 0.1% formic acid and (B) acetonitrile containing 0.1% formic acid were used as mobile phases. Analyst software (version 1.6.3) and Xcalibur (version 1.6.1) were used for data analysis. The calculated results are shown in Table 5 below: Liver microsomal stability data of novel compounds from four species

(human, rat, and mouse).

[Table 5]

| Compound # | Human microsomes (% remaining) | Rat microsomes (% remaining) | Mouse microsomes (% remaining) |
|---|---|---|---|
| 1 | 91.2 | 2.1 | 49.3 |
| 2 | 74.3 | 55.2 | 94.10 |
| 3 | 99.4 | 73.0 | 80.6 |
| 4 | 93.5 | 80.9 | 98.5 |
| 5 | 77.8 | 77.8 | 91.9 |
| 6 | 90 | 66.1 | 87.3 |
| 8 | 83.9 | 76.6 | 77.4 |
| 9 | 5.0 | 17.7 | 83.8 |
| 10 | 69.4 | 84.8 | 87.3 |
| 11 | 81.3 | 73.9 | 87.8 |
| 12 | 62.7 | 70.1 | 85.1 |
| 13 | 79.9 | 47.7 | 84.8 |
| 15 | > 100 | 60 | > 100 |
| 16 | 79.8 | 49.6 | 89.5 |
| 17 | 16.7 | 59.3 | > 100 |
| 18 | 95.8 | 72.9 | 86.2 |
| 20 | > 100 | 82.9 | 95.4 |
| 23 | 85.6 | 69.8 | 86.6 |
| 26 | 96.3 | 95.1 | 99 |
| 28 | 11.7 | 50.7 | 81.8 |
| 30 | > 100 | 91.9 | 83.8 |
| 31 | 99.9 | 88.3 | > 100 |
| 32 | 95.5 | 81.7 | 92.3 |
| 33 | 94.9 | 96.8 | 89 |
| 34 | 88.9 | 97.1 | 78.2 |
| 35 | 94.3 | 74.1 | 95.1 |
| 38 | 87.6 | 96.0 | 86.1 |
| 39 | 83.8 | 82.3 | 88.4 |
| 40 | 94.2 | 87.7 | 93.4 |
| 41 | 70.0 | 19.0 | 65.3 |
| Comparative compound 1 | 37.0 | 1.2 | 1.2 |
| Comparative compound 6 | 84.7 | 83.9 | >100 |

**Experimental Example 6: CYP isoenzyme activity inhibitory effect of novel compounds**

The CYP isoenzyme activity inhibitory effect by the compounds prepared according to Examples 1 to 46 was confirmed. Specifically, human liver microsomes (0.25 mg/mL), 0.1 M phosphate buffer solution (pH 7.4), a substrate drug cocktail of five drug metabolizing enzymes (Phenacetin 50 μM, Diclofenac 10 μM, S-mephenytoin 100 μM, Dextromethorphan 5 μM, and Midazolam 2.5 μM), and the compounds were added at concentrations of 0 or 10 μM, respectively,

and pre-incubated at 37°C for 5 minutes, and then NADPH generation system solution was added and incubated at 37°C for an additional 15 minutes. Thereafter, an acetonitrile solution containing an internal standard (Terfenadine) was added to terminate the reaction, and after centrifugation for 5 minutes (14,000 rpm, 4°C), the supernatant was injected into an LC-MS/MS system to simultaneously analyze the metabolites of the substrate drugs, thereby evaluating the drug metabolizing enzyme inhibitory activity.

The metabolites of each CYP isoenzyme marker drug generated through the reaction were analyzed using a Shimadzu Nexera XR system and TSQ vantage (Thermo). A Kinetex C18 (2.1 × 100 mm, 2.6 μm, particle size; Phenomenex, USA) was used as the HPLC column, and (A) distilled water containing 0.1% formic acid and (B) acetonitrile containing 0.1% formic acid were used as mobile phases, and a gradient program as shown in Table 6 below was applied.

[Table 6]

| Time (min) | Flow rate (mL/min) | %A | %B |
|---|---|---|---|
| 0 | 0.3 | 100 | 0 |
| 1.0 | 0.3 | 60 | 40 |
| 4.0 | 0.3 | 50 | 50 |
| 4.1 | 0.3 | 100 | 0 |
| 7.0 | 0.3 | 100 | 0 |

The generated metabolites were quantified using MRM (Multiple Reaction Monitoring) quantitative mode, and Xcalibur (version 1.6.1) was used for data analysis. To show the CYP isoenzyme activity inhibitory effect of the novel compounds prepared according to the Examples of the disclosure, the % activity of CYP isoenzymes relative to the control group without compound addition is shown in Table 6 below. Additionally, to confirm the difference in effect according to substituents, Comparative Compounds 1 and 5, which are two compounds that share a similar parent nucleus structure with Compounds 1 to 46 of the disclosure while Cy is substituted with aromatic heteroaryl instead of saturated heterocyclyl, were selected as comparative compounds and their activities are shown together.

[Table 7]

| Compound No. | CYP1A2 (% activity) | CYP2C9 (% activity) | CYP2C19 (% activity) | CYP2D6 (% activity) | CYP3A4 (% activity) |
|---|---|---|---|---|---|
| 1 | 80.2 | 82.5 | > 100 | > 100 | 95.5 |
| 2 | 81.7 | 95.1 | 96.7 | > 100 | 95.7 |
| 3 | 99.9 | > 100 | > 100 | > 100 | > 100 |
| 4 | 86.8 | 93.2 | 93.4 | > 100 | 94 |
| 5 | 84.3 | 98 | 90.2 | > 100 | 86.1 |
| 6 | 92.3 | > 100 | 98.4 | > 100 | 96.7 |
| 7 | 57.6 | 67.8 | 95.1 | > 100 | 83.1 |
| 8 | 91.8 | > 100 | > 100 | > 100 | 95 |
| 9 | 68.9 | 60.7 | 86.9 | > 100 | 66.1 |
| 10 | 68.4 | 76.8 | 83.6 | 93.8 | 74.5 |
| 11 | 83.7 | 70.2 | 80.3 | 98.6 | 78.4 |
| 12 | 74.9 | 65.1 | 84.8 | 94.8 | 78.9 |
| 13 | 83 | 75 | 83.3 | > 100 | 84.8 |
| 14 | 89.7 | 88 | 95.5 | > 100 | 96.8 |
| 15 | 80.2 | 88.6 | 87.9 | > 100 | 87.3 |
| 16 | 99.9 | 87 | > 100 | 92.1 | 96.7 |
| 17 | 83.2 | 73.8 | 90 | 89.5 | 87.1 |
| 18 | 98.9 | > 100 | 100 | 97 | 94.6 |

(continued)

| Compound No. | CYP1A2 (% activity) | CYP2C9 (% activity) | CYP2C19 (% activity) | CYP2D6 (% activity) | CYP3A4 (% activity) |
|---|---|---|---|---|---|
| 20 | 55.6 | 48 | 51.8 | 57.5 | 55.4 |
| 23 | 70.6 | 68.5 | 69.6 | 71.5 | 71.7 |
| 24 | 65.1 | 72.0 | 67.9 | 74.2 | 74.7 |
| 25 | 87 | 93.5 | 85.7 | 93.5 | 91.5 |
| 26 | 79.1 | 53.3 | 82.1 | 82.1 | 81.3 |
| 27 | 95.1 | 58.5 | 95.4 | 92.3 | 59.8 |
| 28 | 95.5 | 48.5 | 90.8 | 78 | 87.8 |
| 30 | 88.9 | 86.2 | 86.2 | 88.9 | 74.6 |
| 31 | 92.7 | 88.8 | 90.8 | 93.3 | 88.3 |
| 32 | 78.9 | 65.1 | 77.1 | 92.1 | 73 |
| 33 | 75.5 | 28.8 | 78.6 | 97.9 | 75.4 |
| 34 | 85.8 | 80.8 | 80.6 | 92.4 | 84.3 |
| 35 | 81.5 | 84.3 | 82 | 94.9 | 86.1 |
| 38 | 83.9 | 73.6 | 80 | 95.7 | 86.7 |
| 39 | 84 | 42.9 | 78.6 | 85.9 | 56.5 |
| 40 | 80.2 | 46.9 | 65.7 | 88.6 | 75.5 |
| 41 | 71.9 | 51.2 | 86.7 | 86.8 | 86.5 |
| Comparative compound 6 | 80.2 | 32.0 | 81.2 | 91.8 | 81.7 |

**Experimental Example 7: Pharmacokinetic study of novel compounds**

The pharmacokinetics of the compounds prepared according to Examples 1 to 46 were studied using experimental animal models. Specifically, 7- to 8-week-old SD rats (Koatech, Hana Sangsa Co., Ltd.) weighing approximately 250 to 300 g were purchased and maintained in a small animal simple breeding room set at a temperature of 22 ± 2°C, relative humidity of 50 ± 5%, lighting time of 12 hours (lights on at 8 AM and lights off at 8 PM), and illumination of 150 to 300 lux. Throughout the entire test period, feed was provided ad libitum, RO water was provided ad libitum, and fasting was performed for 16 hours before oral administration of the drug. After preparing the drugs under the conditions shown in Table 7 below, the drugs were administered by intravenous injection (IV) or oral administration (PO). Collected blood at 500 μL was collected in tubes containing heparin sodium and centrifuged at 8000 rpm at 2 to 8°C for 6 minutes to obtain plasma from the blood. 20 μL of the obtained plasma was taken, added to 180 μL of acetonitrile containing an internal standard, vortexed, and centrifuged at 15,000 rpm at 4°C for 5 minutes, and the obtained supernatant was analyzed by LC-MS/MS. The HPLC and MS conditions used for analysis are shown in Tables 8 and 9 below, respectively.

[Table 8]

| Test group | Administration route | Formulation composition | Dose(mg/kg) | Dose volume(μL) |
|---|---|---|---|---|
| 1 | IV | 10% solutol, 10% PEG400<br>80% DW (1% 1N NaOH) | 1 | 250 |
| 2 | PO | 10% solutol, 10% PEG400<br>80% DW (1% 1N NaOH) | 15 | 500 |

[Table 9]

| | |
|---|---|
| HPLC system | Nexera XR system (Shimadzu, Japan) |
| Column | Kinetex C18 Column (2.1×100 mm, 2.6 μm, Particle size; Phenomenex, USA) |

(continued)

| | |
|---|---|
| Injection volume | 2 μL |
| Mobile phase | (A) 0.1% Formic acid aqueous solution<br>(B) 0.1% Formic acid acetonitrile solution |
| Sample analysis time | 3.5 minutes |
| Retention time | 2.2 minutes |

[Table 10]

| | |
|---|---|
| Analysis system | TSQ vantage triple quadruple(Thermo, USA) |
| Molecular weight | 312.35 g/mol |
| Ion source type & Ionization mode | Turbo Spray Ionization, negative mode<br>- MRM transition (m/z): 263.090 → 185.1<br>- CE (V): 19, S-lens:83 |
| Lower Limit of Quantification (LLOQ) | - Plasma: 1 ng/mL<br>- Brain: 1 ng/mL |
| Standard curve range | - Plasma: 1 to 5000 ng/mL<br>- Brain: 1 to 2000 ng/mL |

Specific PK parameters were calculated using Phoenix WinNonlin 6.4 version (Pharsight, USA) program and a non-compartmental analysis model, and the results are summarized in Table 11 below.

[Table 11]

| Compound | Plasma PK parameter | | | | | | | |
|---|---|---|---|---|---|---|---|---|
| | Administ ration route | Dose (mg/kg) | $AUC_{all}$ (hr. ng/mL) | $T_{1/2}$ (hr) | $C_{max}$ (ng/mL) | $T_{max}$ (hr) | CL (mL/mi n/kg) | F (%) |
| 6 | IV | 2 | 1701.34 | 0.58 | 9868.51 | - | 19.5 | 42.0 |
| | PO | 10 | 3,569.92 | 0.89 | 4881.41 | 0.25 | - | |
| 16 | IV | 2 | 4,090.37 | 0.51 | 4092.93 | - | 8.33 | 113.9 |
| | PO | 10 | 23,312.94 | 1.87 | 11,145.04 | 0.67 | - | |
| 20 | IV | 1 | 130.1 | 0.2 | - | - | 131.5 | 113.5 |
| | PO | 15 | 2,214.2 | 1.6 | 2046.6 | 0.4 | | |

**Experimental Example 8: Analysis of cognitive function improvement effect of novel compounds in tau transgenic animal model (TauP301L-BiFC Tg mice)**

Compounds 6, 16, and 20 were orally administered to 8.2-month-old tau transgenic animal model (TauP301L-BiFC tg mouse) 5 times per week at a concentration of 10 mg/kg for a total of 19 weeks (n=10/group). WT mice of similar age to the vehicle-treated group of TauP301L-BiFC mice were used as a control group. Three types of behavioral experiments (Novel Objective Recognition test, Y-maze test, Balance beam test) were performed to confirm the improvement effects on cognitive function and motor function by drug administration, and these experiments and their results were as follows.

(1) Novel Objective Recognition test: To evaluate the object recognition memory ability of mice, two identical objects (Old-Old) were explored for 10 minutes in a 40 cm × 40 cm box. The next day, one object was replaced with a new object (Old-New) and exploration was performed again for 10 minutes, and then the time showing exploratory behavior toward the existing object and the new object was measured. Mice with normal object recognition ability (Recognition index) spent more time exploring the newly changed object, whereas mice with low object recognition ability could not distinguish the new object. The object recognition ability (Recognition index) values were as follows:

(RI, Vehicle= 0.48, n.s., #20= 0.59, $p<0.05$, #6= 0.66, $p<0.0001$, #16= 0.58, $p<0.05$). In TauP301L-BiFC mice, the object recognition function improvement effect by drugs #20, #6, and #16 compared to the vehicle group was confirmed.

(2) Y-maze test: A Y-maze test was performed to evaluate the spatial working memory ability of mice. Whether the mice entered the three arms of the Y-maze by spontaneous alternation behavior for a certain period of time was measured through video recording. An increase in the spontaneous alternation ratio was considered as improved working memory ability. The spontaneous alternation ratio was as follows: (Alteration ratio (%), vehicle= 60.7%, #20= 70.8%, $p<0.05$, #6= 71.1%, $p<0.05$, #16= 76.4%, $p<0.001$). In TauP301L-BiFC mice, the spatial working ability improvement effect by drugs #20, #6, and #16 compared to the vehicle group was confirmed.

(3) Balance beam test: A Balance beam test was performed to evaluate the balance and motor coordination ability of mice. The time taken to cross a rod with a height of 50 cm and length of 80 cm and the number of times the feet went outside the rod during crossing were measured. (Average time taken to cross the rod (sec), vehicle= 10.5s, ##20= 8.0s, n.s, #6= 7.9s, n.s, #16= 6.7s, $p<0.05$), (Average number of times feet went outside the rod (n), vehicle= 1.5, ##20= 0.7, $p<0.01$, #6= 1.1, n.s, #16= 0.8, $p<0.05$). In TauP301L-BiFC mice, the balance and motor function improvement effect by drugs #20, #6, and #16 compared to the vehicle group was confirmed.

The experimental process and results described above are shown in FIG. 1.

**Experimental Example 9: Analysis of tau pathology improvement effect of novel compounds in tau transgenic animal model (TauP301L-BiFC Tg mice)**

After oral administration of Compounds 6, 16, and 20 and completion of behavioral experiments, 13.2-month-old TauP301L-BiFC mice were sacrificed to proceed with [1] brain tissue extraction and [2] brain lysate separation and purification.

[1] First, brain tissue extraction, BiFC fluorescence imaging of brain tissue, and tau phosphorylation antibody immunofluorescence were performed through the following process. TauP301L-BiFC mice were perfused with 0.9% saline and fixed with PBS (pH 7.4) solution containing 4% paraformaldehyde. The brain was extracted and fixed in PBS containing 4% paraformaldehyde at 4°C for 48 to 72 hours. For cryoprotection, the brain was transferred to PBS containing 30% sucrose solution and stored at 4°C until it sank. For frozen sectioning, the brain was immersed in O.C.T (Tissue-TEK) and frozen, and then continuously cut using a brain tissue sectioner. Tissue sections with a thickness of 30 μm were stored in PBS containing 0.05% sodium azide at 4°C.

Brain tissue sections (n=10 per group) were placed on slides, stained with Sudan Black B solution (70% ethanol containing 0.05% Sudan Black B) for 10 minutes to reduce autofluorescence, washed 3 times with PBS containing 0.1% Triton X-100, and then washed with distilled water. For nuclear staining, brain tissue sections were stained with Hoechst (0.5 μg/mL) for 30 minutes. BiFC fluorescence ($\lambda_{ex}$=460-490 nm and $\lambda_{em}$=500-550 nm) images were acquired using Axio Scan.Z1 (ZEISS). For quantitative analysis, the average BiFC-fluorescence intensity was measured in the somatosensory cortex (layer V) and hippocampus CA1, and the habenular region was used as a control region to reduce background fluorescence differences between brain samples. ImageJ software (NIH) was used to mark regions of interest and calculate fluorescence intensity values, and the data were expressed as the average fluorescence intensity and standard deviation of brain tissue images from 10 mice per group.

As a result of Tau-BiFC fluorescence imaging of four regions of total brain tissue (hippocampus (CA1), somatosensory cortex, cerebellum, brainstem), the degree of Tau-BiFC fluorescence appearing as tau aggregation early oligomer formation and aggregate formation was as follows:

(1) Hippocampus (CA1), vehicle = 100%, #20= 79%, $p<0.05$, #6= 80%, $p<0.05$, #16= 72%, $p<0.001$.
(2) Sensory cortex (SCTX), vehicle = 100%, #20= 72%, $p<0.05$, #6= 73%, $p<0.05$, #16= 66%, $p<0.01$.
(3) Cerebellum (CB), vehicle = 100%, #20= 64%, $p<0.05$, #6= 68%, ns, #16= 76%, n.s.
(4) Brain stem, vehicle = 100%, #20= 60%, $p<0.05$, #6= 62%, $p<0.05$, #16= 60%, $p<0.05$.

In TauP301L-BiFC mice, the tau pathology improvement effect through inhibition of tau oligomer formation by Compounds 6, 16, and 20 compared to vehicle was confirmed, and this is shown in FIG. 2.

To measure not only tau oligomer formation but also the degree of tau phosphorylation observed in the tau pathology process, immunofluorescence staining using a tau phosphorylation antibody was performed. For brain tissue immunofluorescence image analysis, brain tissue slices (n=10 per group) were stained with AT8 (pS202/T205) antibody (1:200), and the primary antibody was detected using Alexa Fluor 633-labeled anti-mouse secondary antibody (1:500). Fluorescence images ($\lambda_{ex}$=620-640 nm and $\lambda_{em}$=650-700 nm) were acquired using Axio Scan.Z1 (ZEISS).

The AT8 fluorescence imaging results of a total of two brain tissue regions (hippocampus (CA1), somatosensory cortex) were as follows:

(1) Hippocampus (CA1), vehicle = 100%, #20= 72%, $p<0.01$, #6= 81%, $p<0.05$, #16= 76%, $p<0.01$.
(2) Sensory cortex (SCTX), vehicle = 100%, #20= 54%, $p<0.0001$, #6= 63%, $p<0.001$, #16= 57%, $p<0.0001$.

In TauP301L-BiFC mice, the tau pathology improvement effect through inhibition of tau phosphorylation by Compounds 6, 16, and 20 compared to vehicle was confirmed, and this is shown in FIG. 3.

[2] To verify the tau pathology inhibitory efficacy of the drug confirmed through brain tissue imaging via brain lysate immunoblot, brain lysates were separated and purified, and immunoblot using tau antibody was performed in soluble and insoluble fractions. The brain was weighed, and RIPA lysis buffer containing the corresponding dose of protease and phosphatase inhibitors was added to the brain tissue. The tissue was then disrupted using a homogenizer and incubated at 4°C for 2 hours, and then centrifuged at 13,200 rpm at 4°C for 20 minutes. After centrifugation, the supernatant was collected as a RIPA-soluble fraction and stored at -80°C. To prepare the RIPA-insoluble fraction, the remaining pellet was washed once with RIPA lysis buffer containing 1 M sucrose, and then 2% SDS solution (1 mL per 1 g of tissue) was added and incubated at room temperature for 1 hour. Thereafter, the mixture was centrifuged at 13,200 rpm for 1 minute at room temperature, and the supernatant was collected as a RIPA-insoluble fraction and stored at -80°C.

For tau immunoblot analysis, 20 μg of brain tissue RIPA-soluble sample was separated on a 10% SDS-PAGE gel and transferred to a PVDF membrane, and RIPA-insoluble samples were diluted 10-fold in 2% SDS solution and 20 μL of each sample was loaded on an SDS-PAGE gel. The total tau and phosphorylated tau levels were detected by Tau5, which is an anti-tau antibody, and pS199 and pS396, which are anti-phosphorylated tau antibodies. ImageJ software (NIH) was used to quantify band intensity.

The results of two types of tau phosphorylation immunoblots using RIPA-soluble brain lysates were as follows:

(1) pS199: vehicle = 100%, #20= 60%, $p<0.05$, #6= 48%, $p<0.01$, #16= 24%, $p<0.001$.
(2) pS396: vehicle = 100%, #20= 71%, $p<0.05$, #6= 60%, $p<0.01$, #16= 40%, $p<0.001$.

As a result of immunoblot using brain lysates of TauP301L-BiFC mice, the tau pathology improvement effect through inhibition of tau phosphorylation by Compounds 6, 16, and 20 compared to vehicle in the soluble fraction was confirmed.

The results of total tau immunoblot using RIPA-insoluble brain lysates were as follows:

Total tau (Tau5): vehicle = 100%, #20= 23%, $p<0.0001$, #6= 14%, $p<0.0001$, #16= 20%, $p<0.0001$.

As a result of immunoblot using brain lysates of TauP301L-BiFC mice, the tau pathology improvement effect through inhibition of insoluble tau aggregate formation by Compounds 6, 16, and 20 compared to vehicle in the insoluble fraction was confirmed.

The experimental results are shown in FIG. 4.

While the embodiments have been described with reference to the limited drawings, it will be apparent to one of ordinary skill in the art that various alterations and modifications can be made from the above description. For example, suitable results may be achieved if the described techniques are performed in a different order, and/or if components in a described system, architecture, device, or circuit are combined in a different manner, or replaced or supplemented by other components or their equivalents.

Therefore, other implementations, other embodiments, and equivalents to the claims are also within the scope of the following claims.

## Claims

**1.** A carbonohydrazonoyl dicyanide compound represented by Formula 1 or a pharmaceutically acceptable salt thereof:

[Formula 1]

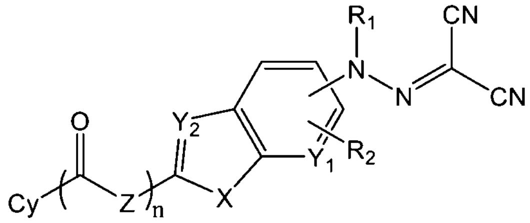

wherein in Formula 1,

n is 0 or 1;
$R_1$ is any one selected from hydrogen, $C_{1-4}$ alkyl, or $C_{1-4}$ alkylcarbonyl;
$R_2$ is absent or halogen;
X is any one selected from O, S or $NR_3$;
$R_3$ is hydrogen, or $C_{1-4}$ alkyl;
$Y_1$ and $Y_2$ are C or N;
Z is absent or NH; and
Cy is a 5- to 10-membered saturated heterocyclyl which is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.

**2.** The carbonohydrazonoyl dicyanide compound or pharmaceutically

acceptable salt thereof of claim 1,
wherein $R_1$ is any one selected from hydrogen, methyl, or acetyl;
$R_2$ is absent, or chlorine;
X is any one selected from O, S, or $NR_3$;
$R_3$ is any one selected from hydrogen, methyl, or ethyl; and
Cy is a 5- to 10-membered saturated heterocyclyl substituted with one or more selected from the group consisting of fluoro, methyl, isopropyl, methoxy, methylcarbonyl, and tert-butoxycarbonyl.

**3.** The carbonohydrazonoyl dicyanide compound or pharmaceutically acceptable salt thereof of claim 1, wherein the carbonohydrazonoyl dicyanide compound represented by Formula 1 is represented by Formula 1-1 or Formula 1-2:

[Formula 1-1]

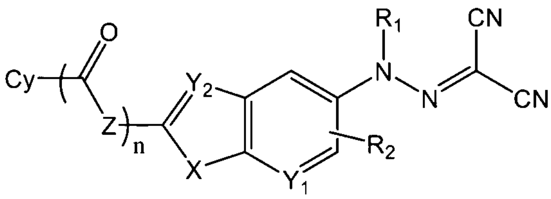

[Formula 1-2]

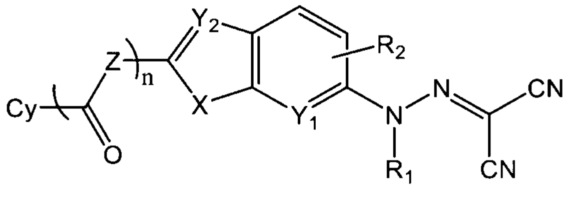

.

**4.** The carbonohydrazonoyl dicyanide compound or pharmaceutically acceptable salt thereof of claim 1, wherein the carbonohydrazonoyl dicyanide compound is

1. (2-morpholinobenzo[d]thiazol-5-yl)carbonohydrazonoyl dicyanide,

2. (2-morpholinobenzo[d]thiazol-6-yl)carbonohydrazonoyl dicyanide,
3. (2-morpholinobenzo[d]oxazol-5-yl)carbonohydrazonoyl dicyanide,
4. (2-morpholinobenzo[d]oxazol-6-yl)carbonohydrazonoyl dicyanide,
5. (1-methyl-2-morpholino-1H-benzo[d]imidazol-6-yl)carbonohydrazonoyl dicyanide,
6. (1-methyl-2-morpholino-1H-benzo[d]imidazol-5-yl)carbonohydrazonoyl dicyanide,
7. (2-morpholino-1H-benzo[d]imidazol-6-yl)carbonohydrazonoyl dicyanide,
8. (2-(4-methylpiperazin-1-yl)benzo[d]thiazol-6-yl)carbonohydrazonoyl dicyanide,
9. (2-(4-methoxypiperidin-1-yl)benzo[d]thiazol-6-yl)carbonohydrazonoyl dicyanide,
10. (2-(2,6-dimethylmorpholino)benzo[d]thiazol-6-yl)carbonohydrazonoyl dicyanide,
11. (2-(3-oxa-8-azabicyclo[3.2.1]octan-8-yl)benzo[d]thiazol-6-yl)carbonohydrazonoyl dicyanide,
12. (2-(2-methylmorpholino)benzo[d]thiazol-6-yl)carbonohydrazonoyl dicyanide,
13. (2-(3-methylmorpholino)benzo[d]thiazol-6-yl)carbonohydrazonoyl dicyanide,
14. (2-(4-methylpiperazin-1-yl)benzo[d]oxazol-6-yl)carbonohydrazonoyl dicyanide,
15. (2-(2-methylmorpholino)benzo[d]oxazol-6-yl)carbonohydrazonoyl dicyanide,
16. (2-(3-methylmorpholino)benzo[d]oxazol-6-yl)carbonohydrazonoyl dicyanide,
17. (2-(4-methoxypiperidin-1-yl)benzo[d]oxazol-6-yl)carbonohydrazonoyl dicyanide,
18. (2-(2,6-dimethylmorpholino)benzo[d]oxazol-6-yl)carbonohydrazonoyl dicyanide,
19. (5-chloro-2-morpholinobenzo[d]oxazol-6-yl)carbonohydrazonoyl dicyanide,
20. (2-(3-oxa-8-azabicyclo[3.2.1]octan-8-yl)benzo[d]oxazol-6-yl)carbonohydrazonoyl dicyanide,
21. tert-butyl 4-(6-(2-(dicyanomethylene)hydrazinyl)benzo[d]oxazol-2-yl)piperazine-1-carboxylate,
22. tert-butyl 4-(6-(2-(dicyanomethylene)hydrazinyl)benzo[d]thiazol-2-yl)piperazine-1-carboxylate,
23. (2-(8-oxa-3-azabicyclo[3.2.1]octan-3-yl)benzo[d]thiazol-6-yl)carbonohydrazonoyl dicyanide,
24. (2-(piperazin-1-yl)benzo[d]thiazol-6-yl)carbonohydrazonoyl dicyanide,
25. (2-(piperazin-1-yl)benzo[d]oxazol-6-yl)carbonohydrazonoyl dicyanide,
26. (2-(8-oxa-3-azabicyclo[3.2.1]octan-3-yl)benzo[d]oxazol-6-yl)carbonohydrazonoyl dicyanide,
27. (2-(4-isopropylpiperazin-1-yl)benzo[d]thiazol-6-yl)carbonohydrazonoyl dicyanide,
28. (2-(4-isopropylpiperazin-1-yl)benzo[d]oxazol-6-yl)carbonohydrazonoyl dicyanide,
29. (2R,6S)-tert-butyl 4-(6-(2-(dicyanomethylene)hydrazinyl)benzo[d]thiazol-2-yl)-2,6-dimethylpiperazine-1-carboxylate,
30. (2-(4-acetylpiperazin-1-yl)benzo[d]thiazol-6-yl)carbonohydrazonoyl dicyanide,
31. (2-(4-acetylpiperazin-1-yl)benzo[d]oxazol-6-yl)carbonohydrazonoyl dicyanide,
32. (1-ethyl-2-morpholino-1H-benzo[d]imidazol-6-yl)carbonohydrazonoyl dicyanide,
33. (1-ethyl-2-morpholino-1H-benzo[d]imidazol-5-yl)carbonohydrazonoyl dicyanide,
34. (2-(3-oxa-8-azabicyclo[3.2.1]octan-8-yl)-1-methyl-1H-benzo[d]imidazol-6-yl)carbonohydrazonoyl dicyanide,
35. (2-(3-oxa-8-azabicyclo[3.2.1]octan-8-yl)-1-methyl-1H-benzo[d]imidazol-5-yl)carbonohydrazonoyl dicyanide,
36. (2-(3-oxa-8-azabicyclo[3.2.1]octan-8-yl)benzo[d]thiazol-6-yl)(methyl)carbonohydrazonoyl dicyanide,
37. acetyl(2-morpholinobenzo[d]thiazol-6-yl)carbonohydrazonoyl dicyanide,
38. (2-(morpholine-4-carboxamido)benzo[d]thiazol-6-yl)carbonohydrazonoyl dicyanide,
39. (2-(2,6-dimethylmorpholine-4-carboxamido)benzo[d]thiazol-6-yl)carbonohydrazonoyl dicyanide,
40. (2-(8-oxa-3-azabicyclo[3.2.1]octane-3-carboxamido)benzo[d]thiazol-6-yl)carbonohydrazonoyl dicyanide,
41. (R)-(2-(3-fluoropyrrolidin-1-yl)benzo[d]oxazol-6-yl)carbonohydrazonoyl dicyanide,
42. (S)-(2-(3-fluoropiperidin-1-yl)benzo[d]oxazol-6-yl)carbonohydrazonoyl dicyanide,
43. (S)-(2-(3-fluoropyrrolidin-1-yl)benzo[d]oxazol-6-yl)carbonohydrazonoyl dicyanide,
44. (2-(3-fluoropiperidin-1-yl)benzo[d]oxazol-6-yl)carbonohydrazonoyl dicyanide,
45. (2-(pyrrolidin-1-yl)benzo[d]oxazol-6-yl)carbonohydrazonoyl dicyanide,
46. (2-(piperidin-1-yl)benzo[d]oxazol-6-yl)carbonohydrazonoyl dicyanide,
47. (R)-N-(2-(3-fluoropiperidin-1-yl)benzo[d]oxazol-6-yl)carbonohydrazonoyl dicyanide,
48. N-(2-(morpholine-4-carbonyl)-1H-pyrrolo[2,3-b]pyridin-5-yl)carbonohydrazonoyl dicyanide, or
49. N-(2-morpholinooxazolo[4,5-b]pyridin-6-yl)carbonohydrazonoyl dicyanide.

**5.** A method of preparing a compound represented by Formula 1, comprising a first step of forming an imine bond by reacting a compound represented by Formula 2, which includes a reactive amine group at one terminal, with sodium nitrite and malononitrile in the presence of an acid; and
optionally, when $R_1$ is a substituent other than hydrogen, further comprising a second step of introducing an $R_1$ substituent into a product obtained from the first step:

[Formula 1]

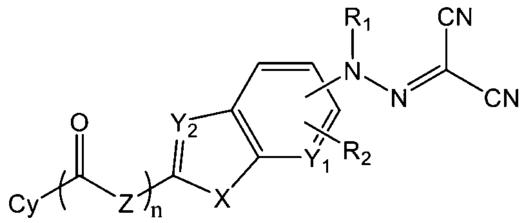

[Formula 2]

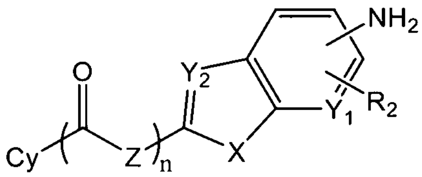

,

wherein in Formulas 1 and 2,

n is 0 or 1;
$R_1$ is any one selected from hydrogen, $C_{1-4}$ alkyl, or $C_{1-4}$ alkylcarbonyl;
$R_2$ is absent or halogen;
X is any one selected from O, S or $NR_3$;
$R_3$ is hydrogen, or $C_{1-4}$ alkyl;
$Y_1$ and $Y_2$ are C or N;
Z is absent or NH; and
Cy is a 5- to 10-membered saturated heterocyclyl which is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.

**6.** The method of claim 5, wherein the second step is performed by:

i) when $R_1$ is $C_{1-6}$ alkyl, reacting with a halogenated alkane in the presence of potassium tert-butoxide or sodium hydride in an organic solvent, and
ii) when $R_1$ is $C_{1-4}$ alkylcarbonyl, reacting with a halogenated alkylcarbonyl in the presence of a base and triethylamine.

**7.** The method of claim 5, wherein the compound represented by Formula 2 is obtained by:

i) when n is 0 and X is S or $NR_3$, reacting Cy-H with a compound represented by Formula 3-a under microwave irradiation,
ii) when n is 0 and X is O, reacting Cy-H with a compound represented by Formula 3-b in the presence of triethylamine,
iii) when n is 1, reacting Cy-H with a compound represented by Formula 3-c, and when $R_N$ is nitro, further reducing the reaction product, and
iv) when Y2 is N, n is 1, and Z is NH, reacting with a compound represented by Formula 3-d:

[Formula 3-a]

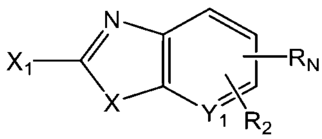

[Formula 3-b]

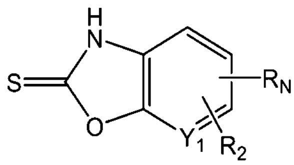

[Formula 3-c]

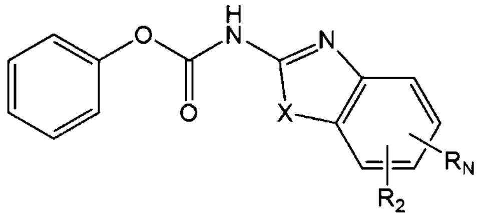

[Formula 3-d]

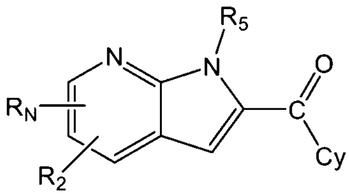

,

wherein in Formulas 3-a to 3-d,

$X_1$ is halogen, $R_N$ is nitro or amino;
$R_5$ is any one selected from t-butoxycarbonyl, hydrogen, or benzyloxycarbonyl; and
Cy is a 5- to 10-membered saturated heterocyclyl which is unsubstituted or substituted with one or more selected from the group consisting of halogen, $C_{1-4}$ alkyl, $C_{1-4}$ alkoxy, $C_{1-4}$ alkylcarbonyl, and $C_{1-4}$ alkoxycarbonyl.

**8.** The method of claim 7, wherein in the compound represented by Formula 3-a, when X is $NR_3$ and $R_3$ is $C_{1-4}$ alkyl, the compound is prepared by preparing a compound in which $R_3$ is hydrogen according to step i) and reacting the compound with halogenated $R_3$ in the presence of DBU (1,8-Diazabicyclo[5.4.0]undec-7-ene).

**9.** The method of claim 7, wherein the compound represented by Formula 3-b is prepared by reacting a compound represented by Formula 4 with potassium ethyl xanthogenate:

[Formula 4]

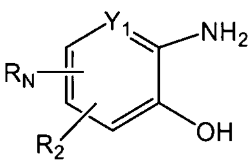

.

**10.** The method of claim 7, wherein the compound represented by Formula 3-c is prepared by reacting a compound represented by Formula 5 with phenyl chloroformate in the presence of triethylamine:

[Formula 5]

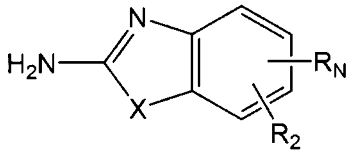

.

**11.** A pharmaceutical composition for preventing or treating a disease caused by aggregation or hyperphosphorylation of tau protein or TDP-43 protein, comprising the compound of claim 1 as an active ingredient.

**12.** The pharmaceutical composition of claim 11, wherein the disease caused by aggregation or hyperphosphorylation of tau protein is selected from the group consisting of Alzheimer's disease, Parkinson's disease, vascular dementia, acute stroke, trauma, cerebrovascular disease, brain cord trauma, spinal cord trauma, peripheral neuropathy, retinopathy, glaucoma, and tauopathies.

**13.** The pharmaceutical composition of claim 12, wherein the tauopathy is selected from the group consisting of chronic traumatic encephalopathy (CTE), primary age-related tauopathy, progressive supranuclear palsy, corticobasal degeneration, Pick's disease, argyrophilic grain disease (AGD), frontotemporal dementia (FTD), Parkinsonism linked to chromosome 17, Lytico-Bodig disease (Parkinson-dementia complex of Guam), ganglioglioma, gangliocytoma, meningioangiomatosis, postencephalitic parkinsonism, subacute sclerosing panencephalitis, lead encephalopathy, tuberous sclerosis, Pantothenate kinase-associated neurodegeneration, lipofuscinosis, posttraumatic stress disorder, and traumatic brain injury.

**14.** The pharmaceutical composition of claim 11, wherein the disease caused by aggregation or hyperphosphorylation of TDP-43 protein is selected from the group consisting of amyotrophic lateral sclerosis (ALS), Familial ALS-SOD1, sporadic amyotrophic lateral sclerosis, superoxide dismutase 1 (SOD1), frontotemporal lobar degeneration (FTLD), ALS-FTLD, Multiple system proteinopathy (MSP), limbic-predominant age-related TDP-43 encephalopathy (LATE)/CART (cerebral age-related TDP-43 with sclerosis), Perry disease, facial onset sensory and motor neuronopathy (FOSMN), and sporadic inclusion body myositis (sIBM).

**15.** The pharmaceutical composition of claim 11, **characterized by** inhibiting aggregation of tau protein or TDP-43 protein.

**16.** The pharmaceutical composition of claim 11, **characterized by** inhibiting hyperphosphorylation of tau protein or TDP-43 protein.

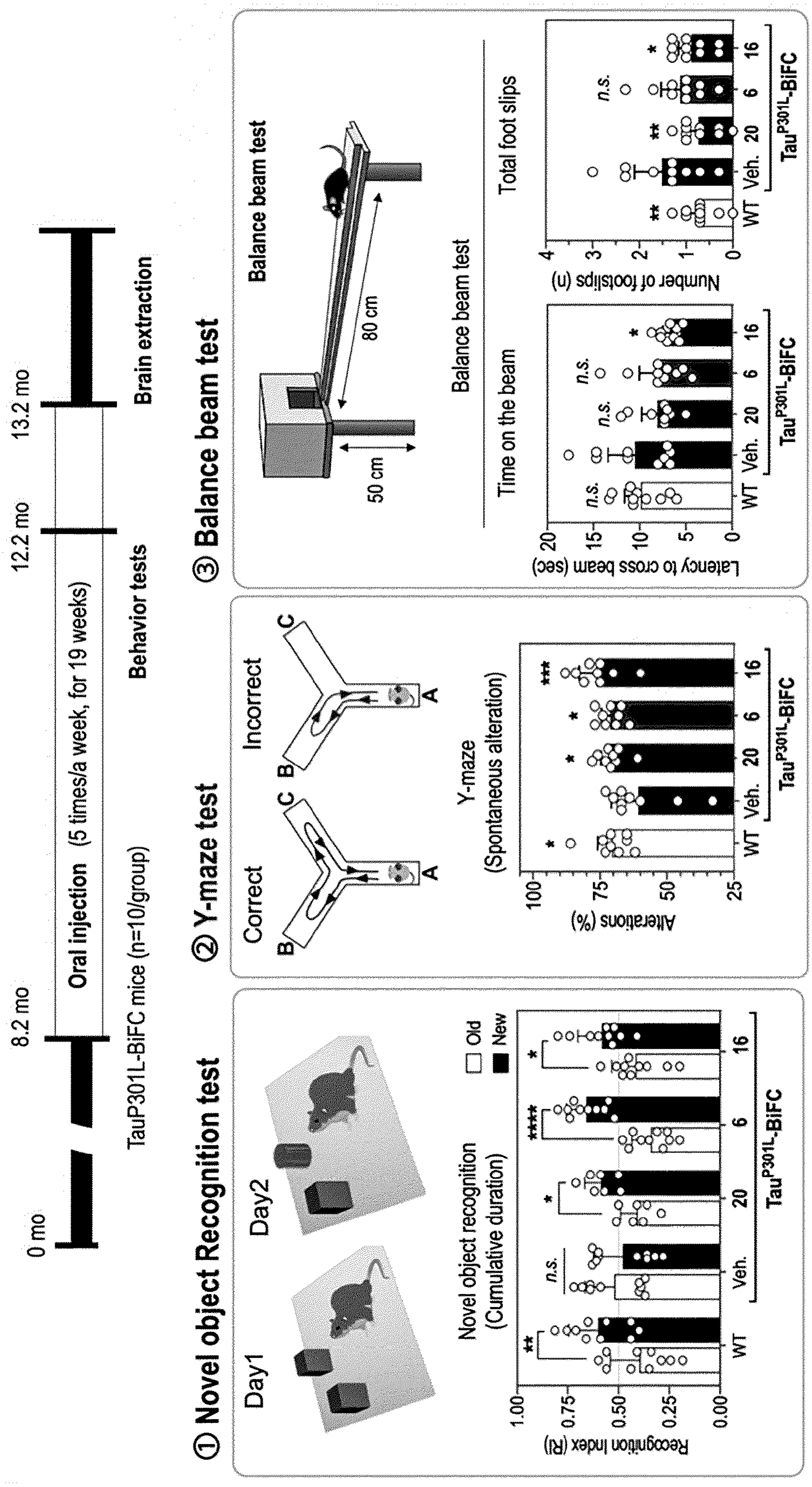
0 mo
8.2 mo
12.2 mo
13.2 mo
Oral injection (5 times/a week, for 19 weeks)
TauP301L-BiFC mice (n=10/group)
Behavior tests
Brain extraction
① Novel object Recognition test
Day1
Day2
Novel object recognition
(Cumulative duration)
Old
New
Recognition Index (RI)
1.00
0.75
0.50
0.25
0.00
**
n.s.
*
****
*
WT
Veh.
20
6
16
TauP301L-BiFC
② Y-maze test
Correct
Incorrect
A
B
C
Y-maze
(Spontaneous alteration)
Alterations (%)
100
75
50
25
*
*
*
***
WT
Veh.
20
6
16
TauP301L-BiFC
③ Balance beam test
Balance beam test
50 cm
80 cm
Balance beam test
Time on the beam
Latency to cross beam (sec)
20
15
10
5
0
n.s.
n.s.
n.s.
*
WT
Veh.
20
6
16
TauP301L-BiFC
Total foot slips
Number of footslips (n)
4
3
2
1
0
**
**
n.s.
*
WT
Veh.
20
6
16
TauP301L-BiFC

FIG. 1

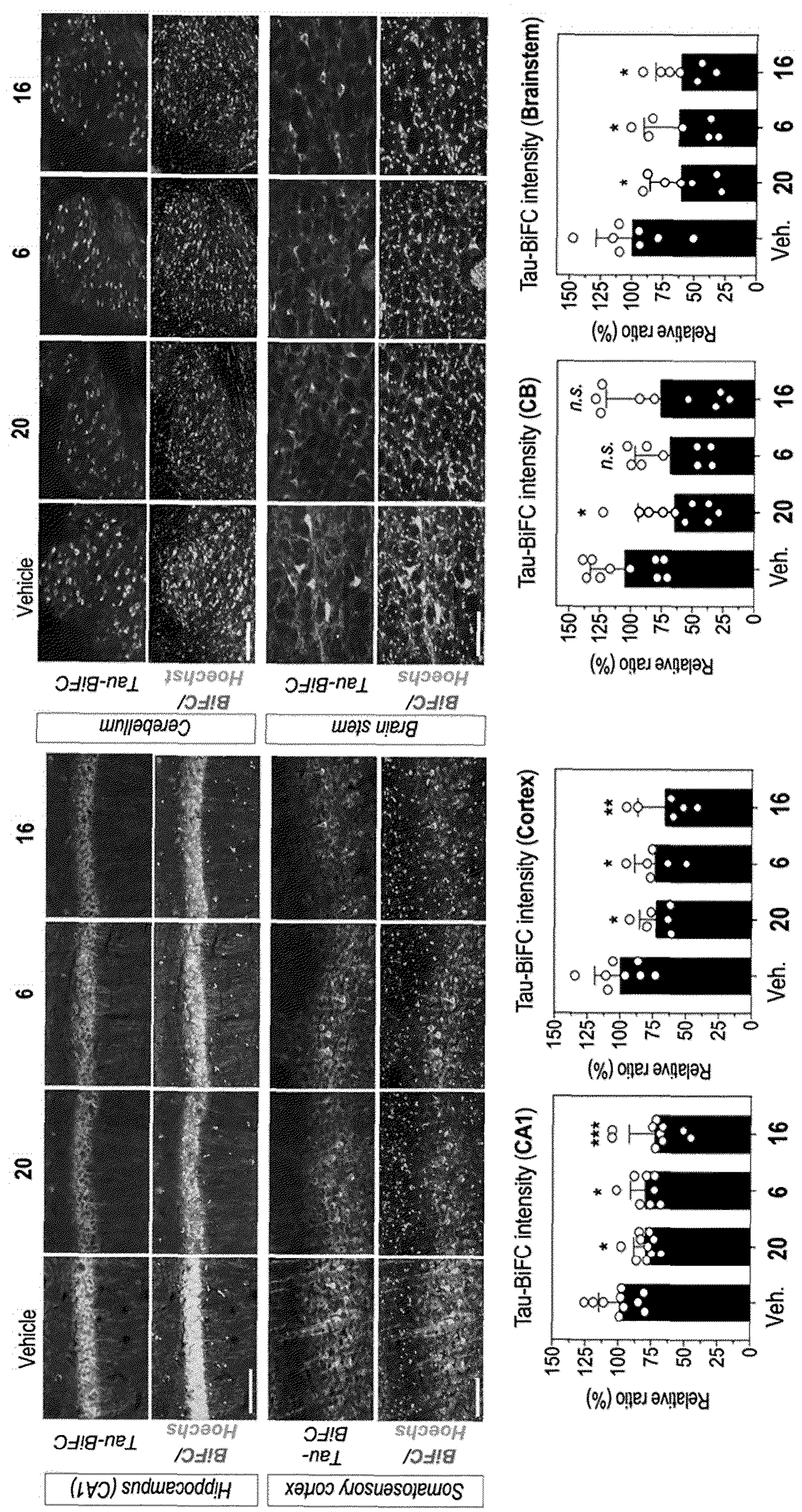
Hippocampus (CA1)
Somatosensory cortex
Cerebellum
Brain stem
Tau-BiFC
BiFC/ Hoechst
Vehicle
20
6
16
Tau-BiFC intensity (CA1)
Tau-BiFC intensity (Cortex)
Tau-BiFC intensity (CB)
Tau-BiFC intensity (Brainstem)
Relative ratio (%)
Veh.
n.s.

FIG. 2

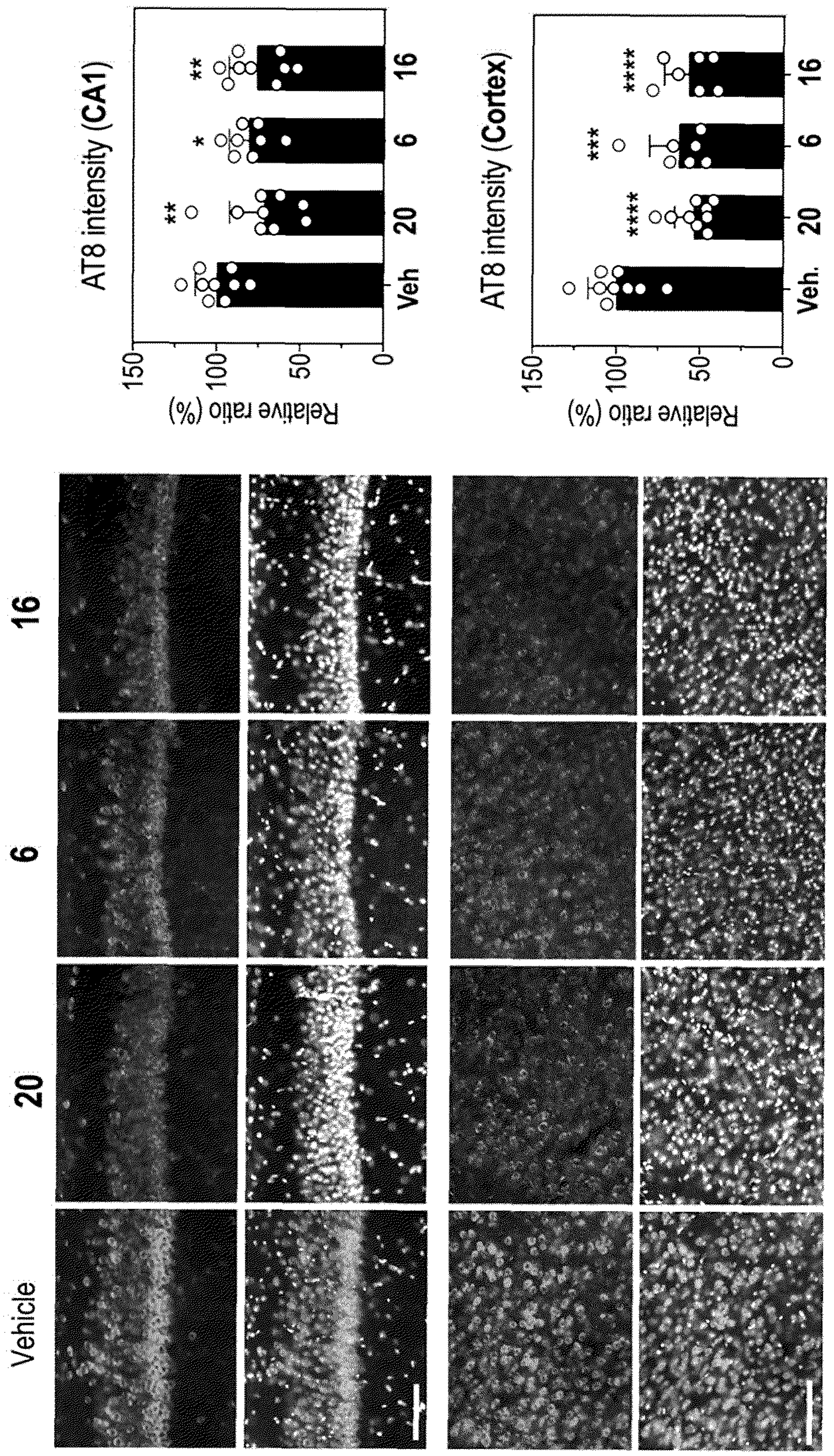
Vehicle
20
6
16
Hippocampus (CA1)
AT8
AT8/Hoechst
Somatosensory cortex
AT8
AT8 /Hoechst
AT8 intensity (CA1)
Relative ratio (%)
150
100
50
0
Veh
20
6
16
**
*
**
AT8 intensity (Cortex)
Relative ratio (%)
150
100
50
0
Veh.
20
6
16
****
***
****

FIG. 3

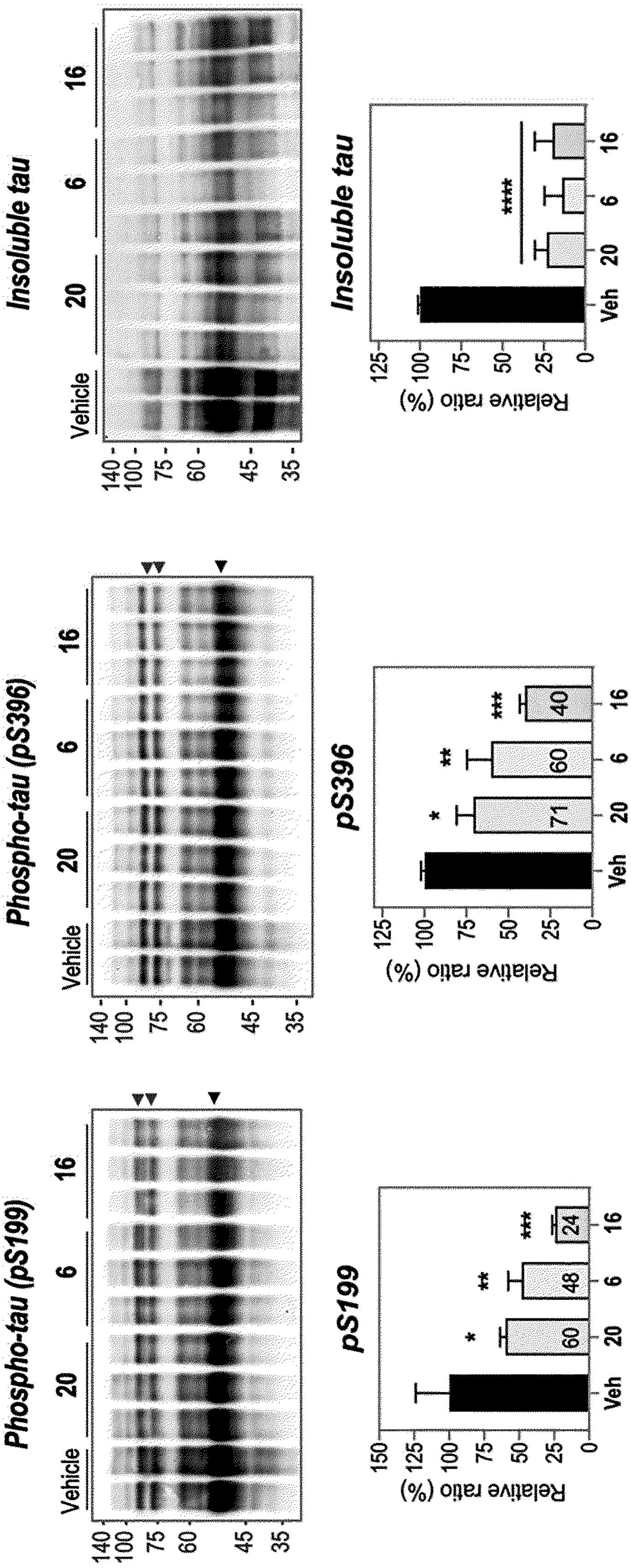
Phospho-tau (pS199)
Vehicle
20
6
16
140
100
75
60
45
35
pS199
Relative ratio (%)
150
125
100
75
50
25
0
*
**
***
60
48
24
Veh
20
6
16
Phospho-tau (pS396)
Vehicle
20
6
16
140
100
75
60
45
35
pS396
Relative ratio (%)
125
100
75
50
25
0
*
**
***
71
60
40
Veh
20
6
16
Insoluble tau
Vehicle
20
6
16
140
100
75
60
45
35
Insoluble tau
Relative ratio (%)
125
100
75
50
25
0
****
Veh
20
6
16

FIG. 4

## INTERNATIONAL SEARCH REPORT

International application No.
**PCT/KR2023/019633**

### A. CLASSIFICATION OF SUBJECT MATTER

**C07D 277/82**(2006.01)i; **A61K 31/5377**(2006.01)i; **A61K 31/5386**(2006.01)i; **A61P 25/28**(2006.01)i; **C07D 263/58**(2006.01)i; **C07D 235/30**(2006.01)i; **C07D 498/08**(2006.01)i; **C07D 413/04**(2006.01)i; **C07D 471/04**(2006.01)i; **C07D 498/04**(2006.01)i

According to International Patent Classification (IPC) or to both national classification and IPC

### B. FIELDS SEARCHED

Minimum documentation searched (classification system followed by classification symbols)

C07D 277/82(2006.01); A61K 31/425(2006.01); C07D 213/04(2006.01); C07D 221/00(2006.01); C07D 235/04(2006.01); C07D 235/26(2006.01); C07D 471/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 카르보노히드라조노일 디시아나이드(carbonohydrazonoyl dicyanide), 포화헤테로고리(saturated heterocycle), 타우 단백질(Tau protein), 티디피-43 단백질(TDP-43 protein), 응집 저해(aggregation inhibition)

### C. DOCUMENTS CONSIDERED TO BE RELEVANT

| Category* | Citation of document, with indication, where appropriate, of the relevant passages | Relevant to claim No. |
|---|---|---|
| A | KR 10-2020-0076808 A (KOREA INSTITUTE OF SCIENCE AND TECHNOLOGY) 30 June 2020 (2020-06-30)<br>See claims 1, 7, 12 and 24; and paragraphs [0262], [0291] and [0292]. | 1-16 |
| A | KR 10-2020-0076655 A (KOREA INSTITUTE OF SCIENCE AND TECHNOLOGY) 29 June 2020 (2020-06-29)<br>See entire document. | 1-16 |
| A | WO 2021-256899 A1 (KOREA INSTITUTE OF SCIENCE AND TECHNOLOGY) 23 December 2021.<br>See entire document. | 1-16 |
| A | US 2022-0380319 A1 (BIAL - R&D INVESTMENTS, S.A.) 01 December 2022 (2022-12-01)<br>See entire document. | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 March 2024** | **27 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/019633**

**C. DOCUMENTS CONSIDERED TO BE RELEVANT**

| Category* | Citation of document, with indication, where appropriate, of the relevant passages | Relevant to claim No. |
|---|---|---|
| A | WO 01-94346 A1 (F. HOFFMANN-LA ROCHE AG) 13 December 2001 (2001-12-13)<br>See entire document. | 1-16 |

Form PCT/ISA/210 (second sheet) (July 2022)

**INTERNATIONAL SEARCH REPORT**
**Information on patent family members**

International application No.
**PCT/KR2023/019633**

| Patent document cited in search report | Publication date (day/month/year) | Patent family member(s) | Publication date (day/month/year) |
|---|---|---|---|
| KR 10-2020-0076808 A | 30 June 2020 | AU 2018-453188 A1 | 08 July 2021 |
| | | AU 2018-453188 B2 | 19 January 2023 |
| | | BR 112021012004 A2 | 08 September 2021 |
| | | CA 3124102 A1 | 25 June 2020 |
| | | CA 3124102 C | 17 October 2023 |
| | | CN 113727972 A | 30 November 2021 |
| | | EP 3901139 A1 | 27 October 2021 |
| | | JP 2022-515787 A | 22 February 2022 |
| | | JP 7375019 B2 | 07 November 2023 |
| | | KR 10-2128509 B1 | 01 July 2020 |
| | | MX 2021007464 A | 10 December 2021 |
| | | US 2022-0396553 A1 | 15 December 2022 |
| | | WO 2020-130214 A1 | 25 June 2020 |
| KR 10-2020-0076655 A | 29 June 2020 | KR 10-2178181 B1 | 13 November 2020 |
| WO 2021-256899 A1 | | AR 122677 A1 | 28 September 2022 |
| | | AU 2023-291666 A1 | 02 February 2023 |
| | | BR 112022025610 A2 | 03 January 2023 |
| | | CA 3182243 A1 | 23 December 2021 |
| | | CN 115867277 A | 28 March 2023 |
| | | EP 4169905 A1 | 26 April 2023 |
| | | JP 2023-530337 A | 14 July 2023 |
| | | KR 10-2021-0157360 A | 28 December 2021 |
| | | TW 202200548 A | 01 January 2022 |
| | | TW I794843 B | 01 March 2023 |
| | | US 2023-0295095 A1 | 21 September 2023 |
| US 2022-0380319 A1 | 01 December 2022 | AU 2020-348771 A1 | 17 March 2022 |
| | | BR 112022004801 A2 | 21 June 2022 |
| | | CA 3151039 A1 | 25 March 2021 |
| | | CN 114787136 A | 22 July 2022 |
| | | EP 4031532 A1 | 27 July 2022 |
| | | JP 2022-548381 A | 18 November 2022 |
| | | KR 10-2022-0102607 A | 20 July 2022 |
| | | MX 2022003236 A | 04 July 2022 |
| | | WO 2021-055591 A1 | 25 March 2021 |
| WO 01-94346 A1 | 13 December 2001 | AR 028683 A1 | 21 May 2003 |
| | | AT E381565 T1 | 15 January 2008 |
| | | AU 2001-267513 B2 | 16 March 2006 |
| | | AU 6751301 A | 17 December 2001 |
| | | BR 0111538 A | 01 July 2003 |
| | | CA 2411716 A1 | 13 December 2001 |
| | | CA 2411716 C | 22 June 2010 |
| | | CN 1261433 C | 28 June 2006 |
| | | CN 1436188 A | 13 August 2003 |
| | | EP 1292596 A1 | 19 March 2003 |
| | | EP 1292596 B1 | 19 December 2007 |
| | | ES 2296761 T3 | 01 May 2008 |
| | | JP 2003-535863 A | 02 December 2003 |
| | | JP 4245348 B2 | 25 March 2009 |
| | | KR 10-0518198 B1 | 04 October 2005 |
| | | KR 10-2003-0016283 A | 26 February 2003 |

Form PCT/ISA/210 (patent family annex) (July 2022)

**INTERNATIONAL SEARCH REPORT**
**Information on patent family members**

International application No.
**PCT/KR2023/019633**

| Patent document cited in search report | Publication date (day/month/year) | Patent family member(s) | Publication date (day/month/year) |
|---|---|---|---|
| | | MX PA02012001 A | 22 April 2003 |
| | | PE 20020298 A1 | 17 April 2002 |
| | | US 2002-0006932 A1 | 17 January 2002 |
| | | US 6482829 B2 | 19 November 2002 |
| | | UY 26754 A1 | 28 December 2001 |
| | | ZA 200209488 B | 23 February 2004 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 1020200039398 A


### Non-patent literature cited in the description


- *Mol. Neurodegeneration*, 2009, vol. 4, 13
- *Biophys. J.*, 2006, vol. 90, 3739-3748
- *J. Biol. Chem.*, 1995, vol. 270, 7679-7688
- Psychiatry. *J. Neurol. Neurosurg*, 2021, vol. 92, 86-95
- Front. Neuroscience. 2022, vol. 16, 815765
- *Geroscience*, 2021, vol. 43, 1627-1634
- *Brain*, 2022, vol. 145, 2769-2784